# EUROPEAN PATENT SPECIFICATION

(11) **EP 1 939 288 B1**
(45) Date of publication and mention of the grant of the patent: **19.12.2012**
(21) Application number: 06810939.6
(22) Date of filing: 29.09.2006
(51) Int. Cl.: C07K 14/705, C07K 16/28

(54) **T-CELL ADHESION MOLECULE AND ANTIBODY DIRECTED AGAINST THE MOLECULE**
T-ZELLADHÄSIONSMOLEKÜL UND GEGEN DAS MOLEKÜL GERICHTETER ANTIKÖRPER
MOLÉCULE D'ADHÉSION DE LYMPHOCYTE T ET ANTICORPS DIRIGÉ CONTRE LA MOLÉCULE

(30) Priority: 29.09.2005 JP 2005285194
(43) Date of publication of application: 02.07.2008
(73) Proprietor: Eisai R&D Management Co., Ltd., Tokyo 112-8088 (JP)
(72) Inventor: OGASAWARA, Hideaki, KAN Research Institute Inc., 6-Chome, Chuo-Ku, Kobe-Shi, Hyogo-Ken 650-0047 (JP); MIZUNO, Keiko, KAN Research Institute Inc., 6-Chome, Chuo-Ku, Kobe-Shi, Hyogo-Ken 650-0047 (JP); ARITA, Yoshihisa, KAN Research Institute Inc., 6-Chome, Chuo-Ku, Kobe-Shi, Hyogo-Ken 650-0047 (JP); NISHIMURA, Miyuki, KAN Research Institute Inc., 6-Chome, Chuo-Ku, Kobe-Shi, Hyogo-Ken 650-0047 (JP); IMAI, Toshio, KAN Research Institute Inc., 6-Chome, Chuo-Ku, Kobe-Shi, Hyogo-Ken 650-0047 (JP)
(74) Representative: Brearley, Helen Rebecca
(86) International application number: PCT/JP2006/319576
(87) International publication number: WO 2007/037430

(56) References cited:
- WO-A2-00/78953
- WO-A2-01/55335
- WO-A2-01/55335
- DATABASE EMBL [Online] 6 September 2005 (2005-09-06), "Mus musculus B6-derived CD11 +ve dendritic cells cDNA, RIKEN full-length enriched library, clone:F730223I20 product:hypothetical Immunoglobulin-like/Immunoglobulin subtype/Ig-like domain profile containing protein, full insert sequence." XP003011533 retrieved from EBI accession no. EMBL:AK155804 Database accession no. AK155804
- DATABASE Geneseq [Online] 29 January 2004 (2004-01-29), "Novel human secreted and transmembrane protein PRO7425." XP002575948 retrieved from EBI accession no. GSP:ADD85423 Database accession no. ADD85423
- THE FANTOM CONSORTIUM, THE RIKEN GENOME EXPLORATION RESEARCH GROUP PHASE I AND II TEAM: 'Analysis of the mouse transcriptome based on functional annotation of 60,770 full-length cDNAs' NATURE vol. 420, 2002, pages 563 - 573, XP001165660
- DATABASE GENBANK [Online] CARNINCI P. AND HAYASHIZAKI Y.: 'High-efficiency full-length cDNA cloning', XP003011531 Retrieved from NCBI Database accession no. (AK170277) & METH. ENZYMOL. vol. 303, 1999, pages 19 - 44
- DATABASE GENBANK [Online] CARNINCI P. AND HAYASHIZAKI Y.: 'High-efficiency full-length cDNA cloning', XP003011532 Retrieved from NCBI Database accession no. (AK036904) & METH. ENZYMOL. vol. 303, 1999, pages 19 - 44
- DATABASE GENBANK [Online] CARNINCI P. AND HAYASHIZAKI Y.: 'High-efficiency full-length cDNA cloning', XP003011533 Retrieved from NCBI Database accession no. (AK155804) & METH. ENZYMOL. vol. 303, 1999, pages 19 - 44
- DATABASE GENBANK [Online] XP003011534 Retrieved from NCBI Database accession no. (BQ713326)

## Description

### Technical Field

A T cell adhesion molecule is expressed on dendritic cells derived from bone marrow by a gene thereof, and a ligand for the adhesion molecule (receptor) is expressed on T cells by a gene thereof. The present invention relates to an antibody against the adhesion molecule or the ligand and a use thereof. Moreover, the present invention also relates to a screening method using the adhesion molecule.

### Background Art

In recent years, it has been reported that a molecule cloned as a dendritic cell activator that is expressed on T cells is a main cytokine for regulating differentiation of osteoclasts (Yasuda, H., et al. (1998) Proc Natl Acad Sci USA 95: 3597-3602). Thus, it has been clarified that an immune system is closely associated with bone metabolism.

Studies in regulation of bone metabolism by immune system-regulating molecules have rapidly progressed, and signal transduction associated with regulation of osteoclast differentiation has also been clarified.

For example, as a molecule involved in osteoclast differentiation, Oscar (Osteoclast-associated receptor) has been known. It has been reported to date that Oscar is an immunoglobulin-like receptor, which is associated with the FcRγ chain and transmits a signal to phospholipase Cγ via an ITAM motif of the FcRγ chain (Kim, N., et al. (2002) J Exp Med 195: 201-209).

In addition, various interactions such as CD80-CD28, CD40-CD40L or ICAM1-LFA1 have been reported for molecules that are expressed on dendritic cells and adhere to a T cell so as to be involved in an interaction regarding an immune response between T cells and dendritic cells.

### Summary of the Invention

The present inventors have identified a gene that is specifically expressed on dendritic cells derived from bone marrow by a subtraction method. Also, the inventors have found that a protein encoded by the aforementioned gene binds to the FcRγ chain constituting the IgE receptor, that expression of the aforementioned protein is enhanced by LPS stimulus, that dendritic cells are activated by antibody cross-link stimulus to the aforementioned protein, that the aforementioned protein has a function of adhering to a T cell, and that an antibody against the aforementioned protein has a therapeutic effect on a collagen-induced arthritis model that is a disease model of rheumatoid arthritis. The inventors have further identified a gene of a ligand for the aforementioned protein, which is expressed on T cells. The present invention is based on these findings.

A membrane or secretory protein comprises the amino acid sequence of SEQ ID NO: 2, SEQ ID NO: 4, SEQ ID NO: 6 or SEQ ID NO: 8, or an amino acid sequence of SEQ ID NO: 2, SEQ ID NO: 4, SEQ ID NO: 6 or SEQ ID NO: 8 which contains one or more conservative substitutions (hereinafter referred to as a first protein).

A further membrane or secretory protein is selected from the following (i), (ii), (iii) and (iv) (hereinafter referred to as a second protein):
(i) a membrane or secretory protein comprising the amino acid sequence of SEQ ID NO: 10;
(ii) a membrane or secretory protein which comprises an amino acid sequence of SEQ ID NO: 10 in which one or more amino acids are inserted, substituted or deleted, or one or more amino acids are added to one or both of ends, and which is functionally equivalent to a protein consisting of the amino acid sequence of SEQ ID NO: 10;
(iii) a membrane or secretory protein which is encoded by a polynucleotide which hybridizes under stringent conditions to a polynucleotide which encodes the amino acid sequence of SEQ ID NO: 10, and which is functionally equivalent to a protein consisting of the amino acid sequence of SEQ ID NO: 10; and
(iv) a membrane or secretory protein which comprises an amino acid sequence having 90% or more identity with the amino acid sequence of SEQ ID NO: 10, and which is functionally equivalent to a protein consisting of the amino acid sequence of SEQ ID NO: 10.

A polynucleotide encodes the novel proteins of the first and second embodiments according to the present invention.

The polynucleotide is selected from the following (v), (vi), (vii) and (viii):
(v) a polynucleotide comprising the nucleotide sequence of SEQ ID NO: 9;
(vi) a polynucleotide which comprises a nucleotide sequence of SEQ ID NO: 9 in which one or more nucleotides are inserted, substituted or deleted, or one or more nucleotides are added to one or both of ends, and which encodes a membrane or secretory protein functionally equivalent to a protein consisting of the amino acid sequence of SEQ ID NO: 10;
(vii) a polynucleotide which hybridizes under stringent conditions with a polynucleotide comprising the nucleotide sequence of SEQ ID NO: 9, and which encodes a membrane or secretory protein functionally equivalent to a protein consisting of the amino acid sequence of SEQ ID NO: 10; and
(viii) a polynucleotide which has 90% or more identity with a polynucleotide comprising the nucleotide sequence of SEQ ID NO: 9, and which encodes a membrane or secretory protein functionally equivalent to a protein consisting of the amino acid sequence of SEQ ID NO: 10.

The present invention provides an antibody against a membrane or secretory protein selected from the following (ix), (x), (xi) and (xii) (hereinafter, occasionally referred to as a TARM (T cell-interacting Activating Receptor on Myeloid cells) protein), and a functional fragment thereof (hereinafter referred to as an antibody of a first embodiment according to the present invention):

(ix) a membrane or secretory protein comprising the amino acid sequence of SEQ ID NO: 2, SEQ ID NO: 4, SEQ ID NO: 6, SEQ ID NO: 8, SEQ ID NO: 10 or SEQ ID NO: 12;
(x) a membrane or secretory protein which comprises an amino acid sequence of SEQ ID NO: 2, SEQ ID NO: 4, SEQ ID NO: 6, SEQ ID NO: 8, SEQ ID NO: 10 or SEQ ID NO: 12 in which one or more amino acids are inserted, substituted or deleted, or one or more amino acids are added to one or both of ends, and which is functionally equivalent to a protein consisting of the amino acid sequence of SEQ ID NO: 2, SEQ ID NO: 4, SEQ ID NO: 6, SEQ ID NO: 8, SEQ ID NO: 10 or SEQ ID NO: 12;
(xi) a membrane or secretory protein which is encoded by a polynucleotide which hybridizes under stringent conditions to a polynucleotide which encodes the amino acid sequence of SEQ ID NO: 2, SEQ ID NO: 4, SEQ ID NO: 6, SEQ ID NO: 8, SEQ ID NO: 10 or SEQ ID NO: 12, and which is functionally equivalent to a protein consisting of the amino acid sequence of SEQ ID NO: 2, SEQ ID NO: 4, SEQ ID NO: 6, SEQ ID NO: 8, SEQ ID NO: 10 or SEQ ID NO: 12; and
(xii) a membrane or secretory protein which comprises an amino acid sequence having 70% or more identity with the amino acid sequence of SEQ ID NO: 2, SEQ ID NO: 4, SEQ ID NO: 6, SEQ ID NO: 8, SEQ ID NO: 10 or SEQ ID NO: 12, and which is functionally equivalent to a protein consisting of the amino acid sequence of SEQ ID NO: 2, SEQ ID NO: 4, SEQ ID NO: 6, SEQ ID NO: 8, SEQ ID NO: 10 or SEQ ID NO: 12;
for use in the treatment of an autoimmune disease.

A ligand protein that is a ligand for the protein according to the present invention, is selected from the following (xiii), (xiv), (xv) and (xvi) (hereinafter, occasionally referred to as a ligand protein or a TARM-L (TARM ligand) protein):
(xiii) a protein comprising the amino acid sequence of SEQ ID NO: 14 or SEQ ID NO: 16;
(xiv) a protein which comprises an amino acid sequence of SEQ ID NO: 14 or SEQ ID NO: 16 in which one or more amino acids are inserted, substituted or deleted, or one or more amino acids are added to one or both of ends, and which is functionally equivalent to a protein consisting of the amino acid sequence of SEQ ID NO: 14 or SEQ ID NO: 16;
(xv) a protein which is encoded by a polynucleotide which hybridizes under stringent conditions to a polynucleotide which encodes the amino acid sequence of SEQ ID NO: 14 or SEQ ID NO: 16, and which is functionally equivalent to a protein consisting of the amino acid sequence of SEQ ID NO: 14 or SEQ ID NO: 16; and
(xvi) a protein which comprises an amino acid sequence having 70% or more identity with the amino acid sequence of SEQ ID NO: 14 or SEQ ID NO: 16, and which is functionally equivalent to a protein consisting of the amino acid sequence of SEQ ID NO: 14 or SEQ ID NO: 16.

A polynucleotide encodes the ligand protein.

The present invention also provides a polynucleotide selected from the following (xvii), (xviii), (xix) and (xx):
(xvii) a polynucleotide comprising the nucleotide sequence of SEQ ID NO: 13 or SEQ ID NO: 15;
(xviii) a polynucleotide which comprises a nucleotide sequence of SEQ ID NO: 13 or SEQ ID NO: 15 in which one or more nucleotides are inserted, substituted or deleted, or one or more nucleotides are added to one or both of ends, and which encodes a protein functionally equivalent to a protein consisting of the amino acid sequence of SEQ ID NO: 14 or SEQ ID NO: 16;
(xix) a polynucleotide which hybridizes under stringent conditions with a polynucleotide comprising the nucleotide sequence of SEQ ID NO: 13 or SEQ ID NO: 15, and which encodes a protein functionally equivalent to a protein consisting of the amino acid sequence of SEQ ID NO: 14 or SEQ ID NO: 16; and
(xx) a polynucleotide which has 70% or more identity with a polynucleotide comprising the nucleotide sequence of SEQ ID NO: 13 or SEQ ID NO: 15, and which encodes a protein functionally equivalent to a protein consisting of the amino acid sequence of SEQ ID NO: 14 or SEQ ID NO: 16.

The present invention provides an antibody against the ligand protein selected from (xiii), (xiv), (xv) and (xvi), and a functional fragment thereof (hereinafter referred to as an antibody of a second embodiment according to the preset invention).

The present invention provides a therapeutic agent for autoimmune diseases comprising, as active ingredients, the antibodies of the first and second embodiments according to the present invention (hereinafter, both antibodies may be referred to as "antibodies according to the present invention"), or functional fragments thereof.

The present invention provides the following screening methods.

According to a screening method of a second embodiment according to the present invention, there is provided a method for screening for a substance that inhibits activation of a dendritic cell or a salt thereof, or a solvate thereof, which comprises the steps of:
(d) contacting an antibody or a functional fragment thereof according to the present invention with a dendritic cell in the presence or absence of a test substance; and
(e) measuring the level of activation of said dendritic cell.

According to a screening method of a third embodiment according to the present invention, there is provided a method for screening for a substance that inhibits a complex formation between a TARM protein and the FcRγ chain or a salt thereof, or a solvate thereof, which comprises the steps of:
(g) contacting an antibody or a functional fragment thereof according to the present invention with a dendritic cell in the presence or absence of a test substance; and
(h) measuring the expression level of the FcRγ chain in said dendritic cell.

### Brief Description of the Drawings

Figure 1 shows the amino acid sequences of mouse TARM genes (ml to 4 and s1), wherein the underline indicates an immunoglobulin (IG) loop structure region and the bold type indicates a transmembrane region.
Figure 2 shows the results obtained by analyzing the mRNA expression of TARM in mouse tissues by real-time PCR using primer set 1.
Figure 3 shows the results obtained by analyzing the mRNA expression of TARM in various types of cells by real-time PCR using primer set 1.
Figure 4A shows the structure and amino acid numbers of an extracellular region used in producing anti-TARM antibodies. Figure 4B shows the results obtained by studying the specificity of the aforementioned antibody for the TARM protein.
Figure 5 shows the results obtained by analyzing expression of mouse proteins on bone marrow-derived dendritic cells.
Figure 6 shows the results obtained by analyzing expression of mouse proteins on cells derived from normal mouse immune tissues.
Figure 7 shows the results obtained by analyzing expression of the mouse protein on c-kit-positive peritoneal mast cells.
Figure 8 shows the results obtained by analyzing expression of the mouse TARM protein in mouse lymph node cells by LPS inflammatory stimulus, wherein the arrows indicate expression of the mouse TARM protein.
Figure 9A shows induction of production of IL-6 from mature bone marrow dendritic cells by anti-TARM antibody stimulus. Figure 9B shows induction of production of MCP-1 from immature bone marrow dendritic cells by anti-TARM antibody stimulus.
Figure 10 shows increases in the FcRγ chain associated with increases in expression of the mouse TARM protein on the cell surface.
Figure 11 shows the results obtained by analyzing a complex formation between the mouse TARM protein and the FcRγ chain by an immunoprecipitation method.
Figure 12 shows the results obtained by analyzing expression of molecules binding to the mouse TARM protein on activated T cells.
Figure 13 shows the ability of activated T cells to adhere to the mouse TARM protein.
Figure 14 shows inhibition of adhesion of Th2 cells to the mouse TARM protein by anti-mouse TARM antibodies.
Figure 15 shows the external findings and changes in body weights over time of a collagen-induced arthritis model, to which an anti-mouse TARM antibody has been administered.
Figure 16 shows the effect of administering the anti-mouse TARM antibody to the collagen-induced arthritis model on serum amyloid A concentrations in plasma thereof.
Figure 17 shows the effect of administering the anti-mouse TARM antibody to the collagen-induced arthritis model on anti-collagen antibody titers in plasma thereof.
Figure 18 shows the results obtained by analyzing mRNA expression of the TARM protein in human tissues by real-time PCR.
Figure 19 shows the amino acid sequences of the human TARM protein, wherein the underline indicates an immunoglobulin (Ig) loop structure region, the bold type indicates a transmembrane region, and the enclosed portion indicates sequences different between hTARM and LOC441864.
Figure 20 shows the ability of activated T cells to adhere to the human TARM protein, and inhibition of adhesion of T cells to the human TARM protein by anti-human TARM antibodies.
Figure 21A shows the results obtained by analyzing expression of molecules binding to the mouse TARM protein in mouse cell lines. Figure 21B shows the results obtained by analyzing expression of the mRNA of ENSMUSG00000035095, a mTARM-L candidate molecule in mouse cell lines by real-time PCR.
Figure 22A shows the specific binding of the mouse TARM-AP chimeric protein to B300.19 cells expressing mTARM-L. Figure 22B shows the specific cell adhesion of B300.19 cells expressing mTARM-L to the mouse TARM-AP chimeric protein.
Figure 23 shows the results obtained by analyzing homology between human and mouse TARM-L proteins.
Figure 24 shows the results obtained by analyzing homology between the human TARM protein and the mouse TARM protein (m3).

### Detailed Description of the Invention

The present invention will be described in detail below. The following descriptions are given just as examples for explaining the present invention, and thus such examples are not intended to limit the present invention only to the embodiments of the present invention. All the technological terms, scientific terms and technical terms used in the present specification have the same meanings as those generally understood by persons skilled in the technical field to which the present invention pertains. Such terms are used for the purpose of only explaining specific embodiments, and thus they are not intended to limit the present invention. The present invention can be carried out in various embodiments, unless it deviates from the gist thereof. All prior art references and patent documents such as patent applications or patent publications cited herein are incorporated herein by reference in their entirety, and can be used to carry out the present invention.

### [Proteins and polynucleotides]

Among genes that are specifically expressed on dendritic cells derived from bone marrow, which were identified, a mouse-derived gene has 5 types of isoforms. Such isoforms of a mouse-derived gene include m1, m2, m3 and m4 that are membrane-bound protein (membrane protein) genes, and s1 that is a secretory-type protein (secretory protein) gene. The nucleotide sequences and amino acid sequences of such isoforms correspond to the following sequence numbers.

| | |
|---|---|
| m1 | SEQ ID NOS: 11 and 12 |
| m2 | SEQ ID NOS: 1 and 2 |
| m3 | SEQ ID NOS: 3 and 4 |
| m4 | SEQ ID NOS: 5 and 6 |
| s1 | SEQ ID NOS: 7 and 8 |

Among the genes that are specifically expressed on bone marrow-derived dendritic cells, one type of membrane protein gene can be mentioned as a human-derived gene. The nucleotide sequence of this gene and the amino acid sequence of a protein encoded by the gene are as shown in SEQ ID NOS: 9 and 10, respectively.

Since the nucleotide sequence of the gene encodes a signal peptide, a protein encoded by the aforementioned gene forms a membrane protein or a secretory protein. The C-terminal of the membrane protein according to the present invention is modified (for example, by deleting a transmembrane portion), so as to obtain a secretory protein. The C-terminal of the secretory protein according to the present invention is modified (for example, by adding a transmembrane portion thereto), so as to obtain a membrane protein.

In the present specification, the expressions "one or more amino acids are inserted, substituted or deleted, or added to one or both of ends" and "one or more nucleotides are inserted, substituted or deleted, or one or more nucleotides are added to one or both of ends" means that the modification has been carried out according to well-known technical methods such as site-directed mutagenesis, or by substitution of a plurality number of amino acids or nucleotides to an extent of being naturally generated. The number of amino acids or nucleotides to be modified may be, for example, 1 to 30, preferably 1 to 20, more preferably 1 to 10, further more preferably 1 to 5, and particularly preferably 1 or 2.

The modified amino acid sequence can preferably be an amino acid sequence having one or more (preferably, one or several, or 1, 2, 3 or 4) conservative substitutions in the amino acid sequence.

The modified nucleotide sequence can preferably be a nucleotide sequence having one or more (for example, one to several, or 1, 2, 3 or 4) mutations which do not affect the functions of a protein consisting of the amino acid sequence of SEQ ID NO: 10.

In the present specification, the term "conservative substitution" means that one or more amino acid residues are substituted with other chemically similar amino acid residues, such that the functions of a protein are not substantially modified. Examples of such conservative substitution include a case where a hydrophobic residue is substituted with another hydrophobic residue and a case where a certain polar residue is substituted with another polar residue having the same electric charge. For every type of amino acids, functionally similar amino acids which can be substituted in such a manner are known in the present technical field. Examples of nonpolar (hydrophobic) amino acids include alanine, valine, isoleucine, leucine, proline, tryptophan, phenylalanine, and methionine. Examples of polar (neutral) amino acids include glycine, serine, threonine, tyrosine, glutamine, asparagine, and cysteine. Examples of positively charged (basic) amino acids include arginine, histidine, and lysine. Examples of negatively charged (acidic) amino acids include aspartic acid and glutamic acid.

In the present specification, the term "hybridize" means to hybridize with a target polynucleotide under stringent conditions. Specifically, there can be exemplified a polynucleotide having at least 70% or more, preferably 80% or more, more preferably 85% or more, further more preferably 90% or more, still further more preferably 95% or more, particularly preferably 98% or more, and most preferably 99% or more identity with the target nucleotide sequence, when such identity is calculated using a default (initialization) parameter with homology search software such as FASTA, BLAST, or Smith-Waterman [Meth. Enzym., 164, 765 (1988)]. In addition, the term "under stringent conditions" means conditions wherein a reaction is carried out in a hybridization buffer that can be commonly used by persons skilled in the art, at a temperature of 40°C to 70°C, and preferably 60°C to 65°C, and the reaction product is then washed with a washing solution having a salt concentration of 15 to 300 mmol/L, and preferably 15 to 60 mmol/L. Such a temperature and a salt concentration can be appropriately adjusted depending on the length of a probe used. Moreover, conditions for washing the product obtained by hybridization can be 0.2 or 2 x SSC, 0.1% SDS, and a temperature of 20°C to 68°C. It is possible to determine stringent conditions (high stringency) or mild conditions (low stringency) by making a difference with a salt concentration or a temperature applied during washing. When such a hybridization difference is made with a salt concentration, 0.2 x SSC, 0.1% SDS can be used as a stringent wash buffer (high stringency wash buffer), and 2 x SSC, 0.1% SDS can be used as a mild wash buffer (low stringency wash buffer). On the other hand, when such a difference is made with a temperature, a temperature of 68°C is applied in the case of high stringency, a temperature of 42°C is applied in the case of moderate stringency, and a room temperature (20°C to 25°C) is applied in the case of low stringency. In all the three above cases, the reaction may be carried out in 0.2 x SSC, 0.1% SDS.

In general, the pre-hybridization is carried out under the same conditions as those for hybridization. However, hybridization and pre-washing are not always carried out under the same conditions.

Hybridization can be carried out in accordance with a known method. In the case of using a commercially available library, hybridization can be carried out according to the method described in instructions included therewith.

In the present specification, the term "identity" (occasionally referred to as "homology") regarding amino acid sequences and nucleotide sequences means the degree of coincidence between the compared sequences in terms of amino acid residues or nucleotide residues that constitute such sequences. At that time, the presence of a gap and the property of amino acids are taken into consideration (Wilbur, Natl. Acad. Sci. U.S.A. 80: 726-730 (1983)). For calculation of homology, commercially available homology search software products such as BLAST (Altschul: J. Mol. Biol. 215: 403-410 (1990)), FASTA (Peasron: Methods in Enzymology 183: 63-69 (1990)), or Smith-Waterman [Meth. Enzym., 164, 765 (1988)] can be used.
The numerical value of such "identity" may be calculated using a homology search program known to persons skilled in the art. For example, such a numerical value as identity can be calculated using a default (initialization) parameter in the homology algorithm BLAST ((Basic local alignment search tool) http://www.ncbi.nlm.nih.gov/BLAST/) of the National Center for Biotechnology Information (NCBI).

In the second protein, an amino acid sequence having 90% or more identity with the amino acid sequence of SEQ ID NO: 10 can be an amino acid sequence having preferably 95% or more, particularly preferably 98% or more, and most preferably 99% or more identity with the aforementioned amino acid sequence.

In the polynucleotide encoding the second protein , a nucleotide sequence having 90% or more identity with the nucleotide sequence of SEQ ID NO: 9 can be a nucleotide sequence having preferably 95% or more, particularly preferably 98% or more, and most preferably 99% or more identity with the aforementioned nucleotide sequence.

In the antibody for use of the first embodiment according to the present invention, an amino acid sequence having 70% or more identity with the amino acid sequence of SEQ ID NO: 2, SEQ ID NO: 4, SEQ ID NO: 6, SEQ ID NO: 8, SEQ ID NO: 10 or SEQ ID NO: 12 can be an amino acid sequence having preferably 80% or more, more preferably 85% or more, further more preferably 90% or more, still further more preferably 95% or more, particularly preferably 98% or more, and most preferably 99% or more identity with the aforementioned amino acid sequence.

In the present invention, if the amino acid sequence of SEQ ID NO: 2, SEQ ID NO: 4, SEQ ID NO: 6, SEQ ID NO: 8, SEQ ID NO: 10 or SEQ ID NO: 12 is given, a nucleotide sequence encoding it can easily be determined. Thus, various nucleotide sequences encoding the amino acid sequence of SEQ ID NO: 2, SEQ ID NO: 4, SEQ ID NO: 6, SEQ ID NO: 8, SEQ ID NO: 10 or SEQ ID NO: 12 can be selected.

Accordingly, a polynucleotide encoding a protein comprising the amino acid sequence of SEQ ID NO: 2, SEQ ID NO: 4, SEQ ID NO: 6, SEQ ID NO: 8, SEQ ID NO: 10 or SEQ ID NO: 12 includes not only a part of or the entire DNA sequence of SEQ ID NO: 1, SEQ ID NO: 3, SEQ ID NO: 5, SEQ ID NO: 7, SEQ ID NO: 9 or SEQ ID NO: 11, but also a DNA sequence encoding the same amino acids, which has a codon having a degeneracy relationship therewith as a DNA sequence. There is an RNA sequence corresponding to the DNA sequence.

An example of the polynucleotide encoding a protein comprising the amino acid sequence of SEQ ID NO: 2, SEQ ID NO: 4, SEQ ID NO: 6, SEQ ID NO: 8, SEQ ID NO: 10 or SEQ ID NO: 12 is a polynucleotide comprising the nucleotide sequence of SEQ ID NO: 1, SEQ ID NO: 3, SEQ ID NO: 5, SEQ ID NO: 7, SEQ ID NO: 9 or SEQ ID NO: 11.

In the present specification, whether or not a certain protein is functionally equivalent to the protein consisting of the amino acid sequence of SEQ ID NO: 2, SEQ ID NO: 4, SEQ ID NO: 6, SEQ ID NO: 8, SEQ ID NO: 10 or SEQ ID NO: 12 can be determined by evaluating a biological phenomenon or functions associated with the expression of the protein consisting of the amino acid sequence of SEQ ID NO: 2, SEQ ID NO: 4, SEQ ID NO: 6, SEQ ID NO: 8, SEQ ID NO: 10 or SEQ ID NO: 12. For example, it can be determined by allowing the certain protein to express by genetic recombination technique and then evaluating whether or not the aforementioned protein functions as a dendritic cell-activating receptor. The protein consisting of the amino acid sequence of SEQ ID NO: 2, SEQ ID NO: 4, SEQ ID NO: 6, SEQ ID NO: 8, SEQ ID NO: 10 or SEQ ID NO: 12 interacts with T cells and has the function of activating dendritic cells. Thus, for the aforementioned evaluation, the following functions can be used as index:
- Function of mediating T cell adhesion (Examples 5, 6, 10 and 11);
- Function of activating dendritic cells by antibody cross-link stimulus (Example 3):
- Function of forming complex with FcRγ chain (Example 4); or
- Combined use of several or all the aforementioned functions.

### [Novel ligand protein and polynucleotide]

Among genes that are specifically expressed on activated T cells as ligands for TARM proteins, which were identified in the present invention, one type of membrane protein gene can be mentioned as a mouse-derived gene. The nucleotide sequence of this gene and the amino acid sequence of a protein encoded by the gene are as shown in SEQ ID NOS: 13 and 14, respectively. In addition, among genes that are specifically expressed on activated T cells as ligands for TARM proteins, one type of membrane protein gene can be mentioned as a human-derived gene. The nucleotide sequence of this gene and the amino acid sequence of a protein encoded by the gene are as shown in SEQ ID NOS: 15 and 16, respectively.

In the ligand protein, an amino acid sequence having 70% or more identity with the amino acid sequence of SEQ ID NO: 14 or SEQ ID NO: 16 can be an amino acid sequence having preferably 80% or more, more preferably 85% or more, further more preferably 90% or more, still further more preferably 95% or more, particularly preferably 98% or more, and most preferably 99% or more identity with the aforementioned amino acid sequence.
In a polynucleotide encoding the ligand protein, a nucleotide sequence having 70% or more identity with the nucleotide sequence of SEQ ID NO: 13 or SEQ ID NO: 15 can be a nucleotide sequence having preferably 80% or more, more preferably 85% or more, further more preferably 90% or more, still further more preferably 95% or more, particularly preferably 98% or more, and most preferably 99% or more identity with the aforementioned nucleotide sequence.

If the amino acid sequence of SEQ ID NO: 14 or SEQ ID NO: 16 is given, a nucleotide sequence encoding it can easily be determined. Thus, various nucleotide sequences encoding the amino acid sequence of SEQ ID NO: 14 or SEQ ID NO: 16 can be selected.

Accordingly, a polynucleotide encoding a protein comprising the amino acid sequence of SEQ ID NO: 14 or SEQ ID NO: 16 includes not only a part of or the entire DNA sequence of SEQ ID NO: 13 or SEQ ID NO: 15, but also a DNA sequence encoding the same amino acids, which has a codon having a degeneracy relationship the with as a DNA sequence. An RNA sequence corresponds to such a DNA sequence.

An example of the polynucleotide encoding a protein comprising the amino acid sequence of SEQ ID NO: 14 or SEQ ID NO: 16 is a polynucleotide comprising the nucleotide sequence of SEQ ID NO: 13 or SEQ ID NO: 15.

In the present specification, whether or not a certain protein is functionally equivalent to the protein consisting of the amino acid sequence of SEQ ID NO: 14 or SEQ ID NO: 16 can be determined by evaluating a biological phenomenon or functions associated with the expression of the protein consisting of the amino acid sequence of SEQ ID NO: 14 or SEQ ID NO: 16. For example, it can be determined by allowing the certain protein to express by genetic recombination technique and then evaluating whether or not the aforementioned protein functions as a ligand on a T cell for a dendritic cell-activating receptor. The protein consisting of the amino acid sequence of SEQ ID NO: 14 or SEQ ID NO: 16 binds to a TARM protein. Thus, for the aforementioned evaluation, the function of binding to such a TARM protein (Example 12) can be used as an index:
The ligand protein has a single transmembrane region, and it is expressed on the cell surface in a direction wherein the N-terminal side thereof can be located in the extracellular space. Accordingly, using the aforementioned protein, antibodies against the aforementioned protein can be produced.
The protein may comprise a polypeptide consisting of at least 6 amino acid residues or all of amino acid sequence of amino acids 1 to 159 of SEQ ID NO: 14 or amino acids 1 to 158 of SEQ ID NO: 16. The aforementioned protein contains a portion corresponding to the extracellular region of the amino acid sequence of a TARM-L protein, and thus it can be used as an antigen for producing antibodies against the aforementioned protein.
The ligand protein may be used for producing antibodies against the aforementioned ligand protein.

### [Antibody]

The antibody according to the present invention can specifically recognize a TARM protein or a TARM-L protein. Accordingly, it is preferable that a TARM protein or a TARM-L protein used for obtaining the antibody according to the present invention should have the antigenecity of TARM or TARM-L. Such a TARM protein or a TARM-L protein includes a protein having an amino acid sequence of a TARM protein or a TARM-L protein in which one or more amino acid residues are deleted, inserted, substituted, or added. It has been known that such a protein maintains the same biological activity as that of the original protein (Mark et al. (1984) Proc. Natl. Acad. Sci. USA 81: 5662-6; Zoller and Smith (1982) Nucleic Acids Res. 10: 6487-500; Wang et al. (1984) Science 224: 1431-3; Dalbadie-McFarland et al. (1982) Proc. Natl. Acad. Sci. USA 79: 6409-13). A method of producing a certain protein by deleting, inserting, substituting or adding one or more amino acids with respect to the original protein, while maintaining the antigenecity of the original protein, has been known. For example, a polynucleotide encoding a mutant protein is prepared by site-directed mutagenesis, and a protein is then allowed to express, as appropriate (Molecular Cloning, A Laboratory Manual 2nd ed., Cold Spring Harbor Press (1989); Current Protocols in Molecular Biology, John Wiley & Sons, (1987-1997), Section 8.1-8.5; Hashimoto-Goto et al. (1995) Gene 152: 271-5; Kinkel (1985) Proc. Natl. Acad. Sci. USA 82: 488-92; Kramer and Fritz (1987) Method. Enzymol 154: 350-67; Kunkel (1988) Method. Enzymol. 85: 2763-6).

The antibody according to the present invention also includes antibodies specific for a part of the TARM protein or TARM-L protein.

That is to say, such a TARM protein or a TARM-L protein used for obtaining the antibody according to the present invention includes not only a polypeptide having the full-length amino acid sequence of the TARM protein or the TARM-L protein, but also a polypeptide fragment having at least 6 amino acid residues (for example, 6, 8, 10, 12, 15 or more amino acid residues) of the TARM protein or the TARM-L protein. In the present specification, the type of the polypeptide fragment of the TARM protein or the TARM-L protein is not particularly limited, as long as it has the antigenecity of the TARM protein or the TARM-L protein.

A preferred polypeptide fragment may be a polypeptide fragment such as the amino terminal or carboxyl terminal of the TARM protein or the TARM-L protein. The antigen determinant site of a polypeptide is estimated by a method of analyzing the hydrophobicity/hydrophilicity on the amino acid sequence of a protein (Kyte-Doolittle (1982) J. Mol. Biol. 157: 105-22) or a method of analyzing a secondary structure (Chou-Fasman (1978) Ann. Rev. Biochem. 47: 251-76). Thereafter, such an antigen determinant site can be confirmed using a computer program (Anal. Biochem. 151: 540-6 (1985)), or by applying means such as a PEPSCAN method of synthesizing a short peptide and confirming the antigenecity thereof (Japanese Patent Laid-Open Publication No. 500684/1985).

The antibody according to the present invention is preferably antibodies that have an influence on the functions of the TARM protein or the TARM-L protein. For example, the meanings of the expression "having an influence on the functions of the TARM protein" include: activation of dendritic cells by the cross-link stimulus of the TARM protein by the antibody according to the first embodiment of the present invention (Example 3); inhibition of adhesion of T cells to the TARM protein by binding the aforementioned antibody to the TARM protein (Examples 6 and 11); and inhibition of a complex formation between the TARM protein and the FcRγ chain by binding the aforementioned antibody to the TARM protein (Example 4). For example, the meanings of the expression "having an influence on the functions of the TARM-L protein" include inhibition of the binding of the TARM-L protein to the TARM protein by binding the antibody of the second embodiment according to the present invention to the TARM-L protein.

The antibody according to the present invention includes: a monoclonal antibody obtained by using the TARM protein or the TARM-L protein as an antigen and immunizing a mammal such as a mouse with the aforementioned antigen; a chimeric antibody and a humanized antibody produced by genetic recombination; and a human antibody produced using a human antibody-producing transgenic animal or the like. When the antibody according to the present invention is administered as a medicament to a human, a human antibody is preferably used in terms of side effects.

The "human antibody" means an antibody wherein all regions are derived from humans. Such a human antibody can be produced by introducing a human antibody gene into a mouse. Such a human antibody can be produced with reference to the methods described, for example, in Nature Genetics, Vol. 7, pp. 13-21, 1994; Nature Genetics, Vol. 15, pp. 146-156, 1997; Japanese Patent Laid-Open Publication No. 504365/1992; Japanese Patent Laid-Open Publication No. 509137/1995; International Publication WO94/25585; Nature, Vol. 368, pp. 856-859, 1994; and Japanese Patent Laid-Open Publication No. 500233/1994. In addition, such a human antibody can also be produced by a phage display method. It can be produced with reference to the method described in Marks, J. D. et al.: J. Mol. Biol., Vol. 222, pp. 581-597, 1991, for example.

The "humanized antibody" is an antibody produced by transplanting (CDR grafting) only the gene sequence of the antigen-binding site (CDR; complementarity determining region) of a mouse antibody into a human antibody gene. Such a humanized antibody can be produced with reference to the methods described in Japanese Patent Laid-Open Publication No. 506458/1992 and Japanese Patent Laid-Open No. 296890/1987, for example.

The "chimeric antibody" is an antibody produced by ligating the variable region of a mouse antibody to the constant region of a human antibody. Specifically, a mouse is immunized with an antigen, and an antibody variable region (V region) that binds to the antigen is cut out of the gene of the mouse monoclonal antibody. The thus obtained V region is then allowed to ligate to an antibody constant region (C region) gene derived from human bone marrow, so as to produce a chimeric antibody. Such a chimeric antibody can be produced with reference to the method described in Japanese Patent Publication No. 73280/1991, for example.

The monoclonal antibody according to the present invention can be obtained using a method well known to persons skilled in the art (e.g. "Current Protocols in Molecular Biology," John Wiley & Sons (1987)), Antibodies: A Laboratory Manual, Ed. Harlow and David Lane, Cold Spring Harbor Laboratory (1988)).

As an immunogen, a fragment of the TARM protein or the TARM-L protein can be used. Otherwise, an antigen synthesized based on the aforementioned amino acid sequence can also be used. Such an antigen may be used in the form of a complex with a carrier protein. In order to prepare a complex of an antigen with a carrier protein, various types of coupling agents can be used. Glutaraldehyde, carbodiimide, a maleimide active ester, and the like can be used. The carrier protein may be commonly used products such as bovine serum albumin, thyroglobulin, or hemocyanin and is generally performed coupling at a ratio of 1 to 5.

Examples of an animal to be immunized include a mouse, a rat, a rabbit, a guinea pig, and a hamster. An inoculation method includes subcutaneous administration, intramuscular administration, and an intraperitoneal administration. For administration, an antigen may be mixed with Freund's complete adjuvant or Freund's incomplete adjuvant. Administration is generally carried out once every 2 to 5 weeks.

Antibody-producing cells obtained from the spleen or lymph node of the immunized animal are subjected to cell fusion with myeloma cells, and they are isolated as hybridomas. As such myeloma cells, cells derived from a mouse, a rat, a human or the like can be used, and are preferably derived from the same species as the antibody-producing cells. However, there are also cases where such a cell fusion can be carried out even between the cells of different species.

The cell fusion can be carried out by a known method such as the method described in Nature, 256, 495, 1975.

Examples of a fusion promoter include polyethylene glycol and Sendai virus. In general, cell fusion can be carried out by allowing antibody-producing cells to react with myeloma cells using polyethylene glycol (mean molecular weight: 1,000 to 4,000) having a concentration of approximately 20% to 50% at a temperature between 20°C and 40°C, and preferably between 30°C and 37°C, at a ratio of the number of the antibody-producing cells to the number of the myeloma cells that is generally approximately 1 : 1 to 10 : 1, for approximately 1 to 10 minutes.

Various types of immunochemical methods can be used for screening antibody-producing hybridomas. Examples of such an immunochemical method include: an ELISA method using a microplate coated with the TARM protein or the TARM-L protein; an EIA method using a microplate coated with an anti-immunoglobulin antibody; and an immunoblot method involving electrophoresing a sample containing the TARM protein and then using a nitrocellulose membrane.

In addition, for screening such antibody-producing hybridomas, a method of screening the hybridomas based on whether or not the aforementioned antibody has an influence on the functions of the TARM protein or the TARM-L protein, can be applied, instead of the aforementioned immunochemical method. Antibody-producing hybridomas can be screened based on the influence of the antibody of the first embodiment according to the present invention on the functions of the TARM protein, for example, based on whether or not dendritic cells are activated by the cross-link stimulus of the TARM protein by the antibody of the first embodiment according to the present invention (Example 3), or whether or not the function of the TARM protein of mediating the T cell adhesion can be inhibited by binding the aforementioned antibody to the TARM protein (Examples 6 and 11), or whether or not a complex formation between the TARM protein and the FcRγ chain can be inhibited by binding the aforementioned antibody to the TARM protein (Example 4). Antibody-producing hybridomas can also be screened based on the influence of the antibody of the second embodiment according to the present invention on the functions of the TARM-L protein, for example, based on whether or not the function of the TARM protein of binding to the TARM-L protein can be inhibited by binding the antibody of the second embodiment according to the present invention to the TARM-L protein. By this screening method, an antibody that has an influence on the functions of the TARM protein or the TARM-L protein, which is a preferred embodiment of the antibody of the present invention, can be selected. Moreover, this screening method may also be carried out as a secondary screening method, which is performed after the aforementioned immunochemical screening method wherein an antibody-producing hybridoma is selected based on whether or not it produces an antibody that binds to the TARM protein or the TARM-L protein.

Furthermore, cloning is carried out on such a well by, for example, a limiting dilution method, so as to obtain clones. Selection and culture of such hybridomas are generally carried out in a medium for animal cells (e.g. RPMI1640) containing 10% to 20% fetal bovine serum, to which HAT (hypoxanthine, aminopterin and thymidine) is added. The clones thus obtained are transplanted into the peritoneal cavity of SCID mice, to which pristane has previously been administered. 10 to 14 days later, ascites containing a high concentration of monoclonal antibodies is collected, and it can be used as a raw material in purification of antibodies. Otherwise, the aforementioned clones are cultured, and the obtained culture can also be used as a raw material in purification of antibodies.

A monoclonal antibody may be purified by a known immunoglobulin purification method. For example, such purification of a monoclonal antibody can be easily achieved by means such as an ammonium sulfate fractionation method, a PEG fractionation method, an ethanol fractionation method, use of an anion exchanger, or affinity chromatography using a protein A column, a protein G column, and a TARM protein.

The "functional fragment" of the present invention means a part of an antibody (a partial fragment), which specifically recognizes the protein of the present invention. Specific examples of such a functional fragment include Fab, Fab', F(ab')₂, a variable region fragment (Fv), a disulfide-bonded Fv, a single-chain antibody (scFv), and a polymer thereof.

Preferred examples of the antibody of the first embodiment according to the present invention include an antibody against the novel protein of the first embodiment according to the present invention, and a functional fragment thereof.

Such preferred examples of the antibody of the first embodiment according to the present invention also include antibodies against the novel protein of the second embodiment according to the present invention, and a functional fragment thereof.

Such preferred examples of the antibody of the first embodiment according to the present invention further include antibodies against the following proteins:
(ix') a membrane or secretory protein comprising the amino acid sequence of SEQ ID NO: 12;
(x') a membrane or secretory protein which comprises an amino acid sequence of SEQ ID NO: 12 in which one or more amino acids are inserted, substituted or deleted, or one or more amino acids are added to one or both of ends, and which is functionally equivalent to a protein consisting of the amino acid sequence of SEQ ID NO: 12;
(xi') a membrane or secretory protein which is encoded by a polynucleotide which hybridizes under stringent conditions to a polynucleotide which encodes the amino acid sequence of SEQ ID NO: 12, and which is functionally equivalent to a protein consisting of the amino acid sequence of SEQ ID NO: 12; and
(xii') a membrane or secretory protein which comprises an amino acid sequence having 70% or more identity with the amino acid sequence of SEQ ID NO: 12, and which is functionally equivalent to a protein consisting of the amino acid sequence of SEQ ID NO: 12.

A more preferred example of the antibody of the first embodiment according to the present invention is antibodies against a membrane or secretory protein comprising the amino acid sequence of SEQ ID NO: 12, or an amino acid sequence of SEQ ID NO: 12 which contains one or more conservative substitutions, or a functional fragment thereof.

A specific example is a monoclonal antibody produced by a hybridoma deposited under the accession No. FERM BP-10376.

Accordingly, the present invention provides a hybridoma (@TARM#6.11) deposited with the National Institute of Advanced Industrial Science and Technology, International Patent Organism Depositary (AIST Tsukuba Central 6, Higashi 1-1-1, Tsukuba, Ibaraki, 305-8566, Japan), under the accession No. FERM BP-10376 on July 15, 2005.

Another more preferred example of the antibody of the first embodiment according to the present invention is antibodies against a membrane or secretory protein comprising the amino acid sequence of SEQ ID NO: 10 or an amino acid sequence of SEQ ID NO: 10 which contains one or more conservative substitutions, or a functional fragment thereof.

A preferred example of the antibody of the second embodiment according to the present invention is antibodies against a membrane or secretory protein comprising the amino acid sequence of SEQ ID NO: 14 or an amino acid sequence of SEQ ID NO: 14 which contains one or more conservative substitutions, or a functional fragment thereof.

Another preferred example of the antibody of the second embodiment according to the present invention is antibodies against a membrane or secretory protein comprising the amino acid sequence of SEQ ID NO: 16 or an amino acid sequence of SEQ ID NO: 16 which contains one or more conservative substitutions, or a functional fragment thereof.
A more preferred example of the antibody of the second embodiment according to the present invention is antibodies recognizing a polypeptide region that is expressed in the extracellular space of the TARM-L protein, or a functional fragment thereof. An example of such an antibody is antibodies against a protein comprising a polypeptide consisting of at least 6 amino acid residues or all of amino acid sequence of amino acids 1 to 159 of SEQ ID NO: 14 or amino acids 1 to 158 of SEQ ID NO: 16, or a functional fragment thereof.

### [Use of antibody and pharmaceutical composition]

### Autoimmune diseases

Since T cells, which are lymphocytes associated with immune responses, synergistically act with dendritic cells having an antigen-presenting function to such T cells, the T cells play a role in various immune responses (Kroczek, RA., et al. (2004) Current Opinion in Immunology 16: 321-327). As described later in examples, it was revealed that expression of a TARM protein on dendritic cells was enhanced by inflammatory stimulus (Example 2), and that dendritic cells adhered to T cells via the TARM protein (Examples 5 and 10). In addition, it was confirmed that dendritic cells were activated by the TARM protein subjected to cross-link stimulus, and that production of IL-6 was induced (Example 3). It has been reported that excessive production of IL-6 is associated with autoimmune diseases (Ishihara, K., et al. (2002) Cytokine & Growth Factor Reviews 13: 357-368). Moreover, adhesion of the T cells to dendritic cells was significantly suppressed by antibodies against the TARM protein (Examples 6 and 11).

Furthermore, in the after-mentioned examples, it was confirmed that the antibody according to the present invention actually had a therapeutic effect on a collagen-induced arthritis model (Example 7). The collagen-induced arthritis model is a model of rheumatoid arthritis that is an autoimmune disease.

Accordingly, the antibody of the first embodiment according to the present invention is useful for the treatment of autoimmune diseases.

An example of such autoimmune diseases is rheumatoid arthritis.

Likewise, it is considered that adhesion of T cells to dendritic cells is suppressed by antibodies against a TARM-L protein. Accordingly, the antibody of the second embodiment according to the present invention is useful for the treatment of autoimmune diseases.

The present invention provides use of the antibody according to the present invention for producing a therapeutic agent for treating autoimmune diseases.

The present invention provides a method for treating autoimmune diseases comprising the step of administering a therapeutically effective amount of the antibody according to the present invention to mammals including a human.

### Agent for inhibiting T cell adhesion

As described later in the examples, adhesion of T cells to dendritic cells was significantly suppressed by antibodies against a TARM protein (Examples 6 and 11). Accordingly, the antibody of the first embodiment according to the present invention can be used as an agent for inhibiting T cell adhesion.

Likewise, it is considered that adhesion of T cells to dendritic cells is suppressed by antibodies against a TARM-L protein. Accordingly, the antibody of the second embodiment according to the present invention can be used as an agent for inhibiting T cell adhesion.

In the present specification, the term "adhesion of T cells" means adhesion of T cells to dendritic cells, namely, the binding of a TARM protein expressed on dendritic cells to a TARM-L protein expressed on T cells. By inhibiting the binding of a TARM protein expressed on dendritic cells to a TARM-L protein expressed on T cells using the agent for inhibiting T cell adhesion according to the present invention, an immune response generated as a result of the interaction between dendritic cells and T cells, such as activation, growth and differentiation of the dendritic cells and T cells, and production of cytokine/chemokine can be suppressed.

### Pharmaceutical composition

The administration route of the antibody according to the present invention is not particularly limited. The aforementioned antibody can be administered to mammals including a human by oral administration or parenteral administration (e.g. intravenous injection, intramuscular injection, subcutaneous administration, rectal administration, percutaneous administration, and local administration). Among them, a parenteral administration, and in particular, intravenous injection is preferable.

The dosage form for oral administration and parenteral administration and the production method thereof are well known to persons skilled in the art. The dosage form for oral administration and parenteral administration can be produced by a conventional process, for example, by mixing the antibody according to the present invention, for example, with a pharmaceutically acceptable carrier.

As such a pharmaceutically acceptable carrier, a substance, which is commonly used in the drug formulation field and does not react with the antibody according to the present invention, is used. Pharmaceutically acceptable carriers include, for example, a commonly used excipient, binder, disintegrator, lubricant, coloring agent, and flavoring agent; and, as necessary, a stabilizer, an emulsifier, an absorption promoter, a surfactant, a pH adjuster, an antiseptic, an antioxidant, an extender, a moistening agent, a surface activator, a dispersant, a buffer, a preservative, a solubilizer, and a soothing agent, and may be formulated according to a conventional method by mixing ingredients commonly used as raw materials for pharmaceutical preparations.

Examples of a dosage form for parenteral administration include injectable preparations (e.g. a drip injection product, an intravenous injection product, an intramuscular injection product, a subcutaneous injection product, and a percutaneous injection product), external preparations (e.g. an ointment, a cataplasm, a lotion), a suppository, an inhalant, eye drops, an eye ointment, nasal drops, ear drops, and a liposomal agent.

An injectable preparation is prepared by dissolving the antibody according to the present invention into distilled water used for injections, for example. A solubilizer, a buffer, a pH adjuster, an isotonizing agent, a soothing agent, a preservative, a stabilizer, etc. can be added to such an injectable preparation, as necessary. In addition, such a injectable preparation can be produced in the form of a freeze-dried product, which will be prepared when used.

Examples of a dosage form for oral administration include solid and liquid dosage forms such as a tablet, a coated tablet, a pill, a parvule, a granule, a powder, a capsule, a syrup, an emulsion, a suspension, an injection, or a lozenge.

The pharmaceutical composition according to the present invention may further contain other therapeutically effective agents. Moreover, components such as a blood flow promoter, a germicide, an antiphlogistic, a cell activator, vitamins, amino acid, a moisturizer, or a keratolytic drug may also be added, as necessary. At the time, the ratio of the active ingredient to the carrier can be changed within a range of 1 to 90% by weight.

A dose of the antibody according to the present invention can be determined by a clinician based on various factors such as an administration route, the type of disease, the degree of symptoms, the age, sex and body weight of a patient, the severity of disease, pharmaceutical findings such as pharmacokinetics and toxicologic characteristics, the presence or absence of use of a drug delivery system, and the possibility of being administered as a portion of the combination with other agents, and may be generally 1 to 5000 mg/day, preferably 10 to 2000 mg/day, and more preferably 50 to 2000 mg/day, for oral administration, and 1 to 5000 mg/day, preferably 5 to 2000 mg/day, and more preferably 50 to 2000 mg/day, for injection administration, each per adult (weight 60 kg), which are administered once or several times per day. When it is administered to a child, the dose may be smaller than that administered to an adult. An administration method, which is actually applied, may be changed by decision of a clinician, and thus the applied dose may be departed from the aforementioned range.

### [Screening method]

### Method for screening for a substance that inhibits adhesion of a T cell to a TARM protein

According to the screening method of the first embodiment of the present invention, there is provided a screening method for screening for a substance that inhibits adhesion of a T cell to a TARM protein.

The TARM protein is expressed on dendritic cells and is associated with an interaction that is involved in the immune response between dendritic cells and T cells. In addition, as a result of cross-link stimulus that is added to the TARM protein, the production of IL-6 that may cause the autoimmune disease can be induced. Accordingly, the screening method of the first embodiment of the present invention can be used for screening for a substance that inhibits adhesion of a T cell to the TARM protein, and can preferably be used for screening for a substance useful for the treatment of the autoimmune disease, and more preferably rheumatoid arthritis.

The screening method of the first embodiment according to the present invention may further comprise the step of (c) comparing the binding activity in the presence of a test substance with that in the absence of a test substance, after step (b).

In step (c), when the binding activity in the presence of a test substance is lower than that in the absence of a test substance, and preferably when it is less than 50%, it can be determined that the test substance inhibits the binding of the T cells to the protein according to the present invention.

The term "contacting" in step (a) is not particularly limited, as long as a TARM protein is allowed to directly come into contact with T cells. For example, it can be carried out by a method of adding the labeled T cells to a plate on which the TARM protein has been immobilized, or by a method of adding the labeled TARM protein to a plate containing T cells.

The T cells are preferably activated T cells, and more preferably activated Th2 cells.

In step (b), the binding activity can be measured by a known method. For example, the labeled T cells are added to a plate on which a TARM protein has been immobilized, and they are then cultured for a certain period of time. Thereafter, unadhered cells are eliminated by washing or the like, and the level of the adhered cells is then measured, thereby measuring the binding activity.

For the aforementioned labeling, a radioisotope, an enzyme, a fluorescent substance (including a fluorescent protein), a luminescent substance, etc. can be used, for example. Examples of a radioisotope used herein include [³H], [¹⁴C], [¹²⁵I], and [³⁵S]. Examples of an enzyme used herein include β-galactosidase, alkaline phosphatase, peroxidase, and luciferase. Examples of a fluorescent substance used herein include fluorescein isothiocyanate, BODIPY, and Calcein-AM (Dojindo Laboratories). Also, as a fluorescent protein, GFP and the like can be used. With regard to such enzymes and fluorescent proteins, the gene thereof can be introduced into a cell and can be then expressed therein. Examples of a luminescent substance used herein include luciferin and lucigenin. In some cases, a biotin-avidin system can be used to allow the aforementioned ligand to bind to a labeling substance.

Moreover, unlabeled T cells are added, and the adhered T cells can be then detected by an antibody that is specific for the T cells, such as an anti-CD3 antibody, or by an antibody that is specific for a helper T cell such as an anti-CD4 antibody.

With regard to binding activity, the added cells have previously been measured, and it can be expressed in the form of the ratio of the adhered cells to the added cells.

### Method for screening for a substance that inhibits activation of a dendritic cell

According to the screening method of the second embodiment of the present invention, there is provided a screening method for screening for a substance that inhibits activation of a dendritic cell.

Dendritic cells can be activated by a TARM protein subjected to cross-link stimulus (Examples 3 and 4). Accordingly, a dendritic cell system that has been subjected to cross-link stimulus with a TARM antibody can be used for screening for a substance that inhibits activation of the dendritic cells.

As stated above, it was demonstrated that the autoimmune disease is caused by activation of dendritic cells. Thus, the method according to the present invention for screening for a substance that inhibits activation of dendritic cells can be used for screening for a substance useful for the treatment of, preferably the autoimmune disease, and more preferably rheumatoid arthritis.

When cross-link stimulus is given to a TARM protein that is expressed on dendritic cells, the dendritic cells become activated. At that time, the TARM protein forms a complex with the FcRγ chain known as a signal-transducing molecule, and the production of IL-6 that causes autoimmune diseases or MCP-1 acting as a chemotactic factor for monocytes is induced. Accordingly, in step (e) of the screening method of the second embodiment according to the present invention, the level of activation of dendritic cells can be measured using, as an index, the amount of IL-6 and/or MCP-1 produced from the dendritic cells. Otherwise, the level of activation of dendritic cells can be measured using, as an index, the expression level of the FcRγ chain in the dendritic cells.

In the screening method of the second embodiment according to the present invention, when the level of activation of dendritic cells is measured using, as an index, the amount of IL-6 and/or MCP-1 produced from the dendritic cells, the screening method may further comprise the step of (f-1) comparing the amount of IL-6 and/or MCP-1 produced in the presence of a test substance with that of IL-6 and/or MCP-1 produced in the absence of a test substance, after step (e). In step (f-1), when the amount of IL-6 and/or MCP-1 produced in the presence of a test substance is lower than that of IL-6 and/or MCP-1 produced in the absence of a test substance, and preferably when it is less than 50%, it can be determined that the test substance inhibits activation of the dendritic cells.

In the screening method of the second embodiment according to the present invention, when the level of activation of dendritic cells is measured using, as an index, the expression level of the FcRγ chain in the dendritic cells, the screening method may further comprise the step of (f-2) comparing the expression level of the FcRγ chain in the presence of a test substance with that of the FcRγ chain in the absence of a test substance, after step (e).

In step (f-2), when the expression level of the FcRγ chain in the presence of a test substance is lower than that of the FcRγ chain in the absence of a test substance, and preferably when it is less than 50%, it can be determined that the test substance inhibits activation of the dendritic cells.

In step (d), the term "contacting" is not particularly limited, as long as a TARM protein on dendritic cells is subjected to cross-link stimulus with the antibody according to the present invention. For example, it can be carried out by culturing the dendritic cells in a medium that contains the antibody according to the present invention.

In step (e), the amount of a protein produced or the expression level can be measured according to a known method. A commercially available kit can also be used.

### Method for screening for a substance that inhibits complex formation between a TARM protein and the FcRγ chain

According to the screening method of the third embodiment according to the present invention, there is provided a method for screening for a substance that inhibits a complex formation between a TARM protein and the FcRγ chain.

The TARM protein is expressed on dendritic cells and forms a complex with the FcRγ chain that has been well known as a signal-transducing molecule. Moreover, it has been suggested that the FcRγ chain forms a complex with the TARM protein, so that the expression thereof on the cell surface is increased. Accordingly, the screening method according to the present invention can be used for screening for a substance that inhibits a complex formation between the TARM protein and the FcRγ chain, and can be preferably used for screening for a substance useful for the treatment of, preferably autoimmune diseases, and more preferably rheumatoid arthritis.

The screening method according to the present invention may further comprise the step of (i) comparing the expression level of the FcRγ chain in the presence of a test substance with that of the FcRγ chain in the absence of a test substance, after step (h).

In step (i), when the expression level of the FcRγ chain in the presence of a test substance is lower than that of the FcRγ chain in the absence of a test substance, and preferably when it is less than 50%, it can be determined that the test substance inhibits a complex formation between the protein according to the present invention and the FcRγ chain.

In step (g), the term "contacting" is not particularly limited, as long as dendritic cells on which a TARM protein and the FcRγ chain have been expressed are allowed to directly come into contact with the antibody according to the present invention. For example, it can be carried out by culturing the dendritic cells in a medium that contains the antibody according to the present invention.

In step (h), the expression level can be measured according to a known method. For example, the expression level can be measured using flow cytometry.

In the present specification, examples of the "test substance" include a synthetic low-molecular-weight compound, a protein, a synthetic peptide, a purified or partially purified polypeptide, an antibody, a bacteria-releasing substance (including a bacterial metabolite), and a nucleic acid (antisense, ribozyme, RNAi, etc.). Preferred examples include a compound or a salt thereof, or a solvate thereof (e.g. a hydrate), but examples are not limited thereto. The "test substance" may be either a novel substance or a known substance.

### Examples

The present invention will be described in detail in the following examples. However, the examples described below are not intended to limit the scope of the present invention. In the examples, the "TARM protein" and "TARM-L protein" may be simply referred to as "TARM" and "TARM-L" at times, respectively. Moreover, the mouse-derived TARM protein and human-derived TARM protein may be simply referred to as "mTARM" and "hTARM" at times, respectively.

### [Example 1] Isolation of mouse TARM gene and expression analysis

### (1) Isolation of mTARM gene

CD4 T cells separated from mouse spleen were differentiated into Th1 or Th2 by *in vitro* culture. A cDNA fragment to be used as a driver or a tester was prepared from Th1 or Th2. Thereafter, Blast search was carried out using the sequence of cDNA fragments obtained during a step of conducting a high-sensitivity subtraction (N-RDA) method. As a result, a gene encoding a cell membrane protein having unknown functions (GenBank^{™} accession No. NM_177363) was obtained.

The following primers were designed based on the sequence of GenBank^{™} (NM_177363), and the expression of the mTARM gene in various organs of a mouse was analyzed.
mTARM F1: GTGACTTTGCAGTGCCAGAA (SEQ ID NO.: 17)
mTARM R1: TGCACAGGAGTTGAGTGTCC (SEQ ID NO.: 18)

Single-stranded cDNA was synthesized from total RNA of each organ (Promega) using RNA PCR kit (TAKARA). Using such single-stranded cDNA as a template, real-time PCR was carried out using ABI7700 (Applied Biosystems). The PCR was carried out using a reaction solution with the following composition (12.5 µl of QuantiTect SYBR Green PCR Master Mix (QIAGEN), 0.25 µl of uracil DNA glycosylase (Invitrogen), 0.125 µl of 100 µM mTARM F primer, 0.125 µl of 100 µM mTARM R primer, 2.5 µl of template cDNA (10 fold diluted), and 7.25 µl of distilled water). For such PCR, after the treatment at 94°C for 10 minutes, a reaction cycle consisting of 94°C-30 seconds and 60°C-1 minute was repeated 35 times. As a result, it was found that mTARM was expressed in kidney.

Thus, using the total RNA of the kidney, 5'-RACE (Rapid Amplification of cDNA Ends) and 3'-RACE were carried out to attempt to determine the full-length gene sequence of mTARM.

First, double-stranded cDNA was synthesized from the total RNA of the mouse kidney using cDNA synthesis kit (TAKARA), and cDNA was then purified using Qiaquick PCR purification kit (QIAGEN). Subsequently, an ad29 adapter (a product obtained by annealing ad29S (acatcactccgt; SEQ ID NO: 19) and ad29A (acggagtgatgtccgtcgacgtatctctgcgttgatacttcagcgtagct; SEQ ID NO: 20)) was added thereto, so as to produce a template of RACE.

1^{st} PCR was carried out using a reaction solution with the following composition (5 µl of 10 x ExTaq buffer, 4 µl of 2.5 mM dNTP, 0.25 µl of ExTaq, 0.5 µl of 100 µM primer (5'PCR4), 0.5 µl of 100 µM Gene specific primer, 1 µl of ad29 adapter-added cDNA (25 fold diluted), and 38.75 µl of distilled water).
The following sequences were used as primers.
5'PCR4: AGCTACGCTGAAGTATCAACGCAGAG (SEQ ID NO.: 21)
mTARM_RACE_5'_4: CTTCTGGCACTGCAGAGTCACCCT (SEQ ID NO.: 22), or
mTARM_RACE_3'_4: GGAGAGTACACCTGTGAATACTAC (SEQ ID NO.: 23)
For such PCR, after the treatment at 94°C for 5 minutes, a reaction cycle consisting of 94°C-30 seconds, 65°C-1 minute, and 72°C-5 minutes was repeated 30 times. Finally, a reaction was carried out at 72°C for 5 minutes.

2^{nd} PCR was carried out using a reaction solution with the following composition (5 µl of 10 x ExTaq buffer, 4 µl of 2.5 mM dNTP, 0.25 µl of ExTaq, 0.5 µl of 100 µM primer (5'PCR1), 0.5 µl of 100 µM Gene specific primer, 1 µl of the 1^{st} PCR product (100 fold diluted), and 38.75 µl of distilled water).
The following sequences were used as primers.
5'PCR1: GTATCAACGCAGAGATACGTCGACGG (SEQ ID NO.: 24)
mTARM_RACE_5'_3: TCCACCTGCGGTCACTGTACCCCT (SEQ ID NO.: 25), or
mTARM_RACE_3'_3: CTACAGAAAAGCATCCCCCCACATCCTTTC (SEQ ID NO.: 26)
   For such PCR, after the treatment at 94°C for 5 minutes, a reaction cycle consisting of 94°C-30 seconds, 65°C-30 seconds, and 72°C-5 minutes was repeated 25 times. Finally, a reaction was carried out at 72°C for 5 minutes. The amplified cDNA fragment was cloned into pCR2.1 (Invitrogen), and the nucleotide sequence thereof was determined using ABI3100 Sequence Analyzer (Applied Biosystems).

As a result, 2 types of cDNAs were obtained in 5'RACE, and 3 types of cDNAs were obtained in 3'RACE, and thus the presence of splicing isoforms was clarified.

Using nucleotide sequence information obtained by RACE, primers for amplifying splicing isoforms were designed. Double-stranded cDNA was synthesized from the total RNA of mouse bone marrow using cDNA synthesis kit (TAKARA), and cDNA was then purified using Qiaquick PCR purification kit (QIAGEN). PCR was carried out using a reaction solution with the following composition (5 µl of 10 x ExTaq buffer, 4 µl of 2.5 mM dNTP, 0.25 µl of ExTaq, 0.5 µl of 100 µM 5' primer, 0.5 µl of 100 µM 3' primer, 1 µl of cDNA (25 fold diluted), and 38.75 µl of distilled water).
The following sequences were used as primers.
mTARM_5'UTR: GCTGATAGTAGACCTGCTGAAGAC (SEQ ID NO.: 27)
mTARM_3'UTR-1: GTCCAGATATGTCCAGGCCTCTG (SEQ ID NO.: 28), or
mTARM_3'UTR-2: TTCAGTTATTTTACCAGGGTTTA (SEQ ID NO.: 29)
For the PCR, after the treatment at 94°C for 5 minutes, a reaction cycle consisting of 94°C-30 seconds, 65°C-30 seconds, and 72°C-5 minutes was repeated 35 times. Finally, a reaction was carried out at 72°C for 5 minutes. With 2 types of primers, 6 types of splicing isoforms could be confirmed. As a result, amplification products were obtained in 5 types out of 6 conbineaions of putative splicing isoforms. The amplified cDNA fragment was cloned into pCR2.1 (Invitrogen), and the nucleotide sequence thereof was determined using ABI3100 Sequence Analyzer.

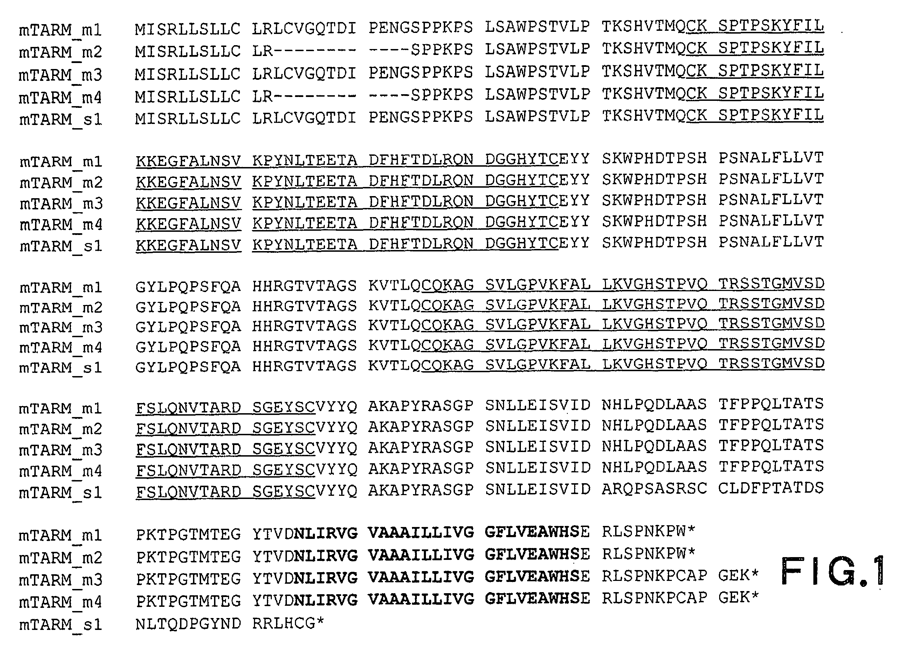

As a result, it was revealed that splicing isoforms encoding 4 types of membrane-bound TARM genes (ml, m2, m3 and m4) and one type of secretory type TARM gene (s1) were present (Figure 1).

### (2) Analysis of expression of mTARM genes

Expression of the mTARM genes in normal mouse tissues was analyzed. As described above, since the splicing isoforms were present, 3 types of primer sets were designed.
Set 1 (Primers were designed such that they could specifically amplify the m1 and m2 isoforms.)
   mTARM_qF2: TCTGTGATAGACAACCATCT (SEQ ID NO.: 30)
   mTARM_qR2: GTCATTGTACCCGGGGTCTT (SEQ ID NO.: 31)
Set 2 (Primers were designed such that they could specifically amplify the m3 and m4 isoforms.)
   mTARM_qF4: ATGACAGAAGGCTACACTGTGGATAA (SEQ ID NO.: 32)
   mTARM_qR3: TCATTTTTCTCCTGGGGCAC (SEQ ID NO.: 33)
Set 3 (Primers were designed such that they could specifically amplify the s1 isoform.)
   mTARM_qF3: GATCTCTGTGATAGATGCAAG (SEQ ID NO.: 34)
   mTARM_qR2: GTCATTGTACCCGGGGTCTT (SEQ ID NO.: 35)

Using RNA PCR kit (TAKARA), single-stranded cDNA was synthesized from total RNA prepared from each mouse organ using RNeasy mini kit (QIAGEN) or from the purchased total RNA of each organ (Promega). Using the thus synthesized single-stranded cDNA as a template, real-time PCR was carried out using ABI7700. The PCR was carried out using a reaction solution with the following composition (12.5 µl of QuantiTect SYBR Green PCR Master Mix (QIAGEN), 0.25 µl of uracil DNA glycosylase (Invitrogen), 0.125 µl of 100 µM F primer, 0.125 µl of 100 µM R primer, 2.5 µl of template cDNA (10 fold diluted), and 7.25 µl of distilled water).

For such PCR, after the treatment at 94°C for 10 minutes, a reaction cycle consisting of 94°C-30 seconds and 60°C-1 minute was repeated 35 times.

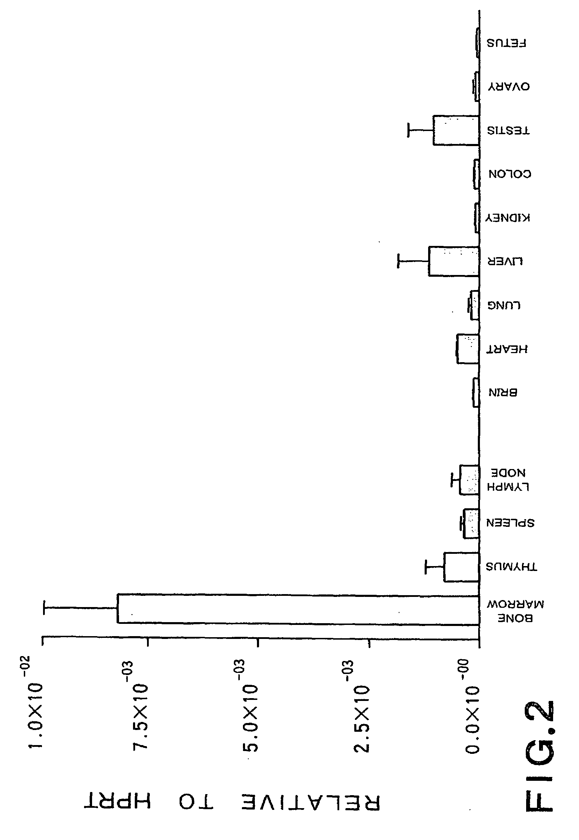

As a result, it was found that mTARM was strongly expressed in bone marrow (Figure 2).

Subsequently, expression of mTARM in various types of cells was analyzed.

Using RNeasy mini kit (QIAGEN), total RNA was prepared from each of cells separated and purified from mouse spleen, cells cultured *in vitro,* and various types of cell lines. Thereafter, single-stranded cDNA was synthesized from the total RNA using RNA PCR kit (TAKARA). Using the single-stranded cDNA as a template, real-time PCR was carried out using ABI7700 in the same manner as that for expression analysis in normal mouse tissues.

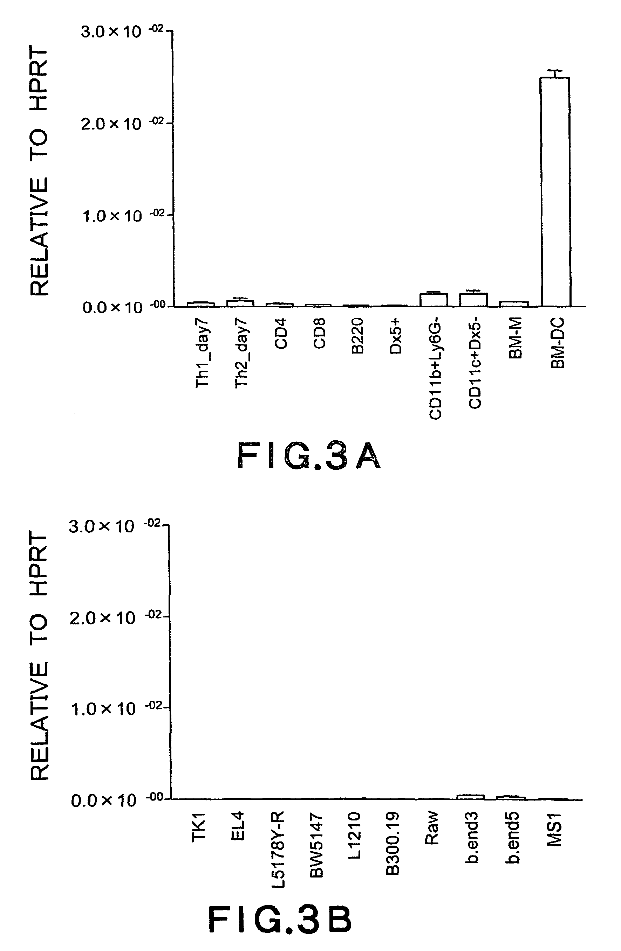

As a result, it was found that mTARM was strongly expressed in bone marrow-derived dendritic cells (Figure 3).

### [Example 2] Preparation of antibody against mouse TARM and expression analysis

### (1) Preparation of mTARM-expressing cells

An mTARM gene expression vector was prepared as follows.
Primers were designed based on the nucleotide sequence of isoform m1.
mTARM F2: cgcgtcgacgccaccATGATCTCTAGGCTCCTTTCCCTT (SEQ ID NO.: 36)
mTARM R2: gcgggcggccgcTTACCAGGGTTTATTTGGAGACAG (SEQ ID NO.: 37)

Using RNA PCR kit (TAKARA), single-stranded cDNA was synthesized from the total RNA of bone marrow. The single-stranded cDNA thus synthesized was used as a template. PCR was carried out using a reaction solution with the following composition (5 µl of 10 x buffer, 4 µl of 2.5 mM dNTP, 0.5 µl of Pyrobest polymerase (TAKARA), 0.5 µl each of 100 µM primers, 1 µl of cDNA, 2.5 µl of DMSO, and 36 µl of distilled water). For such PCR, after the treatment at 94°C for 5 minutes, a reaction cycle consisting of 94°C-30 seconds, 65°C-30 seconds, and 72°C-5 minutes was repeated 35 times. Finally, a reaction was carried out at 72°C for 2 minutes. The amplified cDNA was cloned into pBlueScriptII SK(+) (Stratagene), and the nucleotide sequence thereof was then confirmed using ABI3100 Sequence Analyzer. The obtained cDNA fragment was inserted into an expression vector pMXII IRES EGFP (Oncogene (2000) 19(27): 3050-3058), so as to prepare an mTARM gene expression vector.

Recombinant retrovirus was prepared as follows.

3 x 10⁶ 293/EBNA-1 cells (Invitrogen) were suspended in a medium (D-MEM/10% FBS), were put in a 10cm dish, and were then cultured in a CO₂ incubator for 24 hours. On the following day, the medium was changed with a fresh one, and a transfection solution prepared as described below was then added thereto, so as to carry out transfection. The transfection solution was prepared by adding 600 µl of OPTI-MEM (GIBCO BRL) and 24 µl of TransIT LT1 (TaKaRa) into a 5ml tube to mix them, then incubating the mixture at room temperature for 5 minutes, and then adding 9 µg of an expression vector and 9 µg of pCL-Eco (Imgenex) used as a packaging vector to the reaction mixture, followed by incubating the mixture at room temperature for 5 minutes. 48 hours later, the culture supernatant was recovered, and filtration was then carried out with a 0.45-µm filter, so as to obtain a recombinant virus solution.

B300.19 cells (EMBO J. (1984) 3: 1209-1219) were infected with this recombinant virus as described below, so as to prepare mTARM-expressing cells. The 1 x 10⁶ B300.19 cells were added to a 15ml tube, and they were then centrifuged at 1200 rpm at 25°C for 5 minutes. Thereafter, the culture supernatant was discarded by aspiration. A solution obtained by adding the mixture of 2 µl of polybrene (10 mg/ml) and 2 µl of 55 µM 2-mercaptoethanol to 2 ml of the recombinant virus solution was added to the cells. The obtained mixture was then centrifuged at 2500 rpm at 30°C for 2 hours, so that the cells were infected with the recombinant virus. After completion of the infection, the recombinant virus solution was discarded, and a medium (RPMI-1640/10% FBS/55 µM 2-mercaptoethanol) was added thereto, followed by culture. EGFP-positive cells were separated by cell sorting, so as to obtain mTARM-expressing cells.

### (2) Preparation of chimeric protein fused mTARM extracellular region to SEAP or Fc

First, a pcDNA3.1(+)-SEAP(His)₁₀-Neo vector was prepared as follows.

The endogenous SalI site of a pcDNA3.1(+)-Neo vector (Invitrogen) was digested with SalI, followed by blunting and re-ligation, so that it was deleted. The cDNA fragment of SEAP(His)₁₀ was amplified by PCR using pDREF-SEAP His₆-Hyg (J. Biol. Chem., 1996, 271, 21514-21521) as a template and also using a HindIII-added 5' primer and a XhoI-added 3' primer. The obtained cDNA fragment was digested with HindIII and XhoI, and it was then inserted into the pcDNA3.1(+)-Neo vector in which the SalI site had been deleted.

Subsequently, the extracellular region of mTARM was amplified by PCR using mTARM full-length cDNA as a template and also using a SalI-added 5' primer (mTARM_F2: (SEQ ID NO: 36)) and a NotI-added 3' primer (mTARM_R3: cgcggcggccgcattatccacagtgtagccttctgtcat (SEQ ID NO: 38)). The PCR was carried out in a reaction solution with the following composition (5 µl of 10 x buffer, 4 µl of 2.5 mM dNTP, 0.5 µl of Pyrobest polymerase (TAKARA), 0.5 µl each of 100 µM primers, 1 µl of cDNA, 2.5 µl of DMSO, and 36 µl of distilled water). For such PCR, after the treatment at 94°C for 5 minutes, a reaction cycle consisting of 94°C-30 seconds, 65°C-30 seconds, and 72°C-5 minutes was repeated 35 times. Finally, a reaction was carried out at 72°C for 2 minutes. The amplified cDNA was cloned into pBIueScriptII. SK(+) (Stratagene), and the nucleotide sequence thereof was then determined using ABI3100 Sequence Analyzer. The obtained cDNA fragment was digested with SalI and NotI, and then inserted into the aforementioned expression vector pcDNA3.1(+)-SEAP(His)₁₀-Neo vector, so as to prepare an mTARM-AP expression vector.

Thereby, the mTARM extracellular region is fused through 3 amino acid linker (Ala-Ala-Ala) to secretory-type human placental alkaline phosphatase having a 10-histidine tag (His)₁₀ at the C-terminal thereof to be expressed as a secretory chimeric protein (hereinafter referred to as AP chimeric protein). The obtained AP chimeric protein expression vector was introduced into 293/EBNA-1 cells using TransIT LT1 (TAKARA), and they were then cultured for 4 or 5 days. Thereafter, the culture supernatant was recovered by centrifugation, and the AP chimeric protein secreted into the supernatant was then filtrated with a 0.22-µm filter. Thereafter, Hepes (pH 7.4) and sodium azide were added thereto to final concentrations of 20 mM and 0.02%, respectively, and the obtained product was stored at 4°C. The concentration of the AP chimeric protein was calculated by measuring alkaline phosphatase activity using Aurora AP chemiluminescent reporter gene assay (ICN).

### (3) Preparation of monoclonal antibody against mTARM

For use as an antigen in immunization, first, the mTARM-AP chimeric protein was purified.

Such purification was carried out utilizing the histidine tag existing at the C-terminal of the AP chimeric protein and using His Trap Kit (Amersham Biosciences). A culture supernatant containing the mTARM-AP chimeric protein was added to a 1 ml HiTrap chelating HP column (Amersham Biosciences), followed by washing with a 10 mM imidazole solution. Thereafter, the mTARM-AP chimeric protein was eluted from the column using a 500 mM imidazole solution. The concentration of the mTARM-AP chimeric protein was calculated by the measurement of enzyme activity using Aurora AP chemiluminescent reporter gene assay (ICN) and by protein quantification using Protein Assay kit II (BIO-RAD).

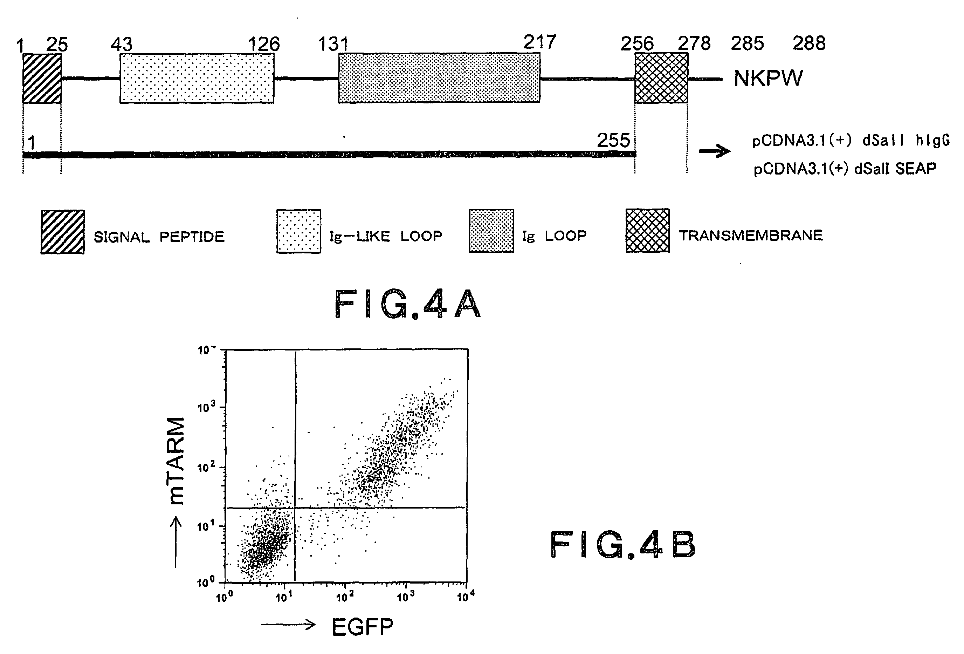

The obtained mTARM-AP chimeric protein was mixed with TiterMax, and was then immunized to a WKY rats (Japan SLC, Inc.). Lymphocytes were isolated from the thus immunized rats. The lymphocytes were mixed with P3 myeloma cells (ATCC), such that the ratio of the P3 myeloma cells to the lymphocytes became 1 : 5. Thereafter, cell fusion was carried out using a PEG1500 solution (Boehringer). Hybridomas were selected in an HAT medium (Invitrogen), and a culture supernatant of the obtained hybridomas was subjected to screening by sandwich ELISA using an mTARM-Fc chimeric protein. Cloning was carried out, and 3 types of clones (#6, #21, and #37) were obtained from positive wells. B300.19 cells, into which mTARM-IRES-EGFP had been introduced, were allowed to react with the anti-mTARM antibodies, followed by FACS analysis. As a result, the generated antibodies reacted only with B300.19 cells in which EGFP was expressed (Figure 4B), and thus the specificity of the anti-mTARM antibodies was confirmed.

Hybridomas producing the obtained anti-mTARM monoclonal antibodies #6, #21, and #37 were inoculated into the peritoneal cavity of nude mice, and ascites was then obtained. Thereafter, antibodies were purified using a Protein G column. The hybridoma producing the anti-mTARM monoclonal antibody #6 was deposited with the National Institute of Advanced Industrial Science and Technology, International Patent Organism Depositary, under the accession No. FERM BP-10376.

### (4) Expression of TARM protein on cultured bone marrow-derived dendritic cells

Using the obtained monoclonal antibody (mAb), expression of a TARM protein on the cell surfaces of the cultured bone marrow-derived dendritic cells was analyzed.

Such cultured bone marrow-derived dendritic cells were prepared by the following method. The bone marrow cells of C57BL/6 male mice (Japan SLC, Inc.) were suspended in a medium (RPMI1640/10% FBS/1 mM sodium pyruvate/55 µM 2-mercaptoethanol) that contained mouse GM-CSF (20 ng/ml) (R & D system) at a concentration of 2 x 10⁶ cells/10 ml. The cells were put in 10-cm non-coated dishes, and were then cultured. 3 days later, 10 ml of medium that contained mouse GM-CSF (20 ng/ml) was added, and the culture was further continued. Further, 3 days later, 10 ml of the culture solution was recovered and then centrifuged. Thereafter, cell aggregates were suspended in 10 ml of fresh medium that contained mouse GM-CSF (20 ng/ml), and the obtained suspension was then returned to the original non-coated 10-cm dishes, followed by culture for 2 days. The immature dendritic cells thus obtained were suspended in a medium that contained LPS (100 ng/ml) (Sigma) at a concentration of 1 x 10⁷ cells/10 ml. The cells were put in 10-cm dishes, and were then cultured for 1 day, so as to obtain mature dendritic cells.

Immature dendritic cells derived from bone marrow were suspended in a FACS buffer (PBS/1% FBS/1 mM EDTA) that contained 5% mouse serum, and they were reacted with antibody #6 or antibody #21 (10 µg/ml). Thereafter, the cells were incubated with PE-labeled donkey anti-rat IgG secondary antibody (Jackson), followed by measurement with FACSCalibur (Becton Dickinson). As a result, both the antibody #6 and antibody #21 showed positive reactions that were stronger than that obtained with rat IgG used as a negative control.

Thus, in order to analyze in detail mTARM-expressing cells, antibody #6 was fluorescently labeled with Alexa 647 monoclonal antibody labeling kit (Molecular Probe). Subsequently, immature dendritic cells or mature dendritic cells obtained by LPS stimulus were suspended in a FACS buffer (PBS/1% FBS/1 mM EDTA) that contained 5% mouse serum and 5% rat serum. FcR blocking solution (BD Pharmingen) was added to the suspension and incubated on ice for 10 minutes. Thereafter, the reaction mixture was reacted on ice for 30 minutes with a fluorescently-labeled anti-mTARM antibody, an anti-CD11c dendritic cell marker antibody, and an anti-CD40 activation marker antibody, and the expression level of the mTARM protein was then measured using FACSCalibur.

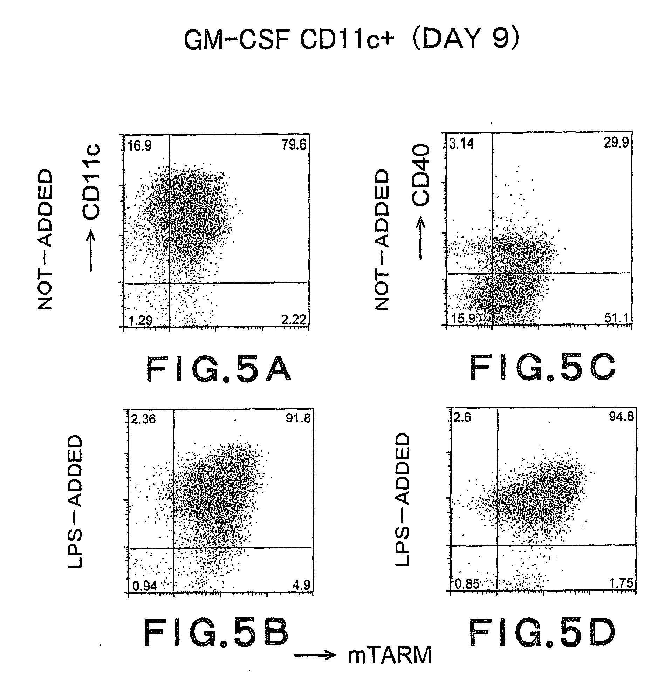

As a result, it was revealed that the mTARM protein was expressed in both immature and mature dendritic cells, that expression of the mTARM protein was enhanced in mature bone marrow dendritic cells rather than in immature dendritic cells, and that the mTARM protein was strongly expressed on cells in which the activation marker CD40 was expressed (Figure 5).

### (5) Expression of TARM protein in normal mouse

Since expression of mTARM was confirmed in bone marrow-derived dendritic cells, expression of an mTARM protein in the immune tissues of normal mice was then analyzed.

Cells were prepared from the bone marrow, peripheral blood, spleen, mesenteric lymph node and Peyer's patch of C57BL/6 male mice. The cells thus prepared were suspended in a FACS buffer (PBS/1% FBS/1 mM EDTA) that contained 5% mouse serum and 5% rat serum. FcR blocking solution was added to the suspension and incubated on ice for 10 minutes. Thereafter, the reaction mixture was reacted on ice for 30 minutes with a fluorescently-labeled anti-mTARM antibody and various types of fluorescently-labeled cell lineage marker antibodies, and the expression level of the mTARM protein was then measured using FACSCalibur.

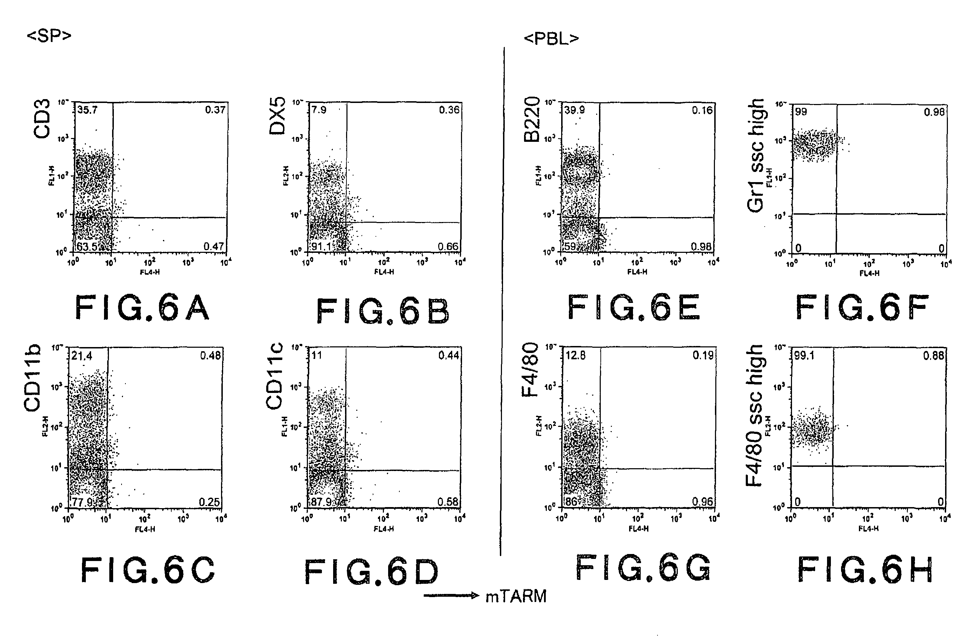

As a result, in the immune tissues of normal mice, expression of the mTARM protein was not observed in all of CD3+ T cells, DX5+ NK cells, CD11b+ myeloid cells and CD11c+ dendritic cells in spleen (SP), and B220+ B cells, F4/80+ monocytes/macrophages, SSC high/Gr1+ neutrophils and SSC high/F4/80+ eosinophils in peripheral blood (PBL) (Figure 6).

Thus, expression of the mTARM protein in normal mouse peripheral tissues was analyzed using cells prepared from the peritoneal cavity thereof.

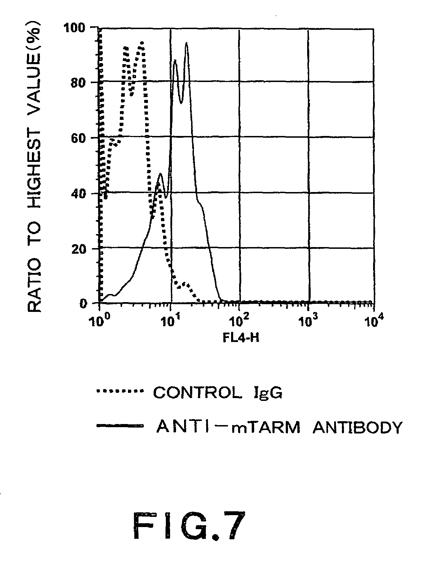

As a result, expression of the mTARM protein was not observed in CD3+ T cells, DX5+ NK cells, CD11c+ dendritic cells, B220+ B cells, F4/80+ monocytes/macrophages, and SSC high/Gr1+ neutrophils from the peritoneal cavity. However, such expression was observed in a part of the CD11b+ myeloid cells. Thus, further analysis was carried out. As a result, it was revealed that the mTARM protein was expressed on c-kit+ mast cells (Figure 7).

### (6) Induction of expression of TARM protein on dendritic cells by LPS inflammatory stimulus

The mTARM protein was expressed on the cultured dendritic cells, but such expression of the mTARM protein was not observed on the dendritic cells prepared from mouse lymphoid and peripheral tissues. Thus, it was considered that expression of the mTARM protein was induced by inflammatory stimulus *in vivo.* Hence, 100 µg of LPS was intraperitoneally administered to C57BL/6 male mice. 14 to 18 hours later, cells were recovered from mesenteric lymph node, and expression of the mTARM protein was then analyzed.

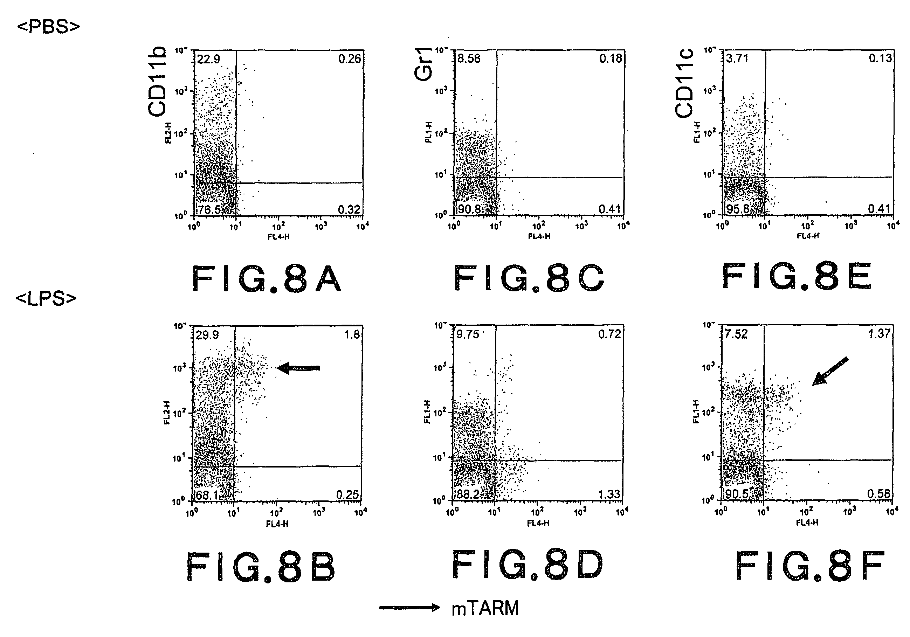

As a result, expression of the mTARM protein was observed on CD11c+/CD11b+ myeloid dendritic cells that had moved to the mesenteric lymph node by the LPS inflammatory stimulus (Figure 8). Accordingly, it was revealed that the mTARM protein was not expressed on the cell surface of dendritic cells *in vivo* at normal condition, but that it was selectively expressed on dendritic cells, and in particular, on myeloid dendritic cells during inflammation. Thus, it was suggested that the mTARM protein functioned on dendritic cells during inflammation.

### [Example 3] Activation of bone marrow-derived cultured dendritic cells by anti-TARM antibody

The mTARM was selectively expressed on dendritic cells. Thus, activation of such dendritic cells by the cross-link stimulus of the mTARM was analyzed.

Bone marrow-derived immature dendritic cells or mature dendritic cells were suspended at a concentration of 1 x 10⁸ cells in 350 µl of a MACS buffer (PBS/1% FBS/2 mM EDTA). 50 µl of FcR blocking solution (Miltenyi) was added to the suspension and incubated at 4°C for 10 minutes. Thereafter, 100 µl of CD11c microbeads (Miltenyi) were added to the reaction mixture and incubated at 4°C for 30 minutes. Thereafter, CD11c+ cells were separated and purified using Auto MACS (Miltenyi). An F(ab)'2 anti-rat IgG antibody (10 µg/ml) (Jackson) was immobilized on a 96-well plate at 37°C for 2 hours, and the plate was then washed with PBS. Thereafter, anti-mTARM antibody #6, #21, or rat IgG (10 µg/ml) used as a negative control was immobilized on the plate at 37°C for 1 hour. The plate was then washed with PBS, and the purified CD11c+ dendritic cells were cultured in a medium, or in a medium that contained an agonistic anti-CD40 antibody (BD Pharmingen) at a final concentration of 2 µg/ml. 24 or 48 hours later, the culture supernatant was recovered, and cytokines were then detected using DuoSet ELISA Development kit (R & D).

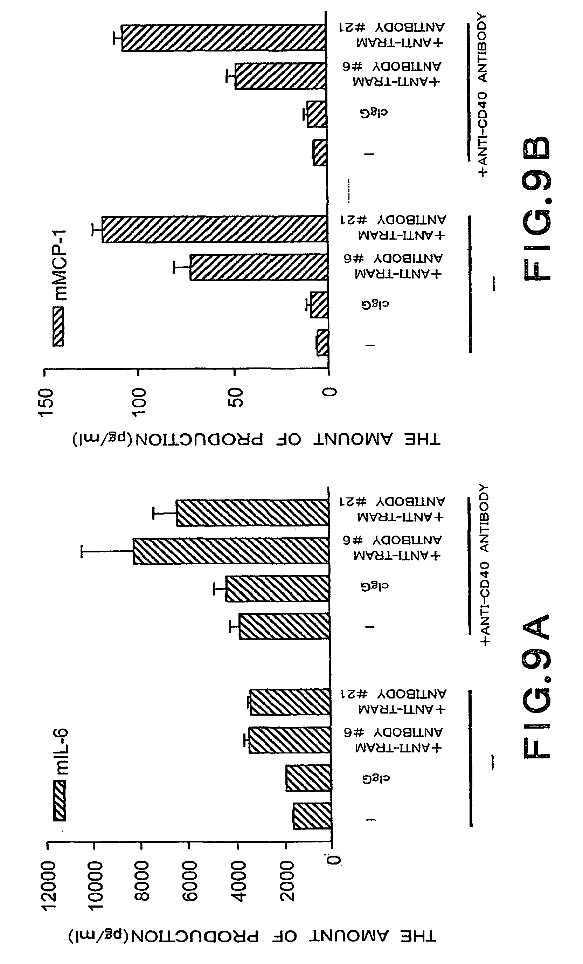

As a result, it was revealed that the anti-mTARM antibody induced production of IL-6 from bone marrow-derived mature dendritic cells at the similar level with that of the anti-CD40 antibody, and had an addictive effect with the anti-CD40 antibody (Figure 9A). Similarly, the aforementioned antibody tended to induce production of IL-6 also from immature dendritic cells. Moreover, the anti-mTARM antibody induced production of MCP-1 from immature bone marrow dendritic cells, but the anti-CD40 antibody did not induce such production of MCP-1 (Figure 9B). Induction of production of MCP-1 by the anti-mTARM antibody was not observed in mature bone marrow dendritic cells. Furthermore, induction of production of IL-12, TNFα, IL-1β, IL-10, and KC was also analyzed, but the anti-mTARM antibody had no significant effects on both immature dendritic cells and mature dendritic cells.

From the above results, it was confirmed that mTARM on dendritic cells is associated with production of specific cytokines and chemokines (e.g. IL-6 and MCP-1).

### [Example 4] Complex formation between TARM and FcRγ chain

It was revealed that mTARM functioned as a dendritic cell -activating receptor. Next, a signal-transducing molecule was examined. The mTARM has a transmembrane region similar to that of Oscar that is involved in osteoclast differentiation. It has been known that Oscar forms a complex with the FcRγ chain, a signal-transducing molecule well known as a component of the IgE receptor. It has been considered that the interaction between a basic amino acid in the cell transmembrane region of Oscar and an acidic amino acid of the FcRγ chain is involved in the aforementioned association. The mTARM also has a basic amino acid in its transmembrane region. On the other hand, DAP10 and DAP12 are known as activation signal-transducing molecules having an acidic amino acid in its transmembrane region, as with the FcRγ chain. Thus, the possibility of a complex formation between the FcRγ chain, DAP10 or DAP12, and the mTARM was analyzed.

Each of the expression vectors of the FcRγ chain, DAP10 and DAP12 was produced by inserting a cDNA fragment amplified using the following primers designed based on the nucleotide sequences as shown in NM_010185, AF072846 and NM_011662 of GenBank^{™} into an expression vector pMXII IRES-Puro designed such that an Flag tag sequence attached to the N-terminal can be expressed on a cell surface.
FcRγ F: cgcctcgagCTGGGAGAGCCGCAGCTCTGCTAT (SEQ ID NO.: 39)
FcRγ R: gcgggcggccgcCTACTGGGGTGGTTTCTCATGCTT (SEQ ID NO.: 40)
DAP10 F: cgcgtcgacCAGACATCGGCAGGTTCCTGCTCC (SEQ ID NO.: 41)
DAP10 R: gcgggcggccgcTCAGCCTCTGCCAGGCATGTTGAT (SEQ ID NO.: 42)
DAP12 F: cgcgtcgacTTAAGTCCCGTACAGGCCCAGAGT (SEQ ID NO.: 43)
DAP12 R: gcgggcggccgcTCATCTGTAATATTGCCTCTGTGT (SEQ ID NO.: 44)
Thereafter, recombinant retrovirus was prepared by the similar method as that applied to prepare mTARM-expressing cells, and 8300.19 cells expressing mTARM were then infected with the recombinant retrovirus. Thereafter, the cells were cultured in the presence of puromycin, and the infected cells were then selected. The obtained B300.19 transfectants were suspended in an FACS buffer (PBS/1% FBS/1 mM EDTA) and reacted with antibody #6 and a mouse anti-Flag antibody (Sigma) at a final concentration of 10 µg/ml. Thereafter, it was reacted with a PE-labeled donkey anti-rat IgG secondary antibody (Jackson) and a Cy5-labeled donkey anti-mouse IgG secondary antibody (Jackson), followed by the measurement of the expression level using FACSCalibur (Becton Dickinson).

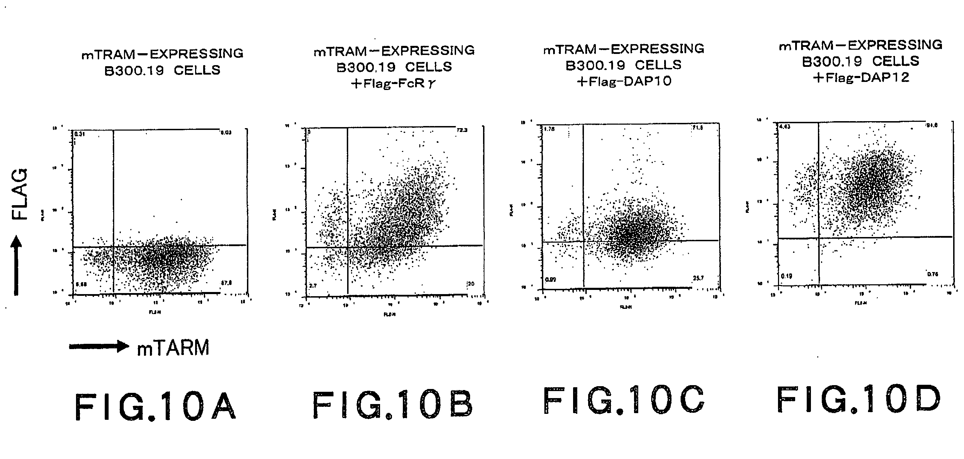

As a result, it was revealed that when the mTARM and the FcRγ chain were simultaneously expressed, the expression level of the FcRγ chain on the cell surface was increased, as the expression level of the mTARM on the cell surface was increased. The expression levels of DAP10 and DAP12 on the cell surface did not depend on the expression level of the mTARM. Accordingly, it was suggested that the expression level of the FcRγ chain on the cell surface was increased by its formation of a complex with the mTARM (Figure 10).

Subsequently, mTARM-binding proteins were biochemically analyzed.

B300.19 transfectants were solubilized in a cell lysis buffer containing 1% digitonin (1% digtonin/50 mM Tris-HCl (pH 7.5)/150 mM NaCl/5 mM NaF/1 mM orthovanadate/Complete^{™} (Roche)). Immunoprecipitation was carried out using an anti-Flag antibody, and immunoblot analysis was then carried out using anti-mTARM antibody #6.

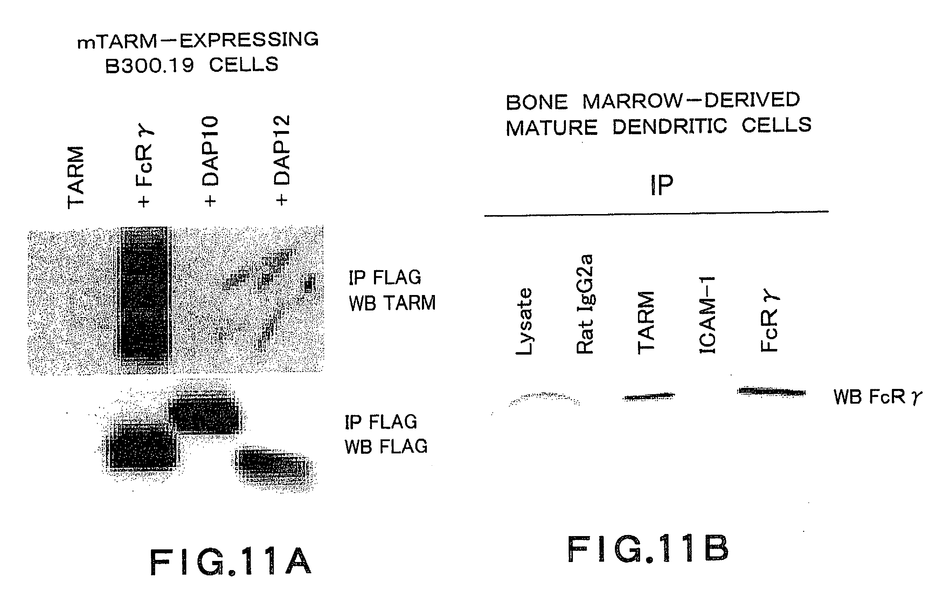

As a result, it was found that the mTARM was immunoprecipitated together with the FcRγ chain, but either DAP10 or DAP12 were not immunoprecipitated together with the mTARM (Figure 11A). Immunoprecipitation of FcRγ chain, DAP10 and DAP12 by the anti-Flag antibody was confirmed by immunoblot analysis with the anti-Flag antibody. In addition, the expression of mTARM, a Flag tagged FcRγ chain, DAP10 and DAP12 in B300.19 transfectants was confirmed by immunoblot analysis of cell lysates using anti-mTARM antibody #6 or the anti-Flag antibody. Accordingly, it was revealed that the FcRγ chain formed a complex with the mTARM in the B300.19 transfectants.

Moreover, bone marrow-derived mature dendritic cells were solubilized in a cell lysis buffer containing 1% digitonin. Immunoprecipitation was carried out using rat IgG as a negative control, anti-mTARM antibody #21, an anti-CD54 antibody and an anti-FcRγ chain antibody, and immunoblot analysis was then carried out using an anti-FcRγ chain antibody.

As a result, it was found that the FcRγ chain was immunoprecipitated together with the mTARM, and that another cell membrane protein CD54 and the FcRγ chain were not co-immunoprecipitated (Figure 11B). Accordingly, it was revealed that the FcRγ chain and the mTARM formed a complex in mature dendritic cells.

From these results, it was suggested that activation of dendritic cells by the cross-link stimulus of mTARM is mediated via signal transduction from the FcRγ chain.

### [Example 5] Adhesion of activated Lymphocytes to immobilized TARM

### (1) Expression of mTARM-binding molecules on activated T cell

It has been known that dendritic cells act as antigen-presenting cells and interact with T cells. Thus, it is tempting to speculate that molecules that bind to mTARM are expressed on T cells and that activation of dendritic cells is regulated via such molecules. Accordingly, the presence or absence of the mTARM-binding molecules on the T cells was analyzed.

4 x 10⁷ C57BL/6 male mouse splenic cells were suspended in 70 µl of a MACS buffer (PBS/1% FBS/2 mM EDTA), and 10 µl of FcR blocking solution (Miltenyi) was added to the suspension. The mixture was then incubated at 4°C for 10 minutes. 100 µlI of CD4 microbeads (Miltenyi) were further added to the reaction mixture and incubated at 4°C for 15 minutes. Thereafter, Auto MACS was used to obtain resting CD4+ T cells. The CD4+ cells, which had been purified with MACS, were suspended in a medium (RPMI1640/10% FBS/1 mM sodium pyruvate/55 µM 2-mercaptoethanol) at a concentration of 1 x 10⁶ cells/ml. Thereafter, the suspension was added to a plate, on which an anti-CD3 antibody (eBioscience) had been immobilized with a 1 µg/ml solution at 37°C for 2 hours, and stimulated in the presence of a 2 µg/ml anti-CD28 antibody (Pharmingen). When CD4+ T cells were differentiated into Th1, such anti-CD3 antibody stimulus was carried out in the presence of mouse IL-12 (10 ng/ml) (Peprotech) and an anti-mouse IL-4 antibody (10 µg/ml) (MP4-25D2; Pharmingen). When CD4+ T cells were differentiated into Th2, such anti-CD3 antibody stimulus was carried out in the presence of mouse IL-4 (15 ng/ml) (Genzyme) and an anti-mouse IL-12 antibody (15 µg/ml) (24910.1; Pharmingen). Two days after the stimulus, in the case of Th1, mouse IL-2 (20 ng/ml) (Genzyme) and mouse IL-12 (10 ng/ml) were added, followed by culture. In the case of Th2, mouse IL-2 (20 ng/ml) and mouse IL-4 (15 ng/ml) were added, followed by culture. Differentiation into Th1 and Th2 was confirmed by production of IFN-γ and IL-4, respectively.

Resting CD4+ T cells immediately after purification with MACS and activated CD4+ T cells 2 and 8 days after CD3 stimulus were used to analyze the binding activity of an mTARM-AP chimeric protein onto a cell surface. These cells were suspended in an FACS buffer (PBS/1% FBS) and reacted on ice for 30 minutes with mTARM-AP chimeric protein or AP protein used as a negative control at a final concentration of 30 µg/ml. Thereafter, a rabbit anti-PLAP antibody (6000 fold dilution) (COSMO BIO Co., Ltd.) was added, and then reacted therewith on ice for 30 minutes. Further, a PE-labeled donkey anti-rabbit IgG (H + L) antibody (50 fold dilution) (Jackson) was added, and then reacted therewith on ice for 30 minutes. The binding activity of the mTARM-AP chimeric protein was measured using FACSCalibur.

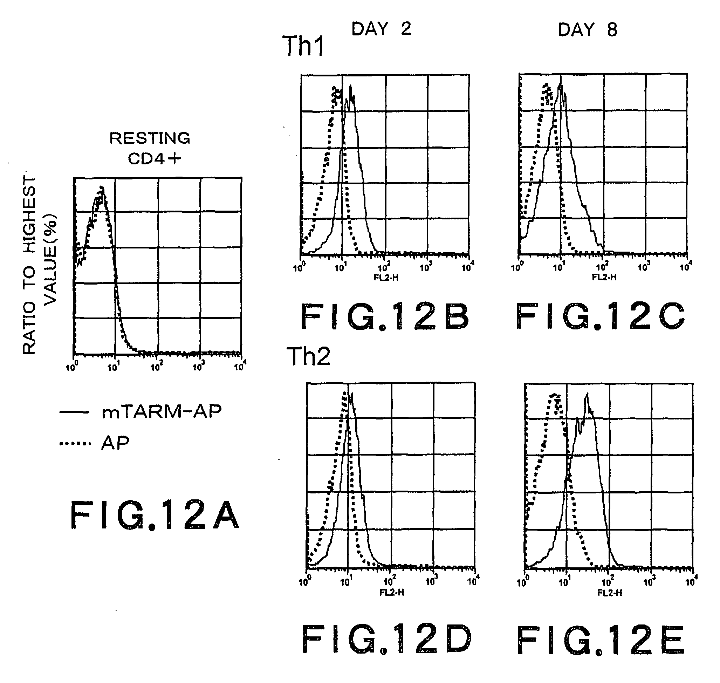

As a result, it was revealed that mTARM-binding molecules were not expressed on a resting CD4+ T cell, but that such expression was induced on activated CD4+ T cells (Figure 12). The mTARM-binding molecules had already been expressed 2 days after the stimulus and the expression thereof was also maintained 8 days after the stimulus. Such mTARM-binding molecules were expressed under both Th1 and Th2 differentiation conditions. 8 days later, expression of the mTARM-binding molecule in Th2 cells tended to be stronger than that in Th1 cells (Figure 12).

### (2) Adhesion of activated T cells to mTARM recombinant protein

Next, the possibility of the function of the mTARM as an adhesion molecule to activated T cells was analyzed.

First, 50 µl of anti-alkaline phosphatase antibody (10 µg/ml) (Seradyn) was added to each well of a 96-well ELISA plate (Nunc) and incubated at 37°C for 30 minutes for immobilization. After washing with PBS, a non-specific binding site was blocked with Block Ace (Dainippon Pharma Co., Ltd.). An AP chimeric protein (10 nM) was added to each well and incubated at room temperature for 30 minutes for immobilization. 6 to 9 days after the stimulus, activated T cells were suspended in a cell adhesion buffer (RPMI1640/0.5% BSA/20 mM HEPES (pH 7.4)), followed by fluorescent labeling with Calcein-AM (Dojindo Laboratories). Thereafter, the 1 x 10⁵ cells were added to each well and incubated at 37°C for 1 hour. Non-adhered cells were eliminated by washing, and a cell lysis buffer (10 mM Tris-HCl (pH 8.0)/1% TritonX-100) was then added thereto. Thereafter, measurement was carried out at an excitation wavelength of 485 nm and a detection wavelength of 535 nm using Wallac ARVO SX 1420 MULTILABEL COUNTER (Perkin Elmer), and the adhered cells were then quantified. With regard to the level of cell adhesion, the ratio of the adhered cells to the added cells was expressed as a percentage.

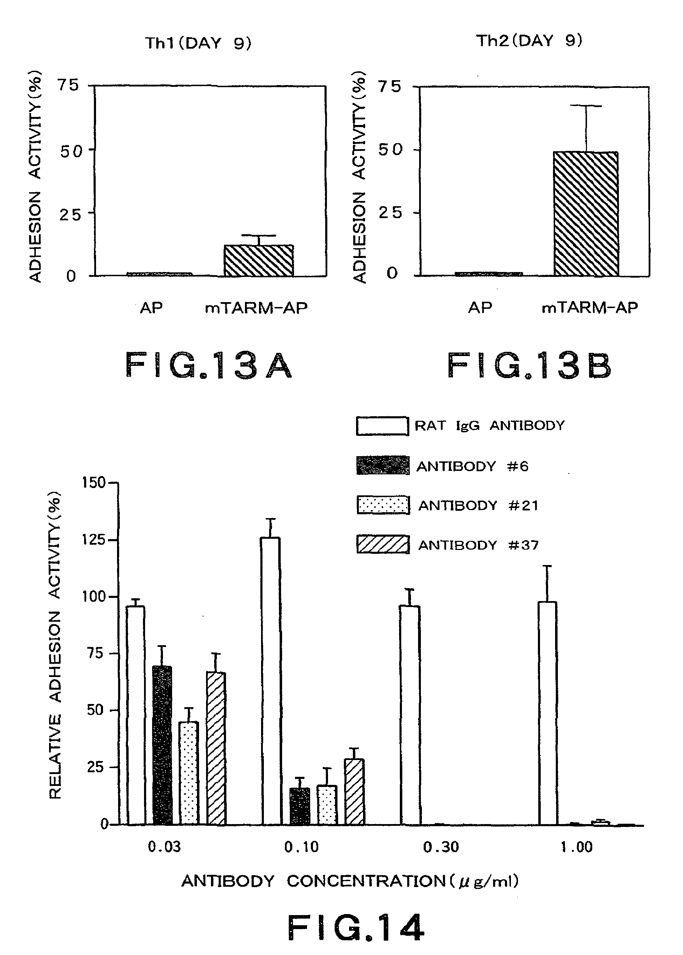

As a result, it was revealed that the mTARM functioned as an adhesion molecule to activated T cells (Figure 13). Both the Th1 and Th2 cells exhibited adhesion activity to the mTARM. The adhesion activity of the Th2 cells to the mTARM tended to be higher than that of the Th1 cells to the mTARM.

### [Example 6] Cell adhesion-inhibiting activity of anti-TARM antibody

The effect of anti-TARM antibodies on cell adhesion of Th2 cells to the mTARM was analyzed.

A 10 µg/ml anti-mTARM antibody was added to Th2 cells and pre-treated at room temperature for 10 minutes. Thereafter, the resultant cells were added to a plate on which an mTARM-AP chimeric protein had been immobilized, and the cell adhesion activity in the presence of the antibody was analyzed.

As a result, adhesion of the Th2 cells to the mTARM-AP chimeric protein was significantly suppressed in all of anti-mTARM antibodies #6, #21 and #37 (Figure 14).

### [Example 7] Therapeutic effect of anti-TARM antibody in collagen-induced arthritis model

A Collagen-Induced Arthritis (CIA) model is a disease model of autoimmune rheumatoid arthritis. Since CD4+ T cell and antibodies that react with type II collagen are detected, it is considered that both cooperate to provoke arthritis. In addition, it is reported that susceptibility to CIA model is linked to MHC class II molecules. The TARM is an activating molecule that is expressed on dendritic cells, and it induces cell adhesion of activated T cells via TARM-binding molecules. Accordingly, it has been considered that the interaction between such dendritic cells and activated T cells is inhibited by an anti-TARM antibody, so as to possibly suppress an immune response associated with dendritic cells or the activated T cells. Thus, the therapeutic effect of the anti-TARM antibody in the CIA model was analyzed.

A 3% bovine joint-derived type II collagen solution (Collagen Gijutsu Kenshukai) and Freund's complete adjuvant (Difco) were mixed in equal amounts to produce an emulsion. Thereafter, 100 µl of the emulsion (150 µg/mouse) was intracutaneously administered at the base of the tail of a 5-week-old DBA/1J mouse (Charles River Laboratories Japan, Inc.), so that the mouse was immunized on day -21 (initial immunization) and on day 0 (booster). From the booster, the anti-mTARM antibody #6 was administered intravenously at a dose of 500 µg twice a week. From 3 days after the booster, the measurement of the body weight and the external evaluation were carried out. On day 13, the mouse was sacrificed. External findings were evaluated using the following score. That is, 0: normal; 1: erythema and mild swelling confined to the mid-foot (tarsus) or ankle joint; 3: erythema and moderate swelling extending from the ankle to the metatarsal joint; 4: erythema and severe swelling at the entire portion ranging from the ankle, foot, and digits.

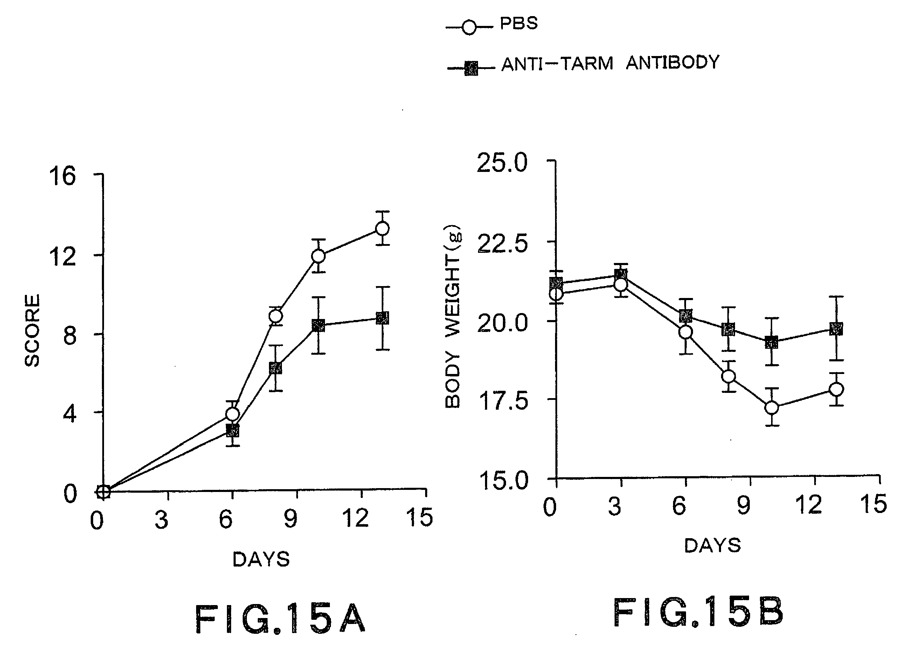

Consequently, as a result of the administration of the anti-mTARM antibody, clear alleviation in the symptoms and suppression of body weight reduction were observed (Figure 15).

Moreover, heparinized blood was collected from the inferior vena cava of the sacrificed mice, and serum amyloid A (SAA) concentration in plasma and antibody titer to collagen were measured. SAA is a plasma protein produced in liver cells by the action of cytokines produced by inflammatory stimulus. The SAA concentration in plasma is used as an index of inflammation. The antibody titer to collagen is used as an index of an antigen-specific immune response.

The SAA concentration in plasma was measured by an ELISA kit (BioSource) using the plasma diluted 8,000-fold.

Moreover, the antibody titer to collagen in plasma was measured as follows.

First, 50 µl of a 5 µg/m) bovine joint-derived type II collagen solution was added to each well of a 96-well ELISA plate (Nunc) and incubated at 4°C overnight for immobilization. Thereafter, the well was washed with T-PBS (0.02% Tween20/PBS), and a non-specifically binding site was then blocked with 1% BSA/PBS. The well was washed with T-PBS 3 times, and 50 µl of plasma that had been diluted 100,000-fold with T-PBS was then added to each well, followed by incubating at room temperature for 2 hours. Thereafter, the well was washed with T-PBS 3 times, and 50 µl each of biotinylated anti-mouse IgG1 (BD) and biotinylated anti-mouse IgG2a (BD) that had been diluted 1,000-fold with T-PBS were then added to each well, followed by incubated at room temperature for 2 hours. The well was washed with T-PBS 3 times, and 50 µl each of HRP-labeled streptavidin (Pierce) that had been diluted 5,000-fold with T-PBS was then added to each well, followed by at room temperature for 30 minutes. Thereafter, chromogenic substrate was added and developed.

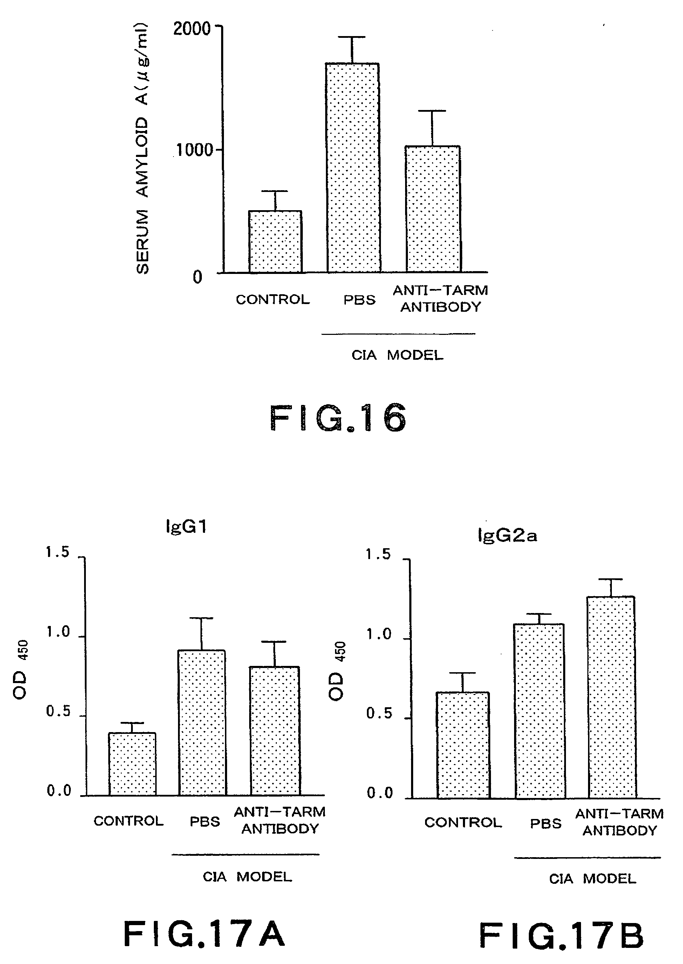

As a result, the SAA concentration in plasma was decreased by administration of the anti-mTARM antibody (Figure 16). However, the anti-collagen antibody titer in plasma was not changed by administration of the anti-mTARM antibody (Figure 17).

### [Example 8] Determination of human TARM full-length gene sequence

In order to identify a human TARM gene, BLAST search was carried out using the mouse TARM gene sequence. As a result, a sequence showing high homology with the mouse TARM cDNA sequence (GenBank Accession No. XM_497642) was discovered. However, no signal sequences existed in an amino acid sequence (LOC441864) encoded by such XM_497642, and thus it was not considered that the aforementioned sequence functions as a cell membrane protein. It was considered that XM_497642 is a putative sequence and that estimation of a cDNA sequence from the genome sequence is incorrect. Hence, it was attempted to determine the full-length gene sequence of human TARM. TARM expression tissues were identified, and the full-length sequence of TARM cDNA was estimated by conducting 5'- and 3'-RACE. Thereafter, based on the sequence of a region encoding the TARM protein, primers were designed, and full-length cDNA was then isolated.

### (1) Expression analysis of human TARM gene

First, expression of hTARM in human tissues was analyzed. Based on GenBank Accession: XM_497642, primers were designed.
hTARM F1: TGTGAATACTACAGAAAAGCATCC (SEQ ID NO.: 45)
hTARM R1: TCCACCTGCGGTCACTGTACCCCT (SEQ ID NO.: 46)
Single-stranded cDNA was synthesized from total RNA of each human organ (Clontech) using RNA PCR kit (TAKARA). Using such single-stranded cDNA as a template, real-time PCR was carried out using ABI7700. The PCR was carried out using a reaction solution with the following composition (12.5 µl of QuantiTect SYBR Green PCR Master Mix (QIAGEN), 0.25 µl of Uracil DNA Glycosylase (Invitrogen), 0.125 µl of 100 µM F primer, 0.125 µl of 100 µM R primer, 2.5 µl of template cDNA (10 fold diluted), and 7.25 µl of distilled water). For such PCR, after the treatment at 94°C for 10 minutes, a reaction cycle consisting of 94°C-30 seconds and 60°C-1 minute was repeated 35 times.

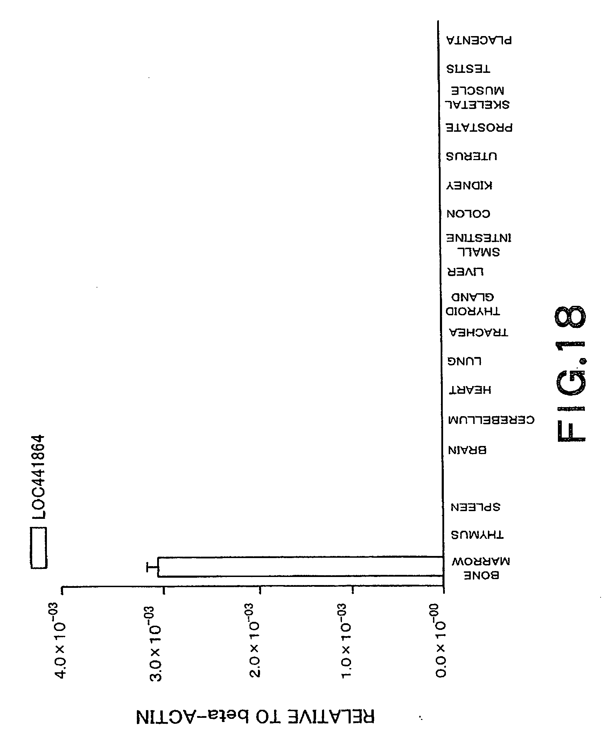

As a result, it was found that the hTARM gene, as with the mTARM gene, was strongly expressed in bone marrow (Figure 18).

### (2) Isolation of human TARM gene

Since the hTARM gene was expressed in bone marrow, the total RNA of the bone marrow was used to carry out 5'-RACE and 3'-RACE, so as to attempt to determine the sequence of a full-length gene of the hTARM.

First, double-stranded cDNA was synthesized from the total RNA of the bone marrow using cDNA synthesis kit (TAKARA), and cDNA was then purified using Qiaquick PCR purification kit (Qiagen). Subsequently, an ad29 adapter was added thereto, and the thus obtained product was used as a template in RACE. 1^{st} PCR was carried out using a reaction solution with the following composition (5 µl of 10 x ExTaq buffer, 4 µl of 2.5 mM dNTP, 0.25 µl of ExTaq, 0.5 µl of 100 µM primer (5'PCR4), 0.5 µl of 100 µM Gene specific primer, 1 µl of ad29 adapter-added cDNA (25 fold diluted), and 38.75 µl of distilled water).

The following sequences were used as primers.
5'PCR4: AGCTACGCTGAAGTATCAACGCAGAG (SEQ ID NO.: 21)
hTARM_RACE_5'_4: CTTCTGGCACTGCAGAGTCACCCT (SEQ ID NO.: 47), or
hTARM_RACE_3'_4: GGAGAGTACACCTGTGAATACTAC (SEQ ID NO.: 48)
   For such PCR, after the treatment at 94°C for 5 minutes, a reaction cycle consisting of 94°C-30 seconds, 65°C-1 minute, and 72°C-5 minutes was repeated 30 times. Finally, a reaction was carried out at 72°C for 5 minutes.

2^{nd} PCR was carried out using a reaction solution with the following composition (5 µl of 10 x ExTaq buffer, 4 µl of 2.5 mM dNTP, 0.25 µl of ExTaq, 0.5 µl of 100 µM primer (5'PCR1), 0.5 µl of 100 µM Gene specific primer, 1 µl of the 1^{st} PCR product (100 fold diluted), and 38.75 µl of distilled water).

The following sequences were used as primers.
5'PCR1: (SEQ ID NO.: 24),
h29B140 F1: TGTGAATACTACAGAAAAGCATCC (SEQ ID NO.: 49), or
h29B140 R1: TCCACCTGCGGTCACTGTACCCCT (SEQ ID NO.: 50)
For such PCR, after the treatment at 94°C for 5 minutes, a reaction cycle consisting of 94°C-30 seconds, 65°C-30 seconds, and 72°C-5 minutes was repeated 25 times. Finally, a reaction was carried out at 72°C for 5 minutes. The amplified cDNA fragment was cloned into pCR2.1 (Invitrogen), and the nucleotide sequence thereof was determined using ABI3100 Sequence Analyzer.

As a result, 5' and 3' nucleotide sequences, which were completely different from the sequence as shown in XM_497642, were obtained (SEQ ID NO: 9).

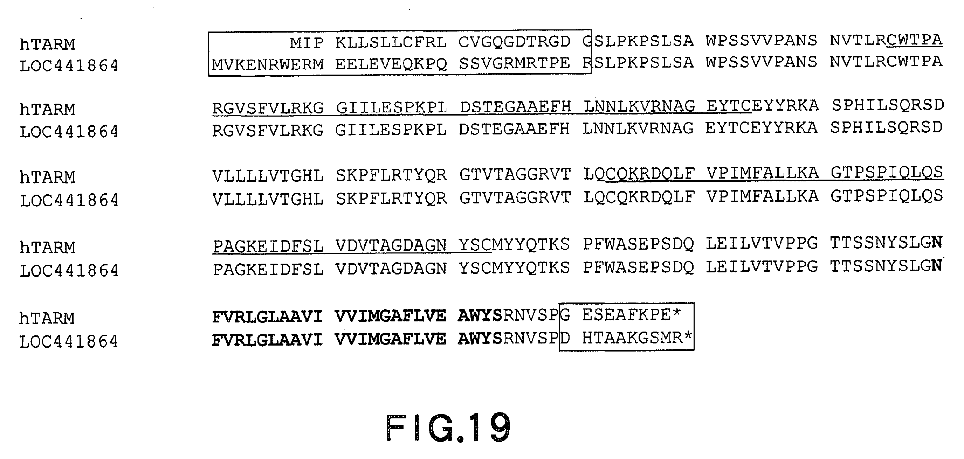

The following primers were designed using the nucleotide sequence information obtained by RACE:
h298140_Sall-Kozac_F:
   cgcgtcgacGCCACCATGATCCCTAAGCTGCTTTCCCTC (SEQ ID NO.: 51)
   h29B140_NotI-R: cgcgcggccgcCTAGCGCATGCTACCCTTGGCAGC (SEQ ID NO.: 52)
With these primers, PCR was carried out using single-stranded cDNA synthesized from the total RNA of bone marrow using RNA PCR kit (TAKARA) as a template. The PCR was carried out in a reaction solution with the following composition (5 µl of 10 x buffer, 4 µl of 2.5 mM dNTP, 0.5 µl of Pyrobest polymerase (TAKARA), 0.5 µl each of 100 µM primers, 1 µl of cDNA, 2.5 µl of DMSO, and 36 µl of distilled water). For such PCR, after the treatment at 94°C for 5 minutes, a reaction cycle consisting of 94°C-30 seconds, 65°C-30 seconds, and 72°C-5 minutes was repeated 35 times. Finally, a reaction was carried out at 72°C for 2 minutes. The amplified cDNA was cloned into pBlueScriptII SK(+) (Stratagene), and the nucleotide sequence thereof was then determined using ABI3100 Sequence Analyzer. The obtained nucleotide sequence of the hTARM cDNA was identical to the sequence determined by RACE (SEQ ID NO: 9). The amino acid sequence (SEQ ID NO: 10) encoded by the hTARM cDNA had a signal sequence necessary for functioning as a cell membrane protein at the N-terminal thereof. The hTARC and the amino acid sequence encoded by XM_497642 (LOC441864) had different N-terminal and C-terminal. Accordingly, it was revealed that such a putative cDNA sequence, XM_497642, estimated from the genome sequence did not actually exist, and that the hTARM cDNA was a real gene encoding the cell membrane protein hTARM (Figure 19).

### [Example 9] Preparation of antibody against human TARM (1) Preparation of human TARM-expressing cells

A human TARM gene expression vector was prepared by inserting the hTARM cDNA obtained in Example 8 into an expression vector pMXII IRES EGFP (Oncogene (2000) 19(27): 3050-3058).

### Recombinant retrovirus was prepared as follows.

293/EBNA-1 cells (Invitrogen) of 3 x 10⁶ cells were suspended in a medium (D-MEM/10% FBS), and put in a 10-cm culture dishes, and cultured in a CO₂ incubator for 24 hours. On the following day, the medium was changed with a fresh one, and a transfection solution prepared as described below was then added thereto, so as to carry out transfection. The transfection solution was prepared by adding 600 µl of OPTI-MEM (GIBCO BRL) and 24 µl of TransIT LT1 (TaKaRa) into a 5ml tube to mix them, then incubating the mixture at room temperature for 5 minutes, and then adding 9 ug of an expression vector and 9 ug of pCL-Eco (Imgenex) used as a packaging vector to the reaction mixture, followed by incubating the mixture at room temperature for 5 minutes. 48 hours later, the culture supernatant was recovered, and filtration was then carried out with a 0.45µm filter, so as to obtain a recombinant virus solution.

B300.19 cells were infected with this recombinant virus as described below, so as to prepare hTARM-expressing cells. The 1 x 10⁶ B300.19 cells were added to a 15ml tube, and they were then centrifuged at 1200 rpm at 25°C for 5 minutes. Thereafter, the culture supernatant was discarded by aspiration. A solution obtained by adding 2 µl of polybrene (10 mg/ml) and 2 µl of 55 µM 2-mercaptoethanol to 2 ml of the recombinant virus solution was added to the cells. The obtained mixture was then centrifuged at 2500 rpm at 30°C for 2 hours, so that the cells were infected with the recombinant virus. After completion of the infection, the recombinant virus solution was discarded, and a medium (RPMI-1640/10% FBS/55 µM 2-mercaptoethanol) was added thereto, followed by culture. EGFP-positive cells were separated by cell sorting, so as to obtain hTARM-expressing cells.

### (2) Preparation of chimeric protein fused hTARM extracellular region to SEAP or Fc

The extracellular region of hTARM was amplified by PCR using hTARM full-length cDNA as a template and also using a SalI-added 5' primer (h29B140_SalI-Kozac_F: cgcgtcgacGCCACCATGATCCCTAAGCTGC TTTCCCTC (SEQ ID NO: 51)) and a NotI-added 3' primer (h29B140_NotI_SEAP_R: gcgggcggccgcACCCAGGGAGTAGTTGCTCGATGT (SEQ ID NO: 53)). The PCR was carried out in a reaction solution with the following composition (5 µl of 10 x buffer, 4 µl of 2.5 mM dNTP, 0.5 µl of Pyrobest polymerase (TAKARA), 0.5 µl each of 100 µM primers, 1 µl of cDNA, 2.5 µl of DMSO, and 36 µl of distilled water). For such PCR, after the treatment at 94°C for 5 minutes, a reaction cycle consisting of 94°C-30 seconds, 65°C-30 seconds, and 72°C-5 minutes was repeated 35 times. Finally, a reaction was carried out at 72°C for 2 minutes. The amplified cDNA was cloned into pBlueScriptII SK(+) (Stratagene), and the nucleotide sequence thereof was then confirmed using ABI3100 Sequence Analyzer. The obtained cDNA fragment was digested with SalI and NotI, and then inserted into pcDNA3.1(+)-SEAP(His)₁₀-Neo vector described in Example 2, so as to prepare an hTARM-AP expression vector.

Thereby, the hTARM extracellular region is fused through three-amino acid linker (Ala-Ala-Ala) to secretory-type human placental alkaline phosphatase having a 10-histidine tag (His)₁₀ at the C-terminal thereof to be expressed as a secretory chimeric protein (hereinafter referred to as AP chimeric protein). The obtained AP chimeric protein expression vector was introduced into 293/EBNA-1 cells using TransIT LT1 (TAKARA) and they were then cultured for 4 or 5 days. Thereafter, the culture supernatant was recovered by centrifugation, and the AP chimeric protein secreted into the supernatant was then filtrated with a 0.22µm filter. Thereafter, Hepes (pH 7.4) and sodium azide were added thereto to final concentrations of 20 mM and 0.02%, respectively, and the obtained product was stored at 4°C. The concentration of the AP chimeric protein was calculated by measuring alkaline phosphatase activity using Aurora AP chemiluminescent reporter gene assay (ICN).

### (3) Preparation of monoclonal antibody against hTARM

For use as an antigen in immunization, first, the hTARM-AP chimeric protein was purified.

Such purification was carried out utilizing the histidine tag existing at the C-terminal of the AP chimeric protein and using His Trap Kit (Amersham Biosciences). A culture supernatant containing the hTARM-AP chimeric protein was added to a 1ml HiTrap chelating HP column (Amersham Biosciences), followed by washing with a 10 mM imidazole solution. Thereafter, the hTARM-AP protein was eluted from the column using a 500 mM imidazole solution. The concentration of the hTARM-AP chimeric protein was calculated by the measurement of enzyme activity using Aurora AP chemiluminescent reporter gene assay (ICN) and by protein quantification using Protein Assay kit II (BIO-RAD).

Subsequently, the obtained hTARM-AP chimeric protein was used as an antigen, and Balb/c mice were immunized with the aforementioned protein by Kohjin Bio Co. Ltd. Lymphocytes were isolated from the immunized mice, and the isolated lymphocytes were then mixed with P3U1 myeloma cells. Thereafter, cell fusion was carried out by a PEG method. Using a culture supernatant of the obtained hybridomas, screening was carried out by ELISA with an hTARM-Fc chimeric protein. Cloning was carried out from positive wells, and 6 types of clones were obtained (#11, #18, #19, #22, #26, and #40). As a result of analyzing specificity by FACS, it was found that all clones reacted only with hTARM-expressing B300.19 cells, and that they did not react with parental B300.19 cells. Hybridomas that produced the anti-hTARM monoclonal antibodies #11, #18, #19, #22, #26 and #40 were inoculated into the peritoneal cavity of a nude mice, and ascites was obtained therefrom. Thereafter, antibodies were purified using a Protein A column.

### [Example 10] Adhesion of activated human T cells to hTARM recombinant protein

Human peripheral blood was collected using heparin, and Ficoll-Paque PLUS (Amersham Biosciences) was added thereto in an equal amount of the human peripheral blood, and mononuclear cells were isolated by density gradient centrifugation at 400 x g for 30 minutes. The isolated cells were suspended in an MACS buffer (PBS/1% FBS/2 mM EDTA), and FcR blocking solution (Miltenyi) was added at 5 µl/1 x 10⁷ cells to the suspension. The obtained mixture was reacted at 4°C for 15 minutes. Human CD25+CD4+ Treg isolation kit (Miltenyi) and Auto MACS were used to obtain resting CD4+ T cells. A PE-labeled mouse anti-human CD25 antibody (Miltenyi) and an FITC-labeled mouse anti-human CD4 antibody (Miltenyi) were added, each in an amount of 1/11 of the solution, to the CD4+ cells that has been purified with MACS. They were then reacted at 4°C for 20 minutes. Thereafter, FACS Aria (BD Biosciences) was used to obtain CD4+ CD25- T cells. The CD4+ CD25- T cells were activated using T cell Activation/Expansion Kit human (Miltenyi). From 3 days after the stimulus, human IL-2 (2 ng/ml) was added to the cells. Using activated CD4 positive T cells obtained on the 6^{th} and 8^{th} day after the stimulus, the possibility of the function of the hTARM-AP chimeric protein as an adhesion molecule to activated T cells was analyzed.

First, 50 µl of anti-alkaline phosphatase antibody (10 µg/ml) (Seradyn) was added to each well of a 96-well ELISA plate (Nunc) and incubated at 37°C for 30 minutes for immobilization. After washing with PBS, a non-specific binding site was blocked with Block Ace (Dainippon Pharma Co., Ltd.). An AP chimeric protein (1 nM) was added to each well and incubated at room temperature for 30 minutes for immobilization. Activated T cells were suspended in a cell adhesion buffer (RPMI1640/0.5% BSA/20 mM HEPES/55 nM 2ME (pH 7.4)), followed by fluorescent labeling with Calcein-AM (Dojindo Laboratories). Thereafter, the 5 x 10⁴ cells were added to each well and then incubated at 37°C for 1 hour. Non-adhered cells were eliminated by washing, and a cell lysis buffer (10 mM Tris-HCl (pH 8.0)/1% TritonX-100) was then added thereto. Thereafter, measurement was carried out at an excitation wavelength of 485 nm and a detection wavelength of 535 nm using Wallac ARVO SX 1420 MULTILABEL COUNTER (Perkin Elmer), and the adhered cells were quantified. With regard to the level of cell adhesion, the ratio of the adhered cells to the added cells was expressed as a percentage.

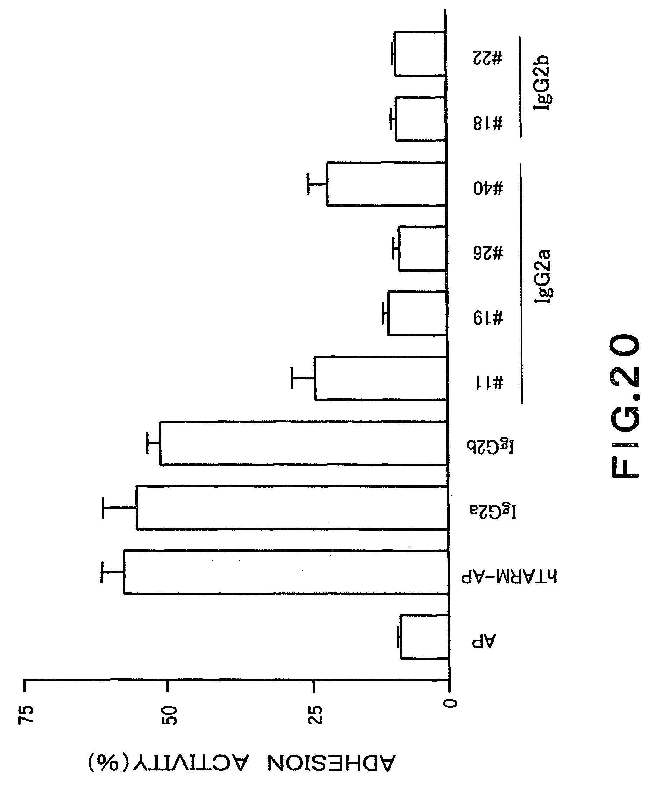

As a result, it was revealed that the hTARM functioned as an adhesion molecule to activated human T cells (Figure 20).

### [Example 11] Cell adhesion-inhibiting activity of anti-TARM antibody

The effect of anti-TARM antibodies on cell adhesion of activated human T cells to the hTARM was analyzed.

An AP chimeric protein was added to each well and then incubated at room temperature for 30 minutes for immobilization. Thereafter, each of 10 µg/ml anti-hTARM antibodies (#11, #18, #19, #22, #26, and #40) was added to each well and pre-treated at room temperature for 30 minutes. Thereafter, fluorescently-labeled activated T cells were added thereto, and cell adhesion activity in the presence of each antibody was analyzed. It is to be noted that an anti-IgG2a antibody was used as a control for #11, #19, #26, and #40, and that an anti-IgG2b antibody was used as a control for #18 and #22.

As a result, it was found that adhesion of the activated T cells to the hTARM-AP chimeric protein was significantly suppressed in the presence of anti-hTARM antibodies #18, #19, #22, and #26, and that partial suppression of such adhesion was observed in the presence of anti-hTARM antibodies #11 and #40 (Figure 20).

### [Example 12] Identification of novel ligand mTARM-L for mTARM

### (1) Selection of mTARM-L (mTARM Ligand) candidate molecules

The mTARM-binding molecules are present on activated T cells (Example 5). Thus, the presence or absence of the mTARM-binding molecules on mouse immune cell-derived cell lines (L5178Y-R, BW5147, and Raw264.7) was analyzed by the method described in Example 5.

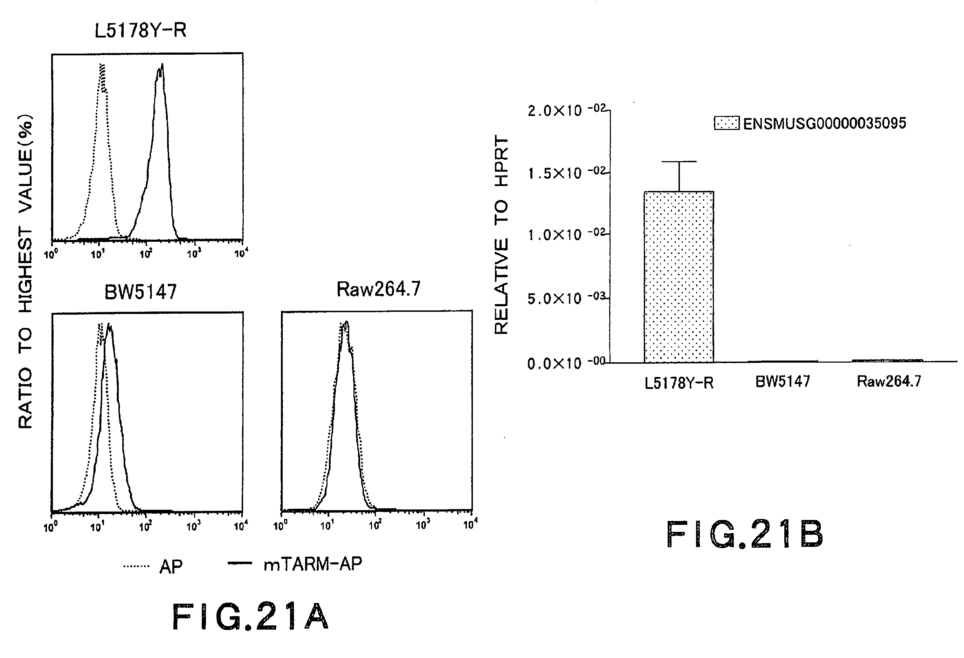

As a result, it was revealed that the mTARM-binding molecules were strongly expressed on the L5178Y-R cells, but that they were hardly expressed on the BW5147 cells and the Raw264.7 cells (Figure 21A).

Subsequently, on the assumption that an unknown ligand for the mTARM belonged to the immunoglobulin superfamily (IgSF), the database of Mouse Ensemble was searched. Focusing attention on 47 IgSF candidates, expression in immune cells was examined by real-time PCR. Total RNA was isolated from the L5178Y-R cells, the BW5147 cells, and Raw264.7 cells using RNeasy mini kit (Qiagen, Hilden, Germany). The real-time PCR was carried out in the presence of SYBR-green using ABI7700 Sequence Detection System (PE Applied-Biosystems).

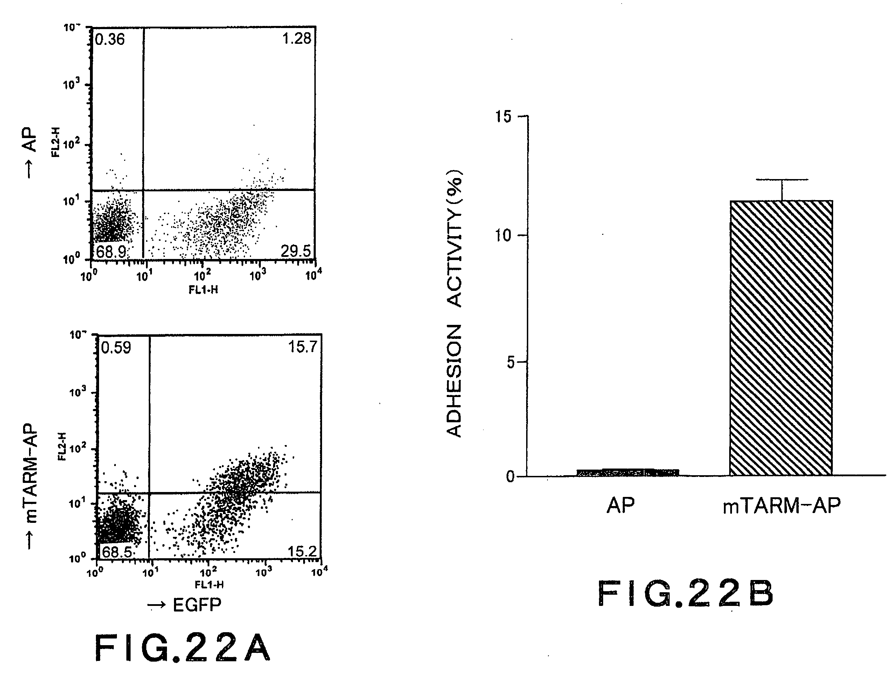

As a result, expression of mRNA of ENSMUSG00000035095 (A530065I17Rik, NM_176953) (using primer #2558: CAGCTGGCAAGAGGAACAGT (SEQ ID NO: 54) and primer #2559: GAGCATCGGCACTTATCTCC (SEQ ID NO: 55)) showed a correlation with the binding activity of the mTARM-AP chimeric protein to the cells (Figure 22B). However, no transmembrane region existed in an amino acid sequence encoded by ENSMUSG00000035095, and thus it was not considered to function as a cell membrane protein. This amino acid sequence was a putative sequence, and thus it was considered that estimation of a cDNA sequence from the genomic sequence was incorrect. Hence, it was first attempted to identify a human homologous gene product. Using an amino acid sequence encoded by ENSMUSG00000035095, database search was carried out. As a result, a human amino acid sequence LOC196264 showing high homology with the amino acid sequence encoded by ENSMUSG00000035095 was discovered. This amino acid sequence related to a cell membrane protein containing one immunoglobulin loop structure region. Accordingly, it was considered that LOC196264 was the full-length human amino acid sequence of a TARM-L candidate. Subsequently, using the amino acid sequence of LOC196264, tblastn search was carried out through the database. As a result, a region encoding a sequence showing homology with the amino acid sequence of LOC196264 was discovered in the genomic nucleotide sequence of mouse BAC clone AC122305. The cDNA sequence and amino acid sequence of a mouse TARM-L candidate were estimated from this sequence, so that the following primers were designed and that cDNA encoding a full-length protein was isolated.
#2693: CGCGTCGACGCCACCATGCAGCTGGCAAGAGGAACAGTA (SEQ ID NO.: 56)
#2694: GCGGGCGGCCGCTCAGTACGCCTCTTCTTCGTAGTC (SEQ ID NO.: 57)
The total RNA of Th1 day 2 was used as a template, and the cDNA was amplified by the method described in Example 1. The amplified cDNA was inserted into an expression vector pMXII IRES EGFP (Oncogene (2000) 19(27): 3050-3058), and an mTARM-L candidate gene expression vector was then prepared. ABI3100 Sequence Analyzer was used to determine the nucleotide sequence thereof (SEQ ID NO: 13).

### (2) Identification of mTARM-L

Next, in order to examine whether or not the mTARM-L candidate was an unknown ligand for TARM, cells, in which this molecule was to be expressed, were prepared by the method described in Example 2. The binding activity of the mTARM-AP chimeric protein onto the cell surface of mTARM-L candidate-expressing cells and the adhesion activity of the mTARM-L candidate-expressing cells to the immobilized mTARM were measured by the method described in Example 5.

As a result, the mTARM-AP specifically bound to EGFP+ mTARM-L-expressing B300.19 cells, but it did not bind to B300.19 cells, into which an EGFP+ control vector had been introduced (Figure 22A). AP used as a negative control did not bind to such EGFP+ mTARM-L-expressing B300.19 cells (Figure 22A).

Moreover, the adhesion activity of B300.19 cells expressing the mTARM-L to AP and the mTARM-AP chimeric protein was examined. As a result, it was found that the aforementioned cell adhered only to the mTARM-AP chimeric protein (Figure 22B).

Thus, the above results demonstrated that the mTARM-L candidate molecule was actually a novel ligand for TARM. This ligand was named as mTARM-L (TARM Ligand).

With regard to hTARM-L, the following primers were designed based on the nucleotide sequence of NM_198275 encoding the amino acid sequence of LOC196264, and cDNA encoding the full-length protein was isolated.
#2721: CGCGTCGACGCCACCATGCAGCAGAGAGGAGCAGCTGGA (SEQ ID NO.: 58)
#2722: GCGGGCGGCCGCTCAATATGTCTCTTCATAGTCTGA (SEQ ID NO.: 59)
Using the total RNA of bone marrow as a template, the cDNA was amplified by the method described in Example 1. The amplified cDNA was inserted into an expression vector pMXII IRES EGFP (Oncogene (2000) 19(27): 3050-3058) to prepare a TARM-L gene expression vector, and the nucleotide sequence thereof (SEQ ID NO: 15) was then determined using ABI3100 Sequence Analyzer.

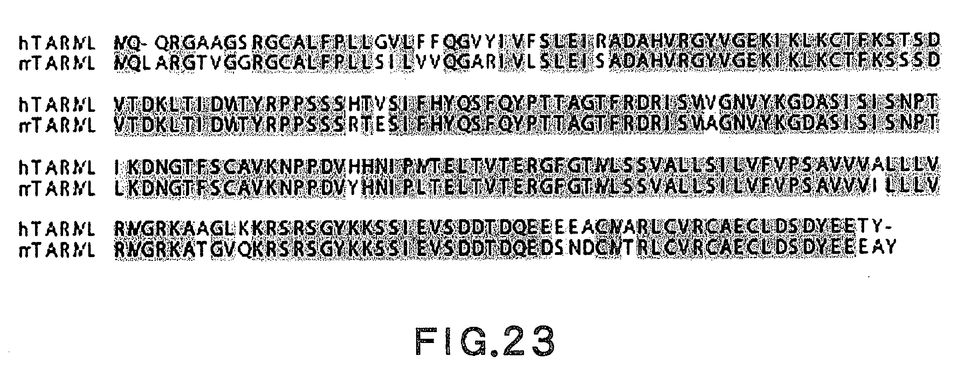

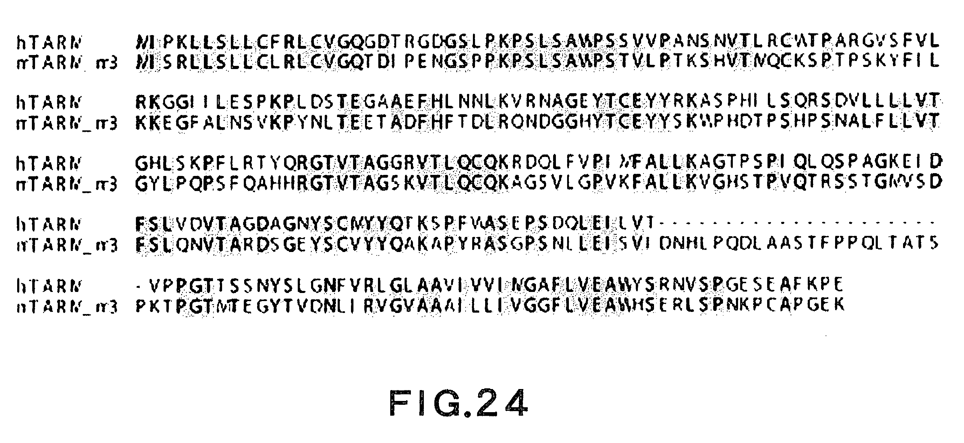

The human and mouse TARM-L were subjected to homology analysis with ClustaIW. The results are shown in Figure 23. The hTARM-L consisted of 235 amino acids, and the mTARM-L consisted of 237 amino acids. They have 86% homology. On the other hand, mTARM m3 (293 amino acids) showed the highest homology with the hTARM (271 amino acids) at a percentage of 50% (Figure 24).

## Claims

1. An antibody against a membrane or secretory protein selected from the following (ix), (x), (xi) and (xii), or a functional fragment thereof:
(ix) a membrane or secretory protein comprising the amino acid sequence of SEQ ID NO: 2, SEQ ID NO: 4, SEQ ID NO: 6, SEQ ID NO: 8, SEQ ID NO: 10 or SEQ ID NO: 12;
(x) a membrane or secretory protein which comprises an amino acid sequence of SEQ ID NO: 2, SEQ ID NO: 4, SEQ ID NO: 6, SEQ ID NO: 8, SEQ ID NO: 10 or SEQ ID NO: 12 in which one or more amino acids are inserted, substituted or deleted, or one or more amino acids are added to one or both of ends, and which is functionally equivalent to a protein consisting of the amino acid sequence of SEQ ID NO: 2, SEQ ID NO: 4, SEQ ID NO: 6, SEQ ID NO: 8, SEQ ID NO: 10 or SEQ ID NO: 12;
(xi) a membrane or secretory protein which is encoded by a polynucleotide which hybridizes under stringent conditions to a polynucleotide which encodes the amino acid sequence of SEQ ID NO: 2, SEQ ID NO: 4, SEQ ID NO: 6, SEQ ID NO: 8, SEQ ID NO: 10 or SEQ ID NO: 12, and which is functionally equivalent to a protein consisting of the amino acid sequence of SEQ ID NO: 2, SEQ ID NO: 4, SEQ ID NO: 6, SEQ ID NO: 8, SEQ ID NO: 10 or SEQ ID NO: 12; and
(xii) a membrane or secretory protein which comprises an amino acid sequence having 70% or more identity with the amino acid sequence of SEQ ID NO: 2, SEQ ID NO: 4, SEQ ID NO: 6, SEQ ID NO: 8, SEQ ID NO: 10 or SEQ ID NO: 12, and which is functionally equivalent to a protein consisting of the amino acid sequence of SEQ ID NO: 2, SEQ ID NO: 4, SEQ ID NO: 6, SEQ ID NO: 8, SEQ ID NO: 10 or SEQ ID NO: 12 for use in treating an autoimmune disease.

2. The antibody or a functional fragment thereof for use according to claim 1, which is an antibody against a membrane protein or secretory proteinselected from the following (i), (ii), (iii) and (iv), or a functional fragment thereof:
(i) a membrane or secretory protein comprising the amino acid sequence of SEQ ID NO: 10;
(ii) a membrane or secretory protein which comprises an amino acid sequence of SEQ ID NO: 10 in which one or more amino acids are inserted, substituted or deleted, or one or more amino acids are added to one or both of ends, and which is functionally equivalent to a protein consisting of the amino acid sequence of SEQ ID NO: 10;
(iii) a membrane or secretory protein which is encoded by a polynucleotide which hybridizes under stringent conditions to a polynucleotide which encodes the amino acid sequence of SEQ ID NO: 10, and which is functionally equivalent to a protein consisting of the amino acid sequence of SEQ ID NO: 10; and
(iv) a membrane or secretory protein which comprises an amino acid sequence having 90% or more identity with the amino acid sequence of SEQ ID NO: 10, and which is functionally equivalent to a protein consisting of the amino acid sequence of SEQ ID NO: 10.

3. The antibody or a functional fragment thereof for use according to claim 2, which is an antibody against a membrane or secretory protein comprising an amino acid sequence of SEQ ID NO: 10, or an amino acid sequence of SEQ ID NO: 10 which contains one or more conservative substitutions, or a functional fragment thereof.

4. The antibody or a functional fragment thereof for use according to claim 1, which is an antibody against a membrane or secretory protein selected from the following (ix'), (x'), (xi') and (xii'), or a functional fragment thereof:
(ix') a membrane or secretory protein comprising the amino acid sequence of SEQ ID NO: 12;
(x') a membrane or secretory protein which comprises an amino acid sequence of SEQ ID NO: 12 in which one or more amino acids are inserted, substituted or deleted, or one or more amino acids are added to one or both of ends, and which is functionally equivalent to a protein consisting of the amino acid sequence of SEQ ID NO: 12;
(xi') a membrane or secretory protein which is encoded by a polynucleotide which hybridizes under stringent conditions to a polynucleotide which encodes the amino acid sequence of SEQ ID NO: 12, and which is functionally equivalent to a protein consisting of the amino acid sequence of SEQ ID NO: 12; and
(xii') a membrane or secretory protein which comprises an amino acid sequence having 70% or more identity with the amino acid sequence of SEQ ID NO: 12, and which is functionally equivalent to a protein consisting of the amino acid sequence of SEQ ID NO: 12.

5. The antibody or a functional fragment thereof for use according to claim 4, which is an antibody against a membrane or secretory protein comprising the amino acid sequence of SEQ ID NO: 12, or an amino acid sequence of SEQ ID NO: 12 which contains one or more conservative substitutions, or a functional fragment thereof.

6. The antibody or a functional fragment thereof for use according to claim 5, which is produced by a hybridoma deposited under the accession No. FERM BP-10376.

7. The antibody or a functional fragment thereof for use according to any one of claims 1 to 6, wherein the antibody is for use in treating rheumatoid arthritis.

8. A hybridoma deposited under the accession No. FERM BP-10376.

9. An antibody against a protein selected from the following (xiii), (xiv), (xv) and (xvi), or a functional fragment thereof:
(xiii) a protein comprising the amino acid sequence of SEQ ID NO: 14 or SEQ ID NO: 16;
(xiv) a protein which comprises an amino acid sequence of SEQ ID NO: 14 or SEQ ID NO: 16 in which one or more amino acids are inserted, substituted or deleted, or one or more amino acids are added to one or both of ends, and which is functionally equivalent to a protein consisting of the amino acid sequence of SEQ ID NO: 14 or SEQ ID NO: 16;
(xv) a protein which is encoded by a polynucleotide which hybridizes under stringent conditions to a polynucleotide which encodes the amino acid sequence of SEQ ID NO: 14 or SEQ ID NO: 16, and which is functionally equivalent to a protein consisting of the amino acid sequence of SEQ ID NO: 14 or SEQ ID NO: 16; and
(xvi) a protein which comprises an amino acid sequence having 70% or more identity with the amino acid sequence of SEQ ID NO: 14 or SEQ ID NO: 16, and which is functionally equivalent to a protein consisting of the amino acid sequence of SEQ ID NO: 14 or SEQ ID NO: 16.

10. The antibody or a functional fragment thereof according to claim 9 for use in treating autoimmune disease.

11. The antibody or a functional fragment thereof for use according to claim 10, for use in the treatment of rheumatoid arthritis.

12. A method for screening for a substance that inhibits activation of a dendritic cell or a salt thereof, or a solvate thereof, which comprises the steps of:
(d) contacting an antibody or a functional fragment thereof according to any one of claims 1 to 7 with a dendritic cell in the presence or absence of a test substance; and
(e) measuring the level of activation of said dendritic cell.

13. The screening method according to claim 12, wherein, in step (e), the level of activation of said dendritic cell is measured using the amount of IL-6 and/or MCP-1 produced from the dendritic cell as an index.

14. The screening method according to claim 13, which further comprises the step of (f-1) comparing the amount of IL-6 and/or MCP-1 produced in the presence of a test substance with the amount of IL-6 and/or MCP-1 produced in the absence of a test substance, after step (e).

15. The screening method according to claim 12, wherein, in step (e), the level of activation of a dendritic cell is measured using the expression level of the FcRγ chain on the dendritic cell as an index.

16. The screening method according to claim 15, which further comprises the step of (f-2) comparing the expression level of the FcRγ chain in the presence of a test substance with the expression level of the FcRγ chain in the absence of a test substance, after step (e).

17. A method for screening for a substance that inhibits a complex formation between a TARM protein and the FcRγ chain or a salt thereof, or a solvate thereof, which comprises the steps of:
(g) contacting an antibody or a functional fragment thereof according to any one of claims 1 to 7 with a dendritic cell in the presence or absence of a test substance; and
(h) measuring the expression level of the FcRγ chain on said dendritic cell,
wherein said TARM protein is a membrane or secretory protein selected from the following (ix), (x), (xi) and (xii):
(ix) a membrane or secretory protein comprising the amino acid sequence of SEQ ID NO: 2, SEQ ID NO: 4, SEQ ID NO: 6, SEQ ID NO: 8, SEQ ID NO: 10 or SEQ ID NO: 12;
(x) a membrane or secretory protein which comprises an amino acid sequence of SEQ ID NO: 2, SEQ ID NO: 4, SEQ ID NO: 6, SEQ ID NO: 8, SEQ ID NO: 10 or SEQ ID NO: 12 in which one or more amino acids are inserted, substituted or deleted, or one or more amino acids are added to one or both of ends, and which is functionally equivalent to a protein consisting of the amino acid sequence of SEQ ID NO: 2, SEQ ID NO: 4, SEQ ID NO: 6, SEQ ID NO: 8, SEQ ID NO: 10 or SEQ ID NO: 12;
(xi) a membrane or secretory protein which is encoded by a polynucleotide which hybridizes under stringent conditions to a polynucleotide which encodes the amino acid sequence of SEQ ID NO: 2, SEQ ID NO: 4, SEQ ID NO: 6, SEQ ID NO: 8, SEQ ID NO: 10 or SEQ ID NO: 12, and which is functionally equivalent to a protein consisting of the amino acid sequence of SEQ ID NO: 2, SEQ ID NO: 4, SEQ ID NO: 6, SEQ ID NO: 8, SEQ ID NO: 10 or SEQ ID NO: 12; and
(xii) a membrane or secretory protein which comprises an amino acid sequence having 70% or more identity with the amino acid sequence of SEQ ID NO: 2, SEQ ID NO: 4, SEQ ID NO: 6, SEQ ID NO: 8, SEQ ID NO: 10 or SEQ ID NO: 12, and which is functionally equivalent to a protein consisting of the amino acid sequence of SEQ ID NO: 2, SEQ ID NO: 4, SEQ ID NO: 6, SEQ ID NO: 8, SEQ ID NO: 10 or SEQ ID NO: 12.

18. The screening method according to claim 17, which further comprises the step of (i) comparing the expression level of the FcRγ chain in the presence of a test substance with the expression level of the FcRγ chain in the absence of a test substance, after step (h).

## Patentansprüche

1. Antikörper gegen ein Membran- oder sekretorisches Protein, das aus den folgenden (ix), (x), (xi) und (xii) ausgewählt ist, oder funktionelles Fragment davon:
(ix) ein Membran- oder sekretorisches Protein, das die Aminosäuresequenz von SEQ ID Nr. 2, SEQ ID Nr. 4, SEQ ID Nr. 6, SEQ ID Nr. 8, SEQ ID Nr. 10 oder SEQ ID Nr. 12 umfasst;
(x) ein Membran- oder sekretorisches Protein, das eine Aminosäuresequenz von SEQ ID Nr. 2, SEQ ID Nr. 4, SEQ ID Nr. 6, SEQ ID Nr. 8, SEQ ID Nr. 10 oder SEQ ID Nr. 12 umfasst, in der eine oder mehrere Aminosäuren insertiert, substituiert oder deletiert sind oder eine oder mehrere Aminosäuren an einem oder beiden Enden hinzugefügt sind, und das zu einem Protein funktionell gleichwertig ist, das aus der Aminosäuresequenz von SEQ ID Nr. 2, SEQ ID Nr. 4, SEQ ID Nr. 6, SEQ ID Nr. 8, SEQ ID Nr. 10 oder SEQ ID Nr. 12 besteht;
(xi) ein Membran- oder sekretorisches Protein, das von einem Polynukleotid codiert wird, das unter stringenten Bedingungen an ein Polynukleotid hybridisiert, das die Aminosäuresequenz von SEQ ID Nr. 2, SEQ ID Nr. 4, SEQ ID Nr. 6, SEQ ID Nr. 8, SEQ ID Nr. 10 oder SEQ ID Nr. 12 codiert, und das zu einem Protein funktionell gleichwertig ist, das aus der Aminosäuresequenz von SEQ ID Nr. 2, SEQ ID Nr. 4, SEQ ID Nr. 6, SEQ ID Nr. 8, SEQ ID Nr. 10 oder SEQ ID Nr. 12 besteht; und
(xii) ein Membran- oder sekretorisches Protein, das eine Aminosäuresequenz mit 70 % oder mehr Identität mit der Aminosäuresequenz von SEQ ID Nr. 2, SEQ ID Nr. 4, SEQ ID Nr. 6, SEQ ID Nr. 8, SEQ ID Nr. 10 oder SEQ ID Nr. 12 umfasst und das zu einem Protein funktionell gleichwertig ist, das aus der Aminosäuresequenz von SEQ ID Nr. 2, SEQ ID Nr. 4, SEQ ID Nr. 6, SEQ ID Nr. 8, SEQ ID Nr. 10 oder SEQ ID Nr. 12 besteht; zur Verwendung bei der Behandlung einer Autoimmunerkrankung.

2. Antikörper oder funktionelles Fragment davon zur Verwendung nach Anspruch 1, der bzw. das ein Antikörper gegen ein Membranprotein oder sekretorisches Protein, das aus den folgenden (i), (ii), (iii) und (iv) ausgewählt ist, oder ein funktionelles Fragment davon ist:
(i) ein Membran- oder sekretorisches Protein, das die Aminosäuresequenz von SEQ ID N r. 10 umfasst;
(ii) ein Membran- oder sekretorisches Protein, das eine Aminosäuresequenz von SEQ ID Nr. 10 umfasst, in der eine oder mehrere Aminosäuren insertiert, substituiert oder deletiert sind oder eine oder mehrere Aminosäuren an einem oder beiden Enden hinzugefügt sind, und das zu einem Protein funktionell gleichwertig ist, das aus der Aminosäuresequenz von SEQ ID Nr. 10 besteht;
(iii) ein Membran- oder sekretorisches Protein, das von einem Polynukleotid codiert wird, das unter stringenten Bedingungen an ein Polynukleotid hybridisiert, das die Aminosäuresequenz von SEQ ID Nr. 10 codiert, und das zu einem Protein funktionell gleichwertig ist, das aus der Aminosäuresequenz von SEQ ID Nr. 10 besteht; und
(iv) ein Membran- oder sekretorisches Protein, das eine Aminosäuresequenz mit 90 % oder mehr Identität mit der Aminosäuresequenz von SEQ ID Nr. 10 umfasst und das zu einem Protein funktionell gleichwertig ist, das aus der Aminosäuresequenz von SEQ ID Nr. 10 besteht.

3. Antikörper oder funktionelles Fragment davon zur Verwendung nach Anspruch 2, der bzw. das ein Antikörper gegen ein Membran- oder sekretorisches Protein, das eine Aminosäuresequenz von SEQ ID Nr. 10 oder eine Aminosäuresequenz von SEQ ID Nr. 10, die eine oder mehrere konservative Substitutionen enthält, umfasst, oder ein funktionelles Fragment davon ist.

4. Antikörper oder funktionelles Fragment davon zur Verwendung nach Anspruch 1, der bzw. das ein Antikörper gegen ein Membran- oder sekretorisches Protein, das aus den folgenden (ix'), (x'), (xi') und (xii') ausgewählt ist, oder ein funktionelles Fragment davon ist:
(ix') ein Membran- oder sekretorisches Protein, das die Aminosäuresequenz von SEQ ID Nr. 12 umfasst;
(x') ein Membran- oder sekretorisches Protein, das eine Aminosäuresequenz von SEQ ID Nr. 12 umfasst, in der eine oder mehrere Aminosäuren insertiert, substituiert oder deletiert sind oder eine oder mehrere Aminosäuren an einem oder beiden Enden hinzugefügt sind, und das zu einem Protein funktionell gleichwertig ist, das aus der Aminosäuresequenz von SEQ ID Nr. 12 besteht;
(xi') ein Membran- oder sekretorisches Protein, das von einem Polynukleotid codiert wird, das unter stringenten Bedingungen an ein Polynukleotid hybridisiert, das die Aminosäuresequenz von SEQ ID Nr. 12 codiert, und das zu einem Protein funktionell gleichwertig ist, das aus der Aminosäuresequenz von SEQ ID Nr. 12 besteht; und
(xii') ein Membran- oder sekretorisches Protein, das eine Aminosäuresequenz mit 70 % oder mehr Identität mit der Aminosäuresequenz von SEQ ID Nr. 12 umfasst und das zu einem Protein funktionell gleichwertig ist, das aus der Aminosäuresequenz von SEQ ID Nr. 12 besteht.

5. Antikörper oder funktionelles Fragment davon zur Verwendung nach Anspruch 4, der bzw. das ein Antikörper gegen ein Membran- oder sekretorisches Protein, das die Aminosäuresequenz von SEQ ID Nr. 12 oder eine Aminosäuresequenz von SEQ ID Nr. 12, die eine oder mehrere konservative Substitutionen enthält, umfasst, oder ein funktionelles Fragment davon ist.

6. Antikörper oder funktionelles Fragment davon zur Verwendung nach Anspruch 5, der bzw. das durch ein Hybridom produziert wird, das unter der Zugangsnr. FERM BP-10376 hinterlegt ist.

7. Antikörper oder funktionelles Fragment davon zur Verwendung nach einem der Ansprüche 1 bis 6, wobei der Antikörper zur Verwendung bei der Behandlung von rheumatoider Arthritis ist.

8. Hybridom, das unter der Zugangsnr. FERM BP-10376 hinterlegt ist.

9. Antikörper gegen ein Protein, das aus den folgenden (xiii), (xiv), (xv) und (xvi) ausgewählt ist, oder funktionelles Fragment davon:
(xiii) ein Protein, das die Aminosäuresequenz von SEQ ID Nr. 14 oder SEQ ID Nr. 16 umfasst;
(xiv) ein Protein, das eine Aminosäuresequenz von SEQ ID Nr. 14 oder SEQ ID Nr. 16 umfasst, in der eine oder mehrere Aminosäuren insertiert, substituiert oder deletiert sind oder eine oder mehrere Aminosäuren an einem oder beiden Enden hinzugefügt sind, und das zu einem Protein funktionell gleichwertig ist, das aus der Aminosäuresequenz von SEQ ID Nr. 14 oder SEQ ID Nr. 16 besteht;
(xv) ein Protein, das von einem Polynukleotid codiert wird, das unter stringenten Bedingungen an ein Polynukleotid hybridisiert, das die Aminosäuresequenz von SEQ ID Nr. 14 oder SEQ ID Nr. 16 codiert, und das zu einem Protein funktionell gleichwertig ist, das aus der Aminosäuresequenz von SEQ ID Nr. 14 oder SEQ ID Nr. 16 besteht; und
(xvi) ein Protein, das eine Aminosäuresequenz mit 70 % oder mehr Identität mit der Aminosäuresequenz von SEQ ID Nr. 14 oder SEQ ID Nr. 16 umfasst und das zu einem Protein funktionell gleichwertig ist, das aus der Aminosäuresequenz von SEQ ID Nr. 14 oder SEQ ID Nr. 16 besteht.

10. Antikörper oder funktionelles Fragment davon nach Anspruch 9 zur Verwendung bei der Behandlung einer Autoimmunerkrankung.

11. Antikörper oder funktionelles Fragment davon zur Verwendung nach Anspruch 10 zur Verwendung bei der Behandlung von rheumatoider Arthritis.

12. Verfahren zum Screenen auf eine Substanz, die die Aktivierung einer dendritischen Zelle hemmt, oder ein Salz davon oder ein Solvat davon, wobei das Verfahren die folgenden Schritte umfasst:
(d) Inkontaktbringen eines Antikörpers oder eines funktionellen Fragments davon nach einem der Ansprüche 1 bis 7 mit einer dendritischen Zelle in Gegenwart oder Abwesenheit einer Testsubstanz; und
(e) Messen des Grads der Aktivierung der dendritischen Zelle.

13. Screeningverfahren nach Anspruch 12, wobei der Grad der Aktivierung der dendritischen Zelle in Schritt (e) unter Verwendung der Menge von IL-6 und/oder MCP-1, die von der dendritischen Zelle produziert wird, als einem Index gemessen wird.

14. Screeningverfahren nach Anspruch 13, das weiterhin den Schritt (f-1) des Vergleichens der Menge von IL-6 und/oder MCP-1, die in Gegenwart einer Testsubstanz produziert wird, mit der Menge von IL-6 und/oder MCP-1, die in Abwesenheit einer Testsubstanz produziert wird, nach Schritt (e) umfasst.

15. Screeningverfahren nach Anspruch 12, wobei der Grad der Aktivierung der dendritischen Zelle in Schritt (e) unter Verwendung des Expressionsniveaus der FcRγ-Kette an der dendritischen Zelle als einem Index gemessen wird.

16. Screeningverfahren nach Anspruch 15, das weiterhin den Schritt (f-2) des Vergleichens des Expressionsniveaus der FcRγ-Kette in Gegenwart einer Testsubstanz mit dem Expressionsniveau der FcRγ-Kette in Abwesenheit einer Testsubstanz nach Schritt (e) umfasst.

17. Verfahren zum Screenen auf eine Substanz, die eine Komplexbildung zwischen einem TARM-Protein und der FcRγ-Kette hemmt, oder ein Salz davon oder ein Solvat davon, wobei das Verfahren die folgenden Schritte umfasst:
(g) Inkontaktbringen eines Antikörpers oder eines funktionellen Fragments davon nach einem der Ansprüche 1 bis 7 mit einer dendritischen Zelle in Gegenwart oder Abwesenheit einer Testsubstanz; und
(h) Messen des Expressionsniveaus der FcRγ-Kette an der dendritischen Zelle,
wobei das TARM-Protein ein Membran- oder sekretorisches Protein ist, das aus den folgenden (ix), (x), (xi) und (xii) ausgewählt ist:
(ix) ein Membran- oder sekretorisches Protein, das die Aminosäuresequenz von SEQ ID Nr. 2, SEQ ID Nr. 4, SEQ ID Nr. 6, SEQ ID Nr. 8, SEQ ID Nr. 10 oder SEQ ID Nr. 12 umfasst;
(x) ein Membran- oder sekretorisches Protein, das eine Aminosäuresequenz von SEQ ID Nr. 2, SEQ ID Nr. 4, SEQ ID Nr. 6, SEQ ID Nr. 8, SEQ ID Nr. 10 oder SEQ ID Nr. 12 umfasst, in der eine oder mehrere Aminosäuren insertiert, substituiert oder deletiert sind oder eine oder mehrere Aminosäuren an einem oder beiden Enden hinzugefügt sind, und das zu einem Protein funktionell gleichwertig ist, das aus der Aminosäuresequenz von SEQ ID Nr. 2, SEQ ID Nr. 4, SEQ ID Nr. 6, SEQ ID Nr. 8, SEQ ID Nr. 10 oder SEQ ID Nr. 12 besteht;
(xi) ein Membran- oder sekretorisches Protein, das von einem Polynukleotid codiert wird, das unter stringenten Bedingungen an ein Polynukleotid hybridisiert, das die Aminosäuresequenz von SEQ ID Nr. 2, SEQ ID Nr. 4, SEQ ID Nr. 6, SEQ ID Nr. 8, SEQ ID Nr. 10 oder SEQ ID Nr. 12 codiert, und das zu einem Protein funktionell gleichwertig ist, das aus der Aminosäuresequenz von SEQ ID Nr. 2, SEQ ID Nr. 4, SEQ ID Nr. 6, SEQ ID Nr. 8, SEQ ID Nr. 10 oder SEQ ID Nr. 12 besteht; und
(xii) ein Membran- oder sekretorisches Protein, das eine Aminosäuresequenz mit 70 % oder mehr Identität mit der Aminosäuresequenz von SEQ ID Nr. 2, SEQ ID Nr. 4, SEQ ID Nr. 6, SEQ ID Nr. 8, SEQ ID Nr. 10 oder SEQ ID Nr. 12 umfasst und das zu einem Protein funktionell gleichwertig ist, das aus der Aminosäuresequenz von SEQ ID Nr. 2, SEQ ID Nr. 4, SEQ ID Nr. 6, SEQ ID Nr. 8, SEQ ID Nr. 10 oder SEQ ID Nr. 12 besteht.

18. Screeningverfahren nach Anspruch 17, das weiterhin den Schritt (i) des Vergleichens des Expressionsniveaus der FcRγ-Kette in Gegenwart einer Testsubstanz mit dem Expressionsniveau der FcRγ-Kette in Abwesenheit einer Testsubstanz nach Schritt (h) umfasst.

## Revendications

1. Anticorps dirigé contre une protéine membranaire ou sécrétrice sélectionnée parmi le groupe constitué des protéines (ix), (x), (xi) et (xii) suivantes, ou un fragment fonctionnel de celui-ci :
(ix) protéine membranaire ou sécrétrice comprenant la séquence d'acides aminés représentée par la SÉQ ID N° 2, la SÉQ ID N° 4, la SÉQ ID N° 6, la SÉQ ID N° 8, la SÉQ ID N° 10 ou la SÉQ ID N° 12 ;
(x) protéine membranaire ou sécrétrice qui comprend une séquence d'acides aminés représentée par la SÉQ ID N° 2, la SÉQ ID N° 4, la SÉQ ID N° 6, la SÉQ ID N° 8, la SÉQ ID N° 10 ou la SÉQ ID N° 12, dans laquelle un ou plusieurs acide(s) aminé(s) est(sont) inséré(s), substitué(s) ou délété(s), ou un ou plusieurs acide(s) aminé(s) est(sont) ajouté(s) à une ou aux deux extrémité(s), et qui est fonctionnellement équivalente à une protéine constituée de la séquence d'acides aminés représentée par la SÉQ ID N° 2, la SÉQ ID N° 4, la SÉQ ID N° 6, la SÉQ ID N° 8, la SÉQ ID N° 10 ou la SÉQ ID N° 12 ;
(xi) protéine membranaire ou sécrétrice qui est codée par un polynucléotide qui s'hybride sous conditions rigoureuses à un polynucléotide qui code pour la séquence d'acides aminés représentée par la SÉQ ID N° 2, la SÉQ ID N° 4, la SÉQ ID N° 6, la SÉQ ID N° 8, la SÉQ ID N° 10 ou la SÉQ ID N° 12, et qui est fonctionnellement équivalente à une protéine constituée de la séquence d'acides aminés représentée par la SÉQ ID N° 2, la SÉQ ID N° 4, la SÉQ ID N° 6, la SÉQ ID N° 8, la SÉQ ID N° 10 ou la SÉQ ID N° 12 ; et
(xii) protéine membranaire ou sécrétrice qui comprend une séquence d'acides aminés dont l'identité de séquence avec la séquence d'acides aminés représentée par la SÉQ ID N° 2, la SÉQ ID N° 4, la SÉQ ID N° 6, la SÉQ ID N° 8, la SÉQ ID N° 10 ou la SÉQ ID N° 12 est égale ou supérieure à 70 %, et qui est fonctionnellement équivalente à une protéine constituée de la séquence d'acides aminés représentée par la SÉQ ID N° 2, la SÉQ ID N° 4, la SÉQ ID N° 6, la SÉQ ID N° 8, la SÉQ ID N° 10 ou la SÉQ ID N° 12,
à utiliser dans le traitement d'une maladie autoimmune.

2. Anticorps ou fragment fonctionnel de celui-ci à utiliser selon la revendication 1, qui est un anticorps dirigé contre une protéine membranaire ou une protéine sécrétrice sélectionnée parmi le groupe constitué des protéines (i), (ii), (iii) et (iv) suivantes, ou un fragment fonctionnel de celui-ci :
(i) protéine membranaire ou sécrétrice comprenant la séquence d'acides aminés représentée par la SÉQ ID N° 10 ;
(ii) protéine membranaire ou sécrétrice qui comprend une séquence d'acides aminés représentée par la SÉQ ID N° 10, dans laquelle un ou plusieurs acide(s) aminé(s) est(sont) insérés(s), substitué(s) ou délété(s), ou un ou plusieurs acide(s) aminé(s) est(sont) ajouté(s) à une ou aux deux extrémité(s), et qui est fonctionnellement équivalente à une protéine constituée de la séquence d'acides aminés représentée par la SÉQ ID N° 10 ;
(iii) protéine membranaire ou sécrétrice qui est codée par une polynucléotide qui s'hybride sous conditions rigoureuses à un polynucléotide qui code pour la séquence d'acides aminés représentée par la SÉQ ID N° 10, et qui est fonctionnellement équivalente à une protéine constituée de la séquence d'acides aminés représentée par la SÉQ ID N° 10 ; et
(iv) protéine membranaire ou sécrétrice qui comprend une séquence d'acides aminés dont l'identité de séquence avec la séquence d'acides aminés représentée par la SÉQ ID N° 10 est égale ou supérieure à 90 %, et qui est fonctionnellement équivalente à une protéine constituée de la séquence d'acides aminés représentée par la SÉQ ID N° 10.

3. Anticorps ou fragment fonctionnel de celui-ci à utiliser selon la revendication 2, qui est un anticorps dirigé contre une protéine membranaire ou sécrétrice comprenant une séquence d'acides aminés représentée par la SÉQ ID N° 10 , ou une séquence d'acides aminés représentée par la SÉQ ID N° 10 qui contient une ou plusieurs substitution(s) conservatrice(s), ou un fragment fonctionnel de celui-ci.

4. Anticorps ou fragment fonctionnel de celui-ci à utiliser selon la revendication 1, qui est un anticorps dirigé contre une protéine membranaire ou sécrétrice sélectionnée parmi le groupe constitué des protéines (ix'), (x'), (xi') et (xii') suivantes, ou un fragment fonctionnel de celui-ci :
(ix') protéine membranaire ou sécrétrice comprenant la séquence d'acides aminés représentée par la SÉQ ID N° 12 ;
(x') protéine membranaire ou sécrétrice qui comprend une séquence d'acides aminés représentée par la SÉQ ID N° 12, dans laquelle un ou plusieurs acide(s) aminé(s) est(sont) inséré(s), substitué(s) ou délété(s), ou un ou plusieurs acides aminés est(sont) ajouté(s) à une ou aux deux extrémité(s), et qui est fonctionnellement équivalente à une protéine constitué de la séquence d'acides aminés représentée par la SÉQ ID N° 12 ;
(xi') protéine membranaire ou sécrétrice qui est codée par un polynucléotide qui s'hybride sous conditions rigoureuses à un polynucléotide qui code pour la séquence d'acides aminés représentée par la SÉQ ID N° 12, et qui est fonctionnellement équivalente à une protéine constituée de la séquence d'acides aminés représentée par la SÉQ ID N° 12 ; et
(xii') protéine membranaire ou sécrétrice qui comprend une séquence d'acides aminés dont l'identité de séquence avec la séquence d'acides aminés représentée par la SÉQ ID N° 12 est égale ou supérieure à 70 %, et qui est fonctionnellement équivalente à une protéine constituée de la séquence d'acides aminés représentée par la SÉQ ID N° 12.

5. Anticorps ou fragment fonctionnel de celui-ci à utiliser selon la revendication 4, qui est un anticorps dirigé contre une protéine membranaire ou sécrétrice comprenant la séquence d'acides aminés représentée par la SÉQ ID N° 12, ou une séquence d'acides aminés représentée par la SÉQ ID N° 12 qui contient une ou plusieurs substitution(s) conservatrice(s), ou un fragment fonctionnel de celui-ci.

6. Anticorps ou fragment fonctionnel de celui-ci à utiliser selon la revendication 5, qui est produit par un hybridome déposé sous le numéro d'entrée FERM BP-10376.

7. Anticorps ou fragment fonctionnel de celui-ci à utiliser selon l'une quelconque des revendications 1 à 6, ledit anticorps étant utilisé dans le traitement de la polyarthrite rhumatoïde.

8. Hybridome déposé sous le numéro d'entrée FERM BP-10376.

9. Anticorps dirigé contre une protéine sélectionnée parmi le groupe constitué des protéines (xiii), (xiv), (xv) et (xvi) suivantes, ou fragment fonctionnel de celui-ci :
(xiii) protéine comprenant la séquence d'acides aminés représentée par la SÉQ ID N° 14 ou la SÉQ ID N° 16 ;
(xiv) protéine qui comprend une séquence d'acides aminés représentée par la SÉQ ID N° 14 ou la SÉQ ID N° 16 dans laquelle un ou plusieurs acide(s) aminé(s) est(sont) inséré(s), substitué(s) ou délété(s), ou un ou plusieurs acides aminés est(sont) ajouté(s) à une ou aux deux extrémité(s), et qui est fonctionnellement équivalente à une protéine constituée de la séquence d'acides aminés représentée par la SÉQ ID N° 14 ou la SÉQ ID N° 16 ;
(xv) protéine qui est codée par un polynucléotide qui s'hybride sous conditions rigoureuses à un polynucléotide qui code pour la séquence d'acides aminés représentée par la SÉQ ID N° 14 ou la SÉQ ID N° 16, et qui est fonctionnellement équivalente à une protéine constituée de la séquence d'acides aminés représentée par la SÉQ ID N° 14 ou la SÉQ ID N° 16; et
(xvi) protéine qui comprend une séquence d'acides aminés dont l'identité de séquence avec la séquence d'acides aminés représentée par la SÉQ ID N° 14 ou la SÉQ ID N° 16 est égale ou supérieure à 70 %, et qui est fonctionnellement équivalente à une protéine constituée de la séquence d'acides aminés représentée par la SÉQ ID N° 14 ou la SÉQ ID N° 16.

10. Anticorps ou fragment fonctionnel de celui-ci selon la revendication 9, à utiliser dans le traitement d'une maladie autoimmune.

11. Anticorps ou fragment fonctionnel de celui-ci à utiliser selon la revendication 10, à utiliser dans le traitement de la polyarthrite rhumatoïde.

12. Méthode de détection d'une substance qui inhibe l'activation d'une cellule dendritique ou d'un sel de celle-ci, ou d'un solvate de celle-ci, qui comprend les étapes qui consistent à :
(d) mettre un anticorps ou un fragment fonctionnel de celui-ci selon l'une quelconque des revendications 1 à 7 en contact avec une cellule dendritique en la présence ou en l'absence d'une substance test ; et
(e) mesurer le degré d'activation de ladite cellule dendritique.

13. Méthode de détection selon la revendication 12, dans laquelle, à l'étape (e), le degré d'activation de ladite cellule dendritique est mesuré en utilisant la quantité d'IL-6 et/ou de MCP-1 produite à partir de la cellule dendritique à titre d'indice.

14. Méthode de détection selon la revendication 13, qui comprend, en outre, l'étape (f-1) qui consiste à comparer la quantité d'IL-6 et/ou de MCP-1 produite en la présence d'une substance test avec la quantité d'IL-6 et/ou de MCP-1 produite en l'absence d'une substance test, après l'étape (e).

15. Méthode de détection selon la revendication 12, dans laquelle, à l'étape (e), le degré d'activation d'une cellule dendritique est mesuré en utilisant le degré d'expression de la chaîne FcRγ sur la cellule dendritique à titre d'indice.

16. Méthode de détection selon la revendication 15, qui comprend, en outre, l'étape (f-1) qui consiste à comparer le degré d'expression de la chaîne FcRγ en la présence d'une substance test avec le degré d'expression de la chaîne FcRγ en l'absence d'une substance test, après l'étape (e).

17. Méthode de détection d'une substance qui inhibe la formation d'un complexe entre une protéine TARM et la chaîne FcRγ ou d'un sel de celle-ci, ou d'un solvate de celle-ci, qui comprend les étapes qui consistent à :
(g) mettre un anticorps ou un fragment fonctionnel de celui-ci selon l'une quelconque des revendications 1 à 7 en contact avec une cellule dendritique en la présence ou en l'absence d'une substance test ; et
(h) mesurer le degré d'expression de la chaîne FcRγ sur ladite cellule dendritique,
dans laquelle ladite protéine TARM est une protéine membranaire ou sécrétrice sélectionnée parmi le groupe constitué des protéines (ix), (x), (xi) et (xii) suivantes :
(ix) protéine membranaire ou sécrétrice comprenant la séquence d'acides aminés représentée par la SÉQ ID N° 2, la SÉQ ID N° 4, la SÉQ ID N° 6, la SÉQ ID N° 8, la SÉQ ID N° 10 ou la SÉQ ID N° 12 ;
(x) protéine membranaire ou sécrétrice qui comprend une séquence d'acides aminés représentée par la SÉQ ID N° 2, la SÉQ ID N° 4, la SÉQ ID N° 6, la SÉQ ID N° 8, la SÉQ ID N° 10 ou la SÉQ ID N° 12, dans laquelle un ou plusieurs acide(s) aminé(s) est(sont)inséré(s), substitué(s) ou délété(s), ou un ou plusieurs acide(s) aminé(s) est(sont) ajouté(s) à une ou aux deux extrémité(s), et qui est fonctionnellement équivalente à une protéine constituée de la séquence d'acides aminés représentée par la SÉQ ID N° 2, la SÉQ ID N° 4, la SÉQ ID N° 6, la SÉQ ID N° 8, la SÉQ ID N° 10 ou la SÉQ ID N° 12 ;
(xi) protéine membranaire ou sécrétrice qui est codée par un polynucléotide qui s'hybride sous conditions rigoureuses à un polynucléotide qui code pour la séquence d'acides aminés représentée par la SÉQ ID N° 2, la SÉQ ID N° 4, la SÉQ ID N° 6, la SÉQ ID N° 8, la SÉQ ID N° 10 ou la SÉQ ID N° 12, et qui est fonctionnellement équivalente à une protéine constituée de la séquence d'acides aminés représentée par la SÉQ ID N° 2, la SÉQ ID N° 4, la SÉQ ID N° 6, la SÉQ ID N° 8, la SÉQ ID N° 10 ou la SÉQ ID N° 12 ; et
(xii) protéine membranaire ou sécrétrice qui comprend une séquence d'acides aminés dont l'identité de séquence avec la séquence d'acides aminés représentée par la SÉQ ID N° 2, la SÉQ ID N° 4, la SÉQ ID N° 6, la SÉQ ID N° 8, la SÉQ ID N° 10 ou la SÉQ ID N° 12 est égale ou supérieure à 70 %, et qui est fonctionnellement équivalente à une protéine constituée de la séquence d'acides aminés représentée par la SÉQ ID N° 2, la SÉQ ID N° 4, la SÉQ ID N° 6, la SÉQ ID N° 8, la SÉQ ID N° 10 ou la SÉQ ID N° 12.

18. Méthode de détection selon la revendication 17, qui comprend, en outre, l'étape (f-1) qui consiste à comparer le degré d'expression de la chaîne FcRγ en la présence d'une substance test avec le degré d'expression de la chaîne FcRγ en l'absence d'une substance test, après l'étape (h).


### SEQUENCE LISTING

<110> Eisai R&D Management Co., Ltd.
<120> T cell adhesion molecules and antibodies thereto
<130> 162119PX
<160> 59
<170> PatentIn version 3.1
<210> 1
   <211> 889
   <212> DNA
   <213> Mus musculus
<220>
   <221> CDS
   <222> (49)..(879)
   <223>
<400> 1
   gctgatagta gacctgctga agacctttgg accagccgct gagccacc atg atc tct        57
                                                        Met Ile Ser
                                                        1
   agg ctc ctt tcc ctt ctc tgc ctc cgg tct cct ccc aag ccc agc ctc        105
   Arg Leu Leu Ser Leu Leu Cys Leu Arg Ser Pro Pro Lys Pro Ser Leu
       5                   10                  15
   agt gcc tgg ccc agc aca gtg ctt ccc acc aag agc cac gtg aca atg        153
   Ser Ala Trp Pro Ser Thr Val Leu Pro Thr Lys Ser His Val Thr Met
   20                  25                  30                  35
   caa tgt aag agc ccc acc ccg agt aaa tac ttc atc ctc aaa aag gaa        201
   Gln Cys Lys Ser Pro Thr Pro Ser Lys Tyr Phe Ile Leu Lys Lys Glu
                   40                  45                  50
   ggt ttc gct ttg aat tct gtg aag cca tat aat ttg aca gag gag acg        249
   Gly Phe Ala Leu Asn Ser Val Lys Pro Tyr Asn Leu Thr Glu Glu Thr
               55                  60                  65
   gct gat ttt cat ttc acc gac cta cga cag aat gat ggc gga cac tac        297
   Ala Asp Phe His Phe Thr Asp Leu Arg Gln Asn Asp Gly Gly His Tyr
           70                  75                  80
   acc tgt gaa tac tat agc aaa tgg ccc cat gac aca ccg tca cac ccc        345
   Thr Cys Glu Tyr Tyr Ser Lys Trp Pro His Asp Thr Pro Ser His Pro
       85                  90                  95
   agc aat gcc ctt ttc ttg ttg gtc aca ggg tac tta cct cag ccc tcc        393
   Ser Asn Ala Leu Phe Leu Leu Val Thr Gly Tyr Leu Pro Gln Pro Ser
   100                 105                 110                 115
   ttt caa gcc cac cac cgg ggt aca gtg act gca gga agc aag gtg act        441
   Phe Gln Ala His His Arg Gly Thr Val Thr Ala Gly Ser Lys Val Thr
                   120                 125                 130
   ttg cag tgc cag aaa gca ggc agt gtc ctc gga ccc gta aag ttt gcg        489
   Leu Gln Cys Gln Lys Ala Gly Ser Val Leu Gly Pro Val Lys Phe Ala
                   135                 140                 145
   tta ctg aag gtg gga cac tca act cct gtg cag aca agg agc tca aca      537
   Leu Leu Lys Val Gly His Ser Thr Pro Val Gln Thr Arg Ser Ser Thr
           150                 155                 160
   gga atg gta tca gac ttc tct ctt cag aat gtg aca gcc aga gac tcg      585
   Gly Met Val Ser Asp Phe Ser Leu Gln Asn Val Thr Ala Arg Asp Ser
       165                 170                 175
   ggg gaa tac agc tgt gtt tac tat cag gca aag gct ccc tat cgg gcc      633
   Gly Glu Tyr Ser Cys Val Tyr Tyr Gln Ala Lys Ala Pro Tyr Arg Ala
   180                 185                 190                 195
   tca ggg ccc agc aat ctc ctt gag atc tct gtg ata gac aac cat ctg      681
   Ser Gly Pro Ser Asn Leu Leu Glu Ile Ser Val Ile Asp Asn His Leu
                   200                 205                 210
   cct caa gat ctt gct gcc tcg act ttc cca ccg caa ctg aca gca acc      729
   Pro Gln Asp Leu Ala Ala Ser Thr Phe Pro Pro Gln Leu Thr Ala Thr
               215                 220                 225
   tca ccc aag acc ccg ggt aca atg aca gaa ggc tac act gtg gat aat      777
   Ser Pro Lys Thr Pro Gly Thr Met Thr Glu Gly Tyr Thr Val Asp Asn
           230                 235                 240
   ctc atc cgg gtc ggt gtg gct gct gca atc ctg cta ata gtg gga ggc      825
   Leu Ile Arg Val Gly Val Ala Ala Ala Ile Leu Leu Ile Val Gly Gly
       245                 250                 255
   ttc ctg gtt gaa gcc tgg cac agt gag cgg ctg tct cca aat aaa ccc      873
   Phe Leu Val Glu Ala Trp His Ser Glu Arg Leu Ser Pro Asn Lys Pro
   260                 265                 270                 275
   tgg taa aataactgaa                                                   889
   Trp
<210> 2
   <211> 276
   <212> PRT
   <213> Mus musculus
<400> 2
   Met Ile Ser Arg Leu Leu Ser Leu Leu Cys Leu Arg Ser Pro Pro Lys
   1               5                   10                  15
   Pro Ser Leu Ser Ala Trp Pro Ser Thr Val Leu Pro Thr Lys Ser His
               20                  25                  30
   Val Thr Met Gln Cys Lys Ser Pro Thr Pro Ser Lys Tyr Phe Ile Leu
           35                  40                  45
   Lys Lys Glu Gly Phe Ala Leu Asn Ser Val Lys Pro Tyr Asn Leu Thr
       50                  55                  60
   Glu Glu Thr Ala Asp Phe His Phe Thr Asp Leu Arg Gln Asn Asp Gly
   65                  70                  75                  80
   Gly His Tyr Thr Cys Glu Tyr Tyr Ser Lys Trp Pro His Asp Thr Pro
                   85                  90                  95
   Ser His Pro Ser Asn Ala Leu Phe Leu Leu Val Thr Gly Tyr Leu Pro
               100                 105                 110
   Gln Pro Ser Phe Gln Ala His His Arg Gly Thr Val Thr Ala Gly Ser
           115                 120                 125
   Lys Val Thr Leu Gln Cys Gln Lys Ala Gly Ser Val Leu Gly Pro Val
       130                 135                 140
   Lys Phe Ala Leu Leu Lys Val Gly His Ser Thr Pro Val Gln Thr Arg
   145                 150                 155                 160
   Ser Ser Thr Gly Met Val Ser Asp Phe Ser Leu Gln Asn Val Thr Ala
                   165                 170                 175
   Arg Asp Ser Gly Glu Tyr Ser Cys Val Tyr Tyr Gln Ala Lys Ala Pro
               180                 185                 190
   Tyr Arg Ala Ser Gly Pro Ser Asn Leu Leu Glu Ile Ser Val Ile Asp
           195                 200                 205
   Asn His Leu Pro Gln Asp Leu Ala Ala Ser Thr Phe Pro Pro Gln Leu
       210                 215                 220
   Thr Ala Thr Ser Pro Lys Thr Pro Gly Thr Met Thr Glu Gly Tyr Thr
   225                 230                 235                 240
   Val Asp Asn Leu Ile Arg Val Gly Val Ala Ala Ala Ile Leu Leu Ile
                   245                 250                 255
   Val Gly Gly Phe Leu Val Glu Ala Trp His Ser Glu Arg Leu Ser Pro
               260                 265                 270
   Asn Lys Pro Trp
           275
<210> 3
   <211> 1054
   <212> DNA
   <213> Mus musculus
<220>
   <221> CDS
   <222> (49)..(930)
   <223>
<400> 3
   gctgatagta gacctgctga agacctttgg accagccgct gagccacc atg atc tct      57
                                                        Met Ile Ser
                                                        1
   agg ctc ctt tcc ctt ctc tgc ctc cgg ctg tgt gtt ggg caa aca gac      105
   Arg Leu Leu Ser Leu Leu Cys Leu Arg Leu Cys Val Gly Gln Thr Asp
       5                   10                  15
   att cct gaa aat ggg tct cct ccc aag ccc agc ctc agt gcc tgg ccc      153
   Ile Pro Glu Asn Gly Ser Pro Pro Lys Pro Ser Leu Ser Ala Trp Pro
   20                  25                  30                  35
   agc aca gtg ctt ccc acc aag agc cac gtg aca atg caa tgt aag agc      201
   Ser Thr Val Leu Pro Thr Lys Ser His Val Thr Met Gln Cys Lys Ser
                   40                  45                  50
   ccc acc ccg agt aaa tac ttc atc ctc aaa aag gaa ggt ttc gct ttg      249
   Pro Thr Pro Ser Lys Tyr Phe Ile Leu Lys Lys Glu Gly Phe Ala Leu
               55                  60                  65
   aat tct gtg aag cca tat aat ttg aca gag gag acg gct gat ttt cat      297
   Asn Ser Val Lys Pro Tyr Asn Leu Thr Glu Glu Thr Ala Asp Phe His
           70                  75                  80
   ttc acc gac cta cga cag aat gat ggc gga cac tac acc tgt gaa tac      345
   Phe Thr Asp Leu Arg Gln Asn Asp Gly Gly His Tyr Thr Cys Glu Tyr
       85                  90                  95
   tat agc aaa tgg ccc cat gac aca ccg tca cac ccc agc aat gcc ctt      393
   Tyr Ser Lys Trp Pro His Asp Thr Pro Ser His Pro Ser Asn Ala Leu
   100                 105                 110                 115
   ttc ttg ttg gtc aca ggg tac tta cct cag ccc tcc ttt caa gcc cac      441
   Phe Leu Leu Val Thr Gly Tyr Leu Pro Gln Pro Ser Phe Gln Ala His
                   120                 125                 130
   cac cgg ggt aca gtg act gca gga agc aag gtg act ttg cag tgc cag      489
   His Arg Gly Thr Val Thr Ala Gly Ser Lys Val Thr Leu Gln Cys Gln
               135                 140                 145
   aaa gca ggc agt gtc ctc gga ccc gta aag ttt gcg tta ctg aag gtg      537
   Lys Ala Gly Ser Val Leu Gly Pro Val Lys Phe Ala Leu Leu Lys Val
           150                 155                 160
   gga cac tca act cct gtg cag aca agg agc tca aca gga atg gta tca      585
   Gly His Ser Thr Pro Val Gln Thr Arg Ser Ser Thr Gly Met Val Ser
       165                 170                 175
   gac ttc tct ctt cag aat gtg aca gcc aga gac tcg ggg gaa tac agc      633
   Asp Phe Ser Leu Gln Asn Val Thr Ala Arg Asp Ser Gly Glu Tyr Ser
   180                 185                 190                 195
   tgt gtt tac tat cag gca aag gct ccc tat cgg gcc tca ggg ccc agc      681
   Cys Val Tyr Tyr Gln Ala Lys Ala Pro Tyr Arg Ala Ser Gly Pro Ser
                   200                 205                 210
   aat ctc ctt gag atc tct gtg ata gac aac cat ctg cct caa gat ctt      729
   Asn Leu Leu Glu Ile Ser Val Ile Asp Asn His Leu Pro Gln Asp Leu
               215                 220                 225
   gct gcc tcg act ttc cca ccg caa ctg aca gca acc tca ccc aag acc      777
   Ala Ala Ser Thr Phe Pro Pro Gln Leu Thr Ala Thr Ser Pro Lys Thr
           230                 235                 240
   ccg ggt aca atg aca gaa ggc tac act gtg gat aat ctc atc cgg gtc      825
   Pro Gly Thr Met Thr Glu Gly Tyr Thr Val Asp Asn Leu Ile Arg Val
       245                 250                 255
   ggt gtg gct gct gca atc ctg cta ata gtg gga ggc ttc ctg gtt gaa      873
   Gly Val Ala Ala Ala Ile Leu Leu Ile Val Gly Gly Phe Leu Val Glu
   260                 265                 270                 275
   gcc tgg cac agt gag cgg ctg tct cca aat aaa ccc tgt gcc cca gga      921
   Ala Trp His Ser Glu Arg Leu Ser Pro Asn Lys Pro Cys Ala Pro Gly
                   280                 285                 290
   gaa aaa tga atcttcggac caaactatct ctgtgaattt atgtgaaatt              970
   Glu Lys
   gatgcagcac tttgggaatc atccagagac aggctgcctc atcctgactc ttcacacaga   1030
   acagaggcct ggacatatct ggac                                          1054
<210> 4
   <211> 293
   <212> PRT
   <213> Mus musculus
<400> 4
   Met Ile Ser Arg Leu Leu Ser Leu Leu Cys Leu Arg Leu Cys Val Gly
   1               5                   10                  15
   Gln Thr Asp Ile Pro Glu Asn Gly Ser Pro Pro Lys Pro Ser Leu Ser
               20                  25                  30
   Ala Trp Pro Ser Thr Val Leu Pro Thr Lys Ser His Val Thr Met Gln
           35                  40                  45
   Cys Lys Ser Pro Thr Pro Ser Lys Tyr Phe Ile Leu Lys Lys Glu Gly
       50                  55                  60
   Phe Ala Leu Asn Ser Val Lys Pro Tyr Asn Leu Thr Glu Glu Thr Ala
   65                  70                  75                      80
   Asp Phe His Phe Thr Asp Leu Arg Gln Asn Asp Gly Gly His Tyr Thr
                   85                  90                  95
   Cys Glu Tyr Tyr Ser Lys Trp Pro His Asp Thr Pro Ser His Pro Ser
               100                 105                 110
   Asn Ala Leu Phe Leu Leu Val Thr Gly Tyr Leu Pro Gln Pro Ser Phe
           115                 120                 125
   Gln Ala His His Arg Gly Thr Val Thr Ala Gly Ser Lys Val Thr Leu
       130                 135                 140
   Gln Cys Gln Lys Ala Gly Ser Val Leu Gly Pro Val Lys Phe Ala Leu
   145                 150                 155                 160
   Leu Lys Val Gly His Ser Thr Pro Val Gln Thr Arg Ser Ser Thr Gly
                   165                 170                 175
   Met Val Ser Asp Phe Ser Leu Gln Asn Val Thr Ala Arg Asp Ser Gly
               180                 185                 190
   Glu Tyr Ser Cys Val Tyr Tyr Gln Ala Lys Ala Pro Tyr Arg Ala Ser
           195                 200                 205
   Gly Pro Ser Asn Leu Leu Glu Ile Ser Val Ile Asp Asn His Leu Pro
       210                 215                 220
   Gln Asp Leu Ala Ala Ser Thr Phe Pro Pro Gln Leu Thr Ala Thr Ser
   225                 230                 235                 240
   Pro Lys Thr Pro Gly Thr Met Thr Glu Gly Tyr Thr Val Asp Asn Leu
                   245                 250                 255
   Ile Arg Val Gly Val Ala Ala Ala Ile Leu Leu Ile Val Gly Gly Phe
               260                 265                 270
   Leu Val Glu Ala Trp His Ser Glu Arg Leu Ser Pro Asn Lys Pro Cys
           275                 280                 285
   Ala Pro Gly Glu Lys
       290
<210> 5
   <211> 1018
   <212> DNA
   <213> Mus musculus
<220>
   <221> CDS
   <222> (49)..(894)
   <223>
<400> 5
   gctgatagta gacctgctga agacctttgg accagccgct gagccacc atg atc tct      57
                                                         Met Ile Ser
                                                         1
   agg ctc ctt tcc ctt ctc tgc ctc cgg tct cct ccc aag ccc agc ctc      105
   Arg Leu Leu Ser Leu Leu Cys Leu Arg Ser Pro Pro Lys Pro Ser Leu
       5                   10                  15
   agt gcc tgg ccc agc aca gtg ctt ccc acc aag agc cac gtg aca atg      153
   Ser Ala Trp Pro Ser Thr Val Leu Pro Thr Lys Ser His Val Thr Met
   20                  25                  30                  35
   caa tgt aag agc ccc acc ccg agt aaa tac ttc atc ctc aaa aag gaa      201
   Gln Cys Lys Ser Pro Thr Pro Ser Lys Tyr Phe Ile Leu Lys Lys Glu
                   40                  45                  50
   ggt ttc gct ttg aat tct gtg aag cca tat aat ttg aca gag gag acg      249
   Gly Phe Ala Leu Asn Ser Val Lys Pro Tyr Asn Leu Thr Glu Glu Thr
               55                  60                  65
   gct gat ttt cat ttc acc gac cta cga cag aat gat ggc gga cac tac      297
   Ala Asp Phe His Phe Thr Asp Leu Arg Gln Asn Asp Gly Gly His Tyr
           70                  75                  80
   acc tgt gaa tac tat agc aaa tgg ccc cat gac aca ccg tca cac ccc      345
   Thr Cys Glu Tyr Tyr Ser Lys Trp Pro His Asp Thr Pro Ser His Pro
       85                  90                  95
   agc aat gcc ctt ttc ttg ttg gtc aca ggg tac tta cct cag ccc tcc      393
   Ser Asn Ala Leu Phe Leu Leu Val Thr Gly Tyr Leu Pro Gln Pro Ser
   100                 105                 110                 115
   ttt caa gcc cac cac cgg ggt aca gtg act gca gga agc aag gtg act      441
   Phe Gln Ala His His Arg Gly Thr Val Thr Ala Gly Ser Lys Val Thr
                   120                 125                 130
   ttg cag tgc cag aaa gca ggc agt gtc ctc gga ccc gta aag ttt gcg      489
   Leu Gln Cys Gln Lys Ala Gly Ser Val Leu Gly Pro Val Lys Phe Ala
               135                 140                 145
   tta ctg aag gtg gga cac tca act cct gtg cag aca agg agc tca aca      537
   Leu Leu Lys Val Gly His Ser Thr Pro Val Gln Thr Arg Ser Ser Thr
           150                 155                 160
   gga atg gta tca gac ttc tct ctt cag aat gtg aca gcc aga gac tcg      585
   Gly Met Val Ser Asp Phe Ser Leu Gln Asn Val Thr Ala Arg Asp Ser
       165                 170                 175
   ggg gaa tac agc tgt gtt tac tat cag gca aag gct ccc tat cgg gcc      633
   Gly Glu Tyr Ser Cys Val Tyr Tyr Gln Ala Lys Ala Pro Tyr Arg Ala
   180                 185                 190                 195
   tca ggg ccc agc aat ctc ctt gag atc tct gtg ata gac aac cat ctg      681
   Ser Gly Pro Ser Asn Leu Leu Glu Ile Ser Val Ile Asp Asn His Leu
                   200                 205                 210
   cct caa gat ctt gct gcc tcg act ttc cca ccg caa ctg aca gca acc      729
   Pro Gln Asp Leu Ala Ala Ser Thr Phe Pro Pro Gln Leu Thr Ala Thr
               215                 220                 225
   tca ccc aag acc ccg ggt aca atg aca gaa ggc tac act gtg gat aat       777
   Ser Pro Lys Thr Pro Gly Thr Met Thr Glu Gly Tyr Thr Val Asp Asn
           230                 235                 240
   ctc atc cgg gtc ggt gtg gct gct gca atc ctg cta ata gtg gga ggc       825
   Leu Ile Arg Val Gly Val Ala Ala Ala Ile Leu Leu Ile Val Gly Gly
       245                 250                 255
   ttc ctg gtt gaa gcc tgg cac agt gag cgg ctg tct cca aat aaa ccc       873
   Phe Leu Val Glu Ala Trp His Ser Glu Arg Leu Ser Pro Asn Lys Pro
   260                 265                 270                 275
   tgt gcc cca gga gaa aaa tga atcttcggac caaactatct ctgtgaattt          924
   Cys Ala Pro Gly Glu Lys
                   280
   atgtgaaatt gatgcagcac tttgggaatc atccagagac aggctgcctc atcctgactc     984
   ttcacacaga acagaggcct ggacatatct ggac                                1018
<210> 6
   <211> 281
   <212> PRT
   <213> Mus musculus
<400> 6
   Met Ile Ser Arg Leu Leu Ser Leu Leu Cys Leu Arg Ser Pro Pro Lys
   1               5                   10                  15
   Pro Ser Leu Ser Ala Trp Pro Ser Thr Val Leu Pro Thr Lys Ser His
               20                  25                  30
   Val Thr Met Gln Cys Lys Ser Pro Thr Pro Ser Lys Tyr Phe Ile Leu
           35                  40                  45
   Lys Lys Glu Gly Phe Ala Leu Asn Ser Val Lys Pro Tyr Asn Leu Thr
       50                  55                  60
   Glu Glu Thr Ala Asp Phe His Phe Thr Asp Leu Arg Gln Asn Asp Gly
   65                  70                  75                  80
   Gly His Tyr Thr Cys Glu Tyr Tyr Ser Lys Trp Pro His Asp Thr Pro
                   85                  90                  95
   Ser His Pro Ser Asn Ala Leu Phe Leu Leu Val Thr Gly Tyr Leu Pro
               100                 105                 110
   Gln Pro Ser Phe Gln Ala His His Arg Gly Thr Val Thr Ala Gly Ser
           115                 120                 125
   Lys Val Thr Leu Gln Cys Gln Lys Ala Gly Ser Val Leu Gly Pro Val
       130                 135                 140
   Lys Phe Ala Leu Leu Lys Val Gly His Ser Thr Pro Val Gln Thr Arg
   145                 150                 155                 160
   Ser Ser Thr Gly Met Val Ser Asp Phe Ser Leu Gln Asn Val Thr Ala
                   165                 170                 175
   Arg Asp Ser Gly Glu Tyr Ser Cys Val Tyr Tyr Gln Ala Lys Ala Pro
               180                 185                 190
   Tyr Arg Ala Ser Gly Pro Ser Asn Leu Leu Glu Ile Ser Val Ile Asp
           195                 200                 205
   Asn His Leu Pro Gln Asp Leu Ala Ala Ser Thr Phe Pro Pro Gln Leu
       210                 215                 220
   Thr Ala Thr Ser Pro Lys Thr Pro Gly Thr Met Thr Glu Gly Tyr Thr
   225                 230                 235                 240
   Val Asp Asn Leu Ile Arg Val Gly Val Ala Ala Ala Ile Leu Leu Ile
                   245                 250                 255
   Val Gly Gly Phe Leu Val Glu Ala Trp His Ser Glu Arg Leu Ser Pro
               260                 265                 270
   Asn Lys Pro Cys Ala Pro Gly Glu Lys
           275                 280
<210> 7
   <211> 932
   <212> DNA
   <213> Mus musculus
<220>
   <221> CDS
   <222> (49) .. (819)
   <223>
<400> 7
   gctgatagta gacctgctga agacctttgg accagccgct gagccacc atg atc tct      57
                                                        Met Ile Ser
                                                        1
   agg ctc ctt tcc ctt ctc tgc ctc cgg ctg tgt gtt ggg caa aca gac      105
   Arg Leu Leu Ser Leu Leu Cys Leu Arg Leu Cys Val Gly Gln Thr Asp
       5                   10                  15
   att cct gaa aat ggg tct cct ccc aag ccc agc ctc agt gcc tgg ccc      153
   Ile Pro Glu Asn Gly Ser Pro Pro Lys Pro Ser Leu Ser Ala Trp Pro
   20                  25                  30                  35
   agc aca gtg ctt ccc acc aag agc cac gtg aca atg caa tgt aag agc       201
   Ser Thr Val Leu Pro Thr Lys Ser His Val Thr Met Gln Cys Lys Ser
                   40                  45                  50
   ccc acc ccg agt aaa tac ttc atc ctc aaa aag gaa ggt ttc gct ttg       249
   Pro Thr Pro Ser Lys Tyr Phe Ile Leu Lys Lys Glu Gly Phe Ala Leu
               55                  60                  65
   aat tct gtg aag cca tat aat ttg aca gag gag acg gct gat ttt cat       297
   Asn Ser Val Lys Pro Tyr Asn Leu Thr Glu Glu Thr Ala Asp Phe His
           70                  75                  80
   ttc acc gac cta cga cag aat gat ggc gga cac tac acc tgt gaa tac       345
   Phe Thr Asp Leu Arg Gln Asn Asp Gly Gly His Tyr Thr Cys Glu Tyr
       85                  90                  95
   tat agc aaa tgg ccc cat gac aca ccg tca cac ccc agc aat gcc ctt       393
   Tyr Ser Lys Trp Pro His Asp Thr Pro Ser His Pro Ser Asn Ala Leu
   100                 105                 110                 115
   ttc ttg ttg gtc aca ggg tac tta cct cag ccc tcc ttt caa gcc cac       441
   Phe Leu Leu Val Thr Gly Tyr Leu Pro Gln Pro Ser Phe Gln Ala His
                   120                 125                 130
   cac cgg ggt aca gtg act gca gga agc aag gtg act ttg cag tgc cag       489
   His Arg Gly Thr Val Thr Ala Gly Ser Lys Val Thr Leu Gln Cys Gln
               135                 140                 145
   aaa gca ggc agt gtc ctc gga ccc gta aag ttt gcg tta ctg aag gtg       537
   Lys Ala Gly Ser Val Leu Gly Pro Val Lys Phe Ala Leu Leu Lys Val
           150                 155                 160
   gga cac tca act cct gtg cag aca agg agc tca aca gga atg gta tca       585
   Gly His Ser Thr Pro Val Gln Thr Arg Ser Ser Thr Gly Met Val Ser
       165                 170                 175
   gac ttc tct ctt cag aat gtg aca gcc aga gac tcg ggg gaa tac agc       633
   Asp Phe Ser Leu Gln Asn Val Thr Ala Arg Asp Ser Gly Glu Tyr Ser
   180                 185                 190                 195
   tgt gtt tac tat cag gca aag gct ccc tat cgg gcc tca ggg ccc agc       681
   Cys Val Tyr Tyr Gln Ala Lys Ala Pro Tyr Arg Ala Ser Gly Pro Ser
                   200                 205                 210
   aat ctc ctt gag atc tct gtg ata gat gca aga caa cca tct gcc tca       729
   Asn Leu Leu Glu Ile Ser Val Ile Asp Ala Arg Gln Pro Ser Ala Ser
               215                 220                 225
   aga tct tgc tgc ctc gac ttt ccc acc gca act gac agc aac ctc acc       777
   Arg Ser Cys Cys Leu Asp Phe Pro Thr Ala Thr Asp Ser Asn Leu Thr
           230                 235                 240
   caa gac ccc ggg tac aat gac aga agg cta cac tgt gga taa               819
   Gln Asp Pro Gly Tyr Asn Asp Arg Arg Leu His Cys Gly
       245                 250                 255
   tctcatccgg gtcggtgtgg ctgctgcaat cctgctaata gtgggaggct tcctggttga     879
   agcctggcac agtgagcggc tgtctccaaa taaaccctgg taaaataact gaa 932
<210> 8
   <211> 256
   <212> PRT
   <213> Mus musculus
<400> 8
   Met Ile Ser Arg Leu Leu Ser Leu Leu Cys Leu Arg Leu Cys Val Gly
   1               5                   10                  15
   Gln Thr Asp Ile Pro Glu Asn Gly Ser Pro Pro Lys Pro Ser Leu Ser
               20                  25                  30
   Ala Trp Pro Ser Thr Val Leu Pro Thr Lys Ser His Val Thr Met Gln
           35                  40                  45
   Cys Lys Ser Pro Thr Pro Ser Lys Tyr Phe Ile Leu Lys Lys Glu Gly
       50                  55                  60
   Phe Ala Leu Asn Ser Val Lys Pro Tyr Asn Leu Thr Glu Glu Thr Ala
   65                  70                  75                  80
   Asp Phe His Phe Thr Asp Leu Arg Gln Asn Asp Gly Gly His Tyr Thr
                   85                  90                  95
   Cys Glu Tyr Tyr Ser Lys Trp Pro His Asp Thr Pro Ser His Pro Ser
               100                 105                 110
   Asn Ala Leu Phe Leu Leu Val Thr Gly Tyr Leu Pro Gln Pro Ser Phe
           115                 120                 125
   Gln Ala His His Arg Gly Thr Val Thr Ala Gly Ser Lys Val Thr Leu
       130                 135                 140
   Gln Cys Gln Lys Ala Gly Ser Val Leu Gly Pro Val Lys Phe Ala Leu
   145                 150                 155                 160
   Leu Lys Val Gly His Ser Thr Pro Val Gln Thr Arg Ser Ser Thr Gly
                   165                 170                 175
   Met Val Ser Asp Phe Ser Leu Gln Asn Val Thr Ala Arg Asp Ser Gly
               180                 185                 190
   Glu Tyr Ser Cys Val Tyr Tyr Gln Ala Lys Ala Pro Tyr Arg Ala Ser
           195                 200                 205
   Gly Pro Ser Asn Leu Leu Glu Ile Ser Val Ile Asp Ala Arg Gln Pro
       210                 215                 220
   Ser Ala Ser Arg Ser Cys Cys Leu Asp Phe Pro Thr Ala Thr Asp Ser
   225                 230                 235                 240
   Asn Leu Thr Gln Asp Pro Gly Tyr Asn Asp Arg Arg Leu His Cys Gly
                   245                 250                 255
<210> 9
   <211> 816
   <212> DNA
   <213> Homo sapiens
<220>
   <221> CDS
   <222> (1)..(816)
   <223>
<400> 9
   atg atc cct aag ctg ctt tcc ctc ctc tgt ttc aga ctg tgc gtg ggc      48
   Met Ile Pro Lys Leu Leu Ser Leu Leu Cys Phe Arg Leu Cys Val Gly
   1               5                   10                  15
   caa gga gac aca agg gga gat ggg tca ctg ccc aag ccg tcc ctc agt      96
   Gln Gly Asp Thr Arg Gly Asp Gly Ser Leu Pro Lys Pro Ser Leu Ser
               20                  25                  30
   gcc tgg ccc agc tcg gtg gtc cct gcc aac agc aat gtg acg ctg cga     144
   Ala Trp Pro Ser Ser Val Val Pro Ala Asn Ser Asn Val Thr Leu Arg
           35                  40                  45
   tgt tgg act cct gcc aga ggt gtg agc ttt gtt ctc agg aag gga gga     192
   Cys Trp Thr Pro Ala Arg Gly Val Ser Phe Val Leu Arg Lys Gly Gly
       50                  55                  60
   att att ctg gag tcc ccg aag ccc ctt gat tct aca gag ggc gcg gcc     240
   Ile Ile Leu Glu Ser Pro Lys Pro Leu Asp Ser Thr Glu Gly Ala Ala
   65                  70                  75                  80
   gaa ttt cac ctc aat aat cta aaa gtc aga aat gct gga gag tac acc     288
   Glu Phe His Leu Asn Asn Leu Lys Val Arg Asn Ala Gly Glu Tyr Thr
                   85                  90                  95
   tgt gaa tac tac aga aaa gca tcc ccc cac atc ctt tca cag cgc agt     336
   Cys Glu Tyr Tyr Arg Lys Ala Ser Pro His Ile Leu Ser Gln Arg Ser
               100                 105                 110
   gac gtc ctt cta ctg ttg gtg aca gga cat tta tct aaa cct ttc ctc     384
   Asp Val Leu Leu Leu Leu Val Thr Gly His Leu Ser Lys Pro Phe Leu
           115                 120                 125
   cga acc tac caa agg ggt aca gtg acc gca ggt gga agg gtg act ctg     432
   Arg Thr Tyr Gln Arg Gly Thr Val Thr Ala Gly Gly Arg Val Thr Leu
       130                 135                 140
   cag tgc cag aag cga gac caa ttg ttt gtg cct atc atg ttc gct cta     480
   Gln Cys Gln Lys Arg Asp Gln Leu Phe Val Pro Ile Met Phe Ala Leu
   145                 150                 155                 160
   ctg aag gca ggg acg cca tca ccc atc cag ctg cag agt cca gcg ggg        528
   Leu Lys Ala Gly Thr Pro Ser Pro Ile Gln Leu Gln Ser Pro Ala Gly
                   165                 170                 175
   aag gag ata gac ttc tct ctg gtg gac gtg aca gcc ggc gat gct ggg        576
   Lys Glu Ile Asp Phe Ser Leu Val Asp Val Thr Ala Gly Asp Ala Gly
               180                 185                 190
   aac tac agc tgc atg tac tac cag aca aag tct ccc ttc tgg gcc tca        624
   Asn Tyr Ser Cys Met Tyr Tyr Gln Thr Lys Ser Pro Phe Trp Ala Ser
           195                 200                 205
   gaa ccc agt gat cag ctt gag ata ttg gtg aca gtt ccc cca ggt acc        672
   Glu Pro Ser Asp Gln Leu Glu Ile Leu Val Thr Val Pro Pro Gly Thr
       210                 215                 220
   aca tcg agc aac tac tcc ctg ggt aac ttc gta cga ctg ggt ctg gct        720
   Thr Ser Ser Asn Tyr Ser Leu Gly Asn Phe Val Arg Leu Gly Leu Ala
   225                 230                 235                 240
   gcc gta att gtg gtt atc atg gga gct ttc ctg gtg gag gcc tgg tac        768
   Ala Val Ile Val Val Ile Met Gly Ala Phe Leu Val Glu Ala Trp Tyr
                   245                 250                 255
   agc cgg aat gtg tct cca ggt gaa tca gag gcc ttc aaa cca gag tga        816
   Ser Arg Asn Val Ser Pro Gly Glu Ser Glu Ala Phe Lys Pro Glu
               260                 265                 270
<210> 10
   <211> 271
   <212> PRT
   <213> Homo sapiens
<400> 10
   Met Ile Pro Lys Leu Leu Ser Leu Leu Cys Phe Arg Leu Cys Val Gly
   1               5                   10                  15
   Gln Gly Asp Thr Arg Gly Asp Gly Ser Leu Pro Lys Pro Ser Leu Ser
               20                  25                  30
   Ala Trp Pro Ser Ser Val Val Pro Ala Asn Ser Asn Val Thr Leu Arg
           35                  40                  45
   Cys Trp Thr Pro Ala Arg Gly Val Ser Phe Val Leu Arg Lys Gly Gly
       50                  55                  60
   Ile Ile Leu Glu Ser Pro Lys Pro Leu Asp Ser Thr Glu Gly Ala Ala
   65                  70                  75                  80
   Glu Phe His Leu Asn Asn Leu Lys Val Arg Asn Ala Gly Glu Tyr Thr
                   85                  90                  95
   Cys Glu Tyr Tyr Arg Lys Ala Ser Pro His Ile Leu Ser Gln Arg Ser
               100                 105                 110
   Asp Val Leu Leu Leu Leu Val Thr Gly His Leu Ser Lys Pro Phe Leu
           115                 120                 125
   Arg Thr Tyr Gln Arg Gly Thr Val Thr Ala Gly Gly Arg Val Thr Leu
       130                 135                 140
   Gln Cys Gln Lys Arg Asp Gln Leu Phe Val Pro Ile Met Phe Ala Leu
   145                 150                 155                 160
   Leu Lys Ala Gly Thr Pro Ser Pro Ile Gln Leu Gln Ser Pro Ala Gly
                   165                 170                 175
   Lys Glu Ile Asp Phe Ser Leu Val Asp Val Thr Ala Gly Asp Ala Gly
               180                 185                 190
   Asn Tyr Ser Cys Met Tyr Tyr Gln Thr Lys Ser Pro Phe Trp Ala Ser
           195                 200                 205
   Glu Pro Ser Asp Gln Leu Glu Ile Leu Val Thr Val Pro Pro Gly Thr
       210                 215                 220
   Thr Ser Ser Asn Tyr Ser Leu Gly Asn Phe Val Arg Leu Gly Leu Ala
   225                 230                 235                 240
   Ala Val Ile Val Val Ile Met Gly Ala Phe Leu Val Glu Ala Trp Tyr
                   245                 250                 255
   Ser Arg Asn Val Ser Pro Gly Glu Ser Glu Ala Phe Lys Pro Glu
               260                 265                 270
<210> 11
   <211> 925
   <212> DNA
   <213> Mus musculus
<220>
   <221> CDS
   <222> (49) .. (915)
   <223>
<400> 11
   gctgatagta gacctgctga agacctttgg accagccgct gagccacc atg atc tct      57
                                                        Met Ile Ser
                                                        1
   agg ctc ctt tcc ctt ctc tgc ctc cgg ctg tgt gtt ggg caa aca gac      105
   Arg Leu Leu Ser Leu Leu Cys Leu Arg Leu Cys Val Gly Gln Thr Asp
       5                   10                  15
   att cct gaa aat ggg tct cct ccc aag ccc agc ctc agt gcc tgg ccc      153
   Ile Pro Glu Asn Gly Ser Pro Pro Lys Pro Ser Leu Ser Ala Trp Pro
   20                  25                  30                  35
   agc aca gtg ctt ccc acc aag agc cac gtg aca atg caa tgt aag agc      201
   Ser Thr Val Leu Pro Thr Lys Ser His Val Thr Met Gln Cys Lys Ser
                   40                  45                  50
   ccc acc ccg agt aaa tac ttc atc ctc aaa aag gaa ggt ttc gct ttg      249
   Pro Thr Pro Ser Lys Tyr Phe Ile Leu Lys Lys Glu Gly Phe Ala Leu
               55                  60                  65
   aat tct gtg aag cca tat aat ttg aca gag gag acg gct gat ttt cat      297
   Asn Ser Val Lys Pro Tyr Asn Leu Thr Glu Glu Thr Ala Asp Phe His
           70                  75                  80
   ttc acc gac cta cga cag aat gat ggc gga cac tac acc tgt gaa tac      345
   Phe Thr Asp Leu Arg Gln Asn Asp Gly Gly His Tyr Thr Cys Glu Tyr
       85                  90                  95
   tat agc aaa tgg ccc cat gac aca ccg tca cac ccc agc aat gcc ctt      393
   Tyr Ser Lys Trp Pro His Asp Thr Pro Ser His Pro Ser Asn Ala Leu
   100                 105                 110                 115
   ttc ttg ttg gtc aca ggg tac tta cct cag ccc tcc ttt caa gcc cac      441
   Phe Leu Leu Val Thr Gly Tyr Leu Pro Gln Pro Ser Phe Gln Ala His
                   120                 125                 130
   cac cgg ggt aca gtg act gca gga agc aag gtg act ttg cag tgc cag      489
   His Arg Gly Thr Val Thr Ala Gly Ser Lys Val Thr Leu Gln Cys Gln
               135                 140                 145
   aaa gca ggc agt gtc ctc gga ccc gta aag ttt gcg tta ctg aag gtg      537
   Lys Ala Gly Ser Val Leu Gly Pro Val Lys Phe Ala Leu Leu Lys Val
           150                 155                 160
   gga cac tca act cct gtg cag aca agg agc tca aca gga atg gta tca      585
   Gly His Ser Thr Pro Val Gln Thr Arg Ser Ser Thr Gly Met Val Ser
       165                 170                 175
   gac ttc tct ctt cag aat gtg aca gcc aga gac tcg ggg gaa tac agc      633
   Asp Phe Ser Leu Gln Asn Val Thr Ala Arg Asp Ser Gly Glu Tyr Ser
   180                 185                 190                 195
   tgt gtt tac tat cag gca aag gct ccc tat cgg gcc tca ggg ccc agc      681
   Cys Val Tyr Tyr Gln Ala Lys Ala Pro Tyr Arg Ala Ser Gly Pro Ser
                   200                 205                 210
   aat ctc ctt gag atc tct gtg ata gac aac cat ctg cct caa gat ctt      729
   Asn Leu Leu Glu Ile Ser Val Ile Asp Asn His Leu Pro Gln Asp Leu
               215                 220                 225
   gct gcc tcg act ttc cca ccg caa ctg aca gca acc tca ccc aag acc      777
   Ala Ala Ser Thr Phe Pro Pro Gln Leu Thr Ala Thr Ser Pro Lys Thr
           230                 235                 240
   ccg ggt aca atg aca gaa ggc tac act gtg gat aat ctc atc cgg gtc      825
   Pro Gly Thr Met Thr Glu Gly Tyr Thr Val Asp Asn Leu Ile Arg Val
       245                 250                 255
   ggt gtg gct gct gca atc ctg cta ata gtg gga ggc ttc ctg gtt gaa       873
   Gly Val Ala Ala Ala Ile Leu Leu Ile Val Gly Gly Phe Leu Val Glu
   260                 265                 270                 275
   gcc tgg cac agt gag cgg ctg tct cca aat aaa ccc tgg taa               915
   Ala Trp His Ser Glu Arg Leu Ser Pro Asn Lys Pro Trp
                   280                 285
   aataactgaa                                                            925
<210> 12
   <211> 288
   <212> PRT
   <213> Mus musculus
<400> 12
   Met Ile Ser Arg Leu Leu Ser Leu Leu Cys Leu Arg Leu Cys Val Gly
   1               5                   10                  15
   Gln Thr Asp Ile Pro Glu Asn Gly Ser Pro Pro Lys Pro Ser Leu Ser
               20                  25                  30
   Ala Trp Pro Ser Thr Val Leu Pro Thr Lys Ser His Val Thr Met Gln
           35                  40                  45
   Cys Lys Ser Pro Thr Pro Ser Lys Tyr Phe Ile Leu Lys Lys Glu Gly
       50                  55                  60
   Phe Ala Leu Asn Ser Val Lys Pro Tyr Asn Leu Thr Glu Glu Thr Ala
   65                  70                  75                  80
   Asp Phe His Phe Thr Asp Leu Arg Gln Asn Asp Gly Gly His Tyr Thr
                   85                  90                  95
   Cys Glu Tyr Tyr Ser Lys Trp Pro His Asp Thr Pro Ser His Pro Ser
               100                 105                 110
   Asn Ala Leu Phe Leu Leu Val Thr Gly Tyr Leu Pro Gln Pro Ser Phe
           115                 120                 125
   Gln Ala His His Arg Gly Thr Val Thr Ala Gly Ser Lys Val Thr Leu
       130                 135                 140
   Gln Cys Gln Lys Ala Gly Ser Val Leu Gly Pro Val Lys Phe Ala Leu
   145                 150                 155                 160
   Leu Lys Val Gly His Ser Thr Pro Val Gln Thr Arg Ser Ser Thr Gly
                   165                 170                 175
   Met Val Ser Asp Phe Ser Leu Gln Asn Val Thr Ala Arg Asp Ser Gly
               180                 185                 190
   Glu Tyr Ser Cys Val Tyr Tyr Gln Ala Lys Ala Pro Tyr Arg Ala Ser
           195                 200                 205
   Gly Pro Ser Asn Leu Leu Glu Ile Ser Val Ile Asp Asn His Leu Pro
       210                 215                 220
   Gln Asp Leu Ala Ala Ser Thr Phe Pro Pro Gln Leu Thr Ala Thr Ser
   225                 230                 235                 240
   Pro Lys Thr Pro Gly Thr Met Thr Glu Gly Tyr Thr Val Asp Asn Leu
                   245                 250                 255
   Ile Arg Val Gly Val Ala Ala Ala Ile Leu Leu Ile Val Gly Gly Phe
               260                 265                 270
   Leu Val Glu Ala Trp His Ser Glu Arg Leu Ser Pro Asn Lys Pro Trp
           275                 280                 285
<210> 13
   <211> 714
   <212> DNA
   <213> Mus musculus
<400> 13
   atgcagctgg caagaggaac agtaggaggc cgtggctgcg ctctctttcc actgctgagc     60
   atcctagtcg tccagggtgc gcgtatcgtc ctctccttgg agataagtgc cgatgctcac    120
   gtccgaggct atgtgggaga gaagatcaag ttgaaatgca ccttcaagtc atcttcagat    180
   gtcactgaca agctgaccat agactggaca taccgccctc ccagcagcag ccgcacagag    240
   tctatttttc actatcagtc tttccagtac ccgaccacag cgggcacatt ccgagaccgg    300
   atctcctggg ccggaaatgt ctacaaaggg gatgcgtcca tcagtatcag caaccccact    360
   ctaaaggaca atgggacgtt cagctgtgct gtgaagaacc ctccagacgt gtaccacaat    420
   atccccctaa cagagctcac ggtcacagaa agggggttcg gcaccatgct gtcttctgtg    480
   gcccttctct ccatcctcgt cttcgtcccc tcagcagtgg tggtcattct gctgctggtg    540
   cgaatgggga ggaaggcaac aggggtgcag aagaggagca ggtctggcta taagaagtct    600
   tccattgaag tttccgatga cactgaccag gaggacagca atgactgcat gacgaggctt    660
   tgtgtccgct gtgcagagtg tctggattca gactacgaag aagaggcgta ctga          714
<210> 14
   <211> 237
   <212> PRT
   <213> Mus musculus
<400> 14
   Met Gln Leu Ala Arg Gly Thr Val Gly Gly Arg Gly Cys Ala Leu Phe
   1               5                   10                  15
   Pro Leu Leu Ser Ile Leu Val Val Gln Gly Ala Arg Ile Val Leu Ser
               20                  25                  30
   Leu Glu Ile Ser Ala Asp Ala His Val Arg Gly Tyr Val Gly Glu Lys
           35                  40                  45
   Ile Lys Leu Lys Cys Thr Phe Lys Ser Ser Ser Asp Val Thr Asp Lys
       50                  55                  60
   Leu Thr Ile Asp Trp Thr Tyr Arg Pro Pro Ser Ser Ser Arg Thr Glu
   65                  70                  75                  80
   Ser Ile Phe His Tyr Gln Ser Phe Gln Tyr Pro Thr Thr Ala Gly Thr
                   85                  90                  95
   Phe Arg Asp Arg Ile Ser Trp Ala Gly Asn Val Tyr Lys Gly Asp Ala
               100                 105                 110
   Ser Ile Ser Ile Ser Asn Pro Thr Leu Lys Asp Asn Gly Thr Phe Ser
           115                 120                 125
   Cys Ala Val Lys Asn Pro Pro Asp Val Tyr His Asn Ile Pro Leu Thr
       130                 135                 140
   Glu Leu Thr Val Thr Glu Arg Gly Phe Gly Thr Met Leu Ser Ser Val
   145                 150                 155                 160
   Ala Leu Leu Ser Ile Leu Val Phe Val Pro Ser Ala Val Val Val Ile
                   165                 170                 175
   Leu Leu Leu Val Arg Met Gly Arg Lys Ala Thr Gly Val Gln Lys Arg
               180                 185                 190
   Ser Arg Ser Gly Tyr Lys Lys Ser Ser Ile Glu Val Ser Asp Asp Thr
           195                 200                 205
   Asp Gln Glu Asp Ser Asn Asp Cys Met Thr Arg Leu Cys Val Arg Cys
       210                 215                 220
   Ala Glu Cys Leu Asp Ser Asp Tyr Glu Glu Glu Ala Tyr
   225                 230                 235
<210> 15
   <211> 708
   <212> DNA
   <213> Homo sapiens
<400> 15
   atgcagcaga gaggagcagc tggaagccgt ggctgcgctc tcttccctct gctgggcgtc     60
   ctgttcttcc agggtgttta tatcgtcttt tccttggaga ttcgtgcaga tgcccatgtc    120
   cgaggttatg ttggagaaaa gatcaagttg aaatgcactt tcaagtcaac ttcagatgtc    180
   actgacaagc ttactataga ctggacatat cgccctccca gcagcagcca cacagtatca    240
   atatttcatt atcagtcttt ccagtaccca accacagcag gcacatttcg ggatcggatt    300
   tcctgggttg gaaatgtata caaaggggat gcatctataa gtataagcaa ccctaccata    360
   aaggacaatg ggacattcag ctgtgctgtg aagaatcccc cagatgtgca ccataatatt    420
   cccatgacag agctaacagt cacagaaagg ggttttggca ccatgctttc ctctgtggcc    480
   cttctttcca tccttgtctt tgtgccctca gccgtggtgg ttgctctgct gctggtgaga    540
   atggggagga aggctgctgg gctgaagaag aggagcaggt ctggctataa gaagtcatct    600
   attgaggttt ccgatgacac tgatcaggag gaggaagagg cgtgtatggc gaggctttgt    660
   gtccgttgcg ctgagtgcct ggattcagac tatgaagaga catattga                 708
<210> 16
   <211> 235
   <212> PRT
   <213> Homo sapiens
<400> 16
   Met Gln Gln Arg Gly Ala Ala Gly Ser Arg Gly Cys Ala Leu Phe Pro
   1               5                   10                  15
   Leu Leu Gly Val Leu Phe Phe Gln Gly Val Tyr Ile Val Phe Ser Leu
               20                  25                  30
   Glu Ile Arg Ala Asp Ala His Val Arg Gly Tyr Val Gly Glu Lys Ile
           35                  40                  45
   Lys Leu Lys Cys Thr Phe Lys Ser Thr Ser Asp Val Thr Asp Lys Leu
       50                  55                  60
   Thr Ile Asp Trp Thr Tyr Arg Pro Pro Ser Ser Ser His Thr Val Ser
   65                  70                  75                  80
   Ile Phe His Tyr Gln Ser Phe Gln Tyr Pro Thr Thr Ala Gly Thr Phe
                   85                  90                  95
   Arg Asp Arg Ile Ser Trp Val Gly Asn Val Tyr Lys Gly Asp Ala Ser
               100                 105                 110
   Ile Ser Ile Ser Asn Pro Thr Ile Lys Asp Asn Gly Thr Phe Ser Cys
           115                 120                 125
   Ala Val Lys Asn Pro Pro Asp Val His His Asn Ile Pro Met Thr Glu
       130                 135                 140
   Leu Thr Val Thr Glu Arg Gly Phe Gly Thr Met Leu Ser Ser Val Ala
   145                 150                 155                 160
   Leu Leu Ser Ile Leu Val Phe Val Pro Ser Ala Val Val Val Ala Leu
                   165                 170                 175
   Leu Leu Val Arg Met Gly Arg Lys Ala Ala Gly Leu Lys Lys Arg Ser
               180                 185                 190
   Arg Ser Gly Tyr Lys Lys Ser Ser Ile Glu Val Ser Asp Asp Thr Asp
           195                 200                 205
   Gln Glu Glu Glu Glu Ala Cys Met Ala Arg Leu Cys Val Arg Cys Ala
       210                 215                 220
   Glu Cys Leu Asp Ser Asp Tyr Glu Glu Thr Tyr
   225                 230                 235
<210> 17
   <211> 20
   <212> DNA
   <213> Artificial
<220>
   <223> primer
<400> 17
   gtgactttgc agtgccagaa 20
<210> 18
   <211> 20
   <212> DNA
   <213> Artificial
<220>
   <223> primer
<400> 18
   tgcacaggag ttgagtgtcc 20
<210> 19
   <211> 12
   <212> DNA
   <213> Artificial
<220>
   <223> primer
<400> 19
   acatcactcc gt 12
<210> 20
   <211> 50
   <212> DNA
   <213> Artificial
<220>
   <223> primer
<400> 20
   acggagtgat gtccgtcgac gtatctctgc gttgatactt cagcgtagct 50
<210> 21
   <211> 26
   <212> DNA
   <213> Artificial
<220>
   <223> primer
<400> 21
   agctacgctg aagtatcaac gcagag 26
<210> 22
   <211> 24
   <212> DNA
   <213> Artificial
<220>
   <223> primer
<400> 22
   cttctggcac tgcagagtca ccct 24
<210> 23
   <211> 24
   <212> DNA
   <213> Artificial
<220>
   <223> primer
<400> 23
   ggagagtaca cctgtgaata ctac 24
<210> 24
   <211> 26
   <212> DNA
   <213> Artificial
<220>
   <223> primer
<400> 24
   gtatcaacgc agagatacgt cgacgg 26
<210> 25
   <211> 24
   <212> DNA
   <213> Artificial
<220>
   <223> primer
<400> 25
   tccacctgcg gtcactgtac ccct 24
<210> 26
   <211> 30
   <212> DNA
   <213> Artificial
<220>
   <223> primer
<400> 26
   ctacagaaaa gcatcccccc acatcctttc 30
<210> 27
   <211> 24
   <212> DNA
   <213> Artificial
<220>
   <223> primer
<400> 27
   gctgatagta gacctgctga agac 24
<210> 28
   <211> 23
   <212> DNA
   <213> Artificial
<220>
   <223> primer
<400> 28
   gtccagatat gtccaggcct ctg 23
<210> 29
   <211> 23
   <212> DNA
   <213> Artificial
<220>
   <223> primer
<400> 29
   ttcagttatt ttaccagggt tta 23
<210> 30
   <211> 20
   <212> DNA
   <213> Artificial
<220>
   <223> primer
<400> 30
   tctgtgatag acaaccatct 20
<210> 31
   <211> 20
   <212> DNA
   <213> Artificial
<220>
   <223> primer
<400> 31
   gtcattgtac ccggggtctt 20
<210> 32
   <211> 26
   <212> DNA
   <213> Artificial
<220>
   <223> primer
<400> 32
   atgacagaag gctacactgt ggataa 26
<210> 33
   <211> 20
   <212> DNA
   <213> Artificial
<220>
   <223> primer
<400> 33
   tcatttttct cctggggcac 20
<210> 34
   <211> 21
   <212> DNA
   <213> Artificial
<220>
   <223> primer
<400> 34
   gatctctgtg atagatgcaa g 21
<210> 35
   <211> 20
   <212> DNA
   <213> Artificial
<220>
   <223> primer
<400> 35
   gtcattgtac ccggggtctt 20
<210> 36
   <211> 39
   <212> DNA
   <213> Artificial
<220>
   <223> primer
<400> 36
   cgcgtcgacg ccaccatgat ctctaggctc ctttccctt 39
<210> 37
   <211> 36
   <212> DNA
   <213> Artificial
<220>
   <223> primer
<400> 37
   gcgggcggcc gcttaccagg gtttatttgg agacag 36
<210> 38
   <211> 39
   <212> DNA
   <213> Artificial
<220>
   <223> primer
<400> 38
   cgcggcggcc gcattatcca cagtgtagcc ttctgtcat 39
<210> 39
   <211> 33
   <212> DNA
   <213> Artificial
<220>
   <223> primer
<400> 39
   cgcctcgagc tgggagagcc gcagctctgc tat 33
<210> 40
   <211> 36
   <212> DNA
   <213> Artificial
<220>
   <223> primer
<400> 40
   gcgggcggcc gcctactggg gtggtttctc atgctt 36
<210> 41
   <211> 33
   <212> DNA
   <213> Artificial
<220>
   <223> primer
<400> 41
   cgcgtcgacc agacatcggc aggttcctgc tcc 33
<210> 42
   <211> 36
   <212> DNA
   <213> Artificial
<220>
   <223> primer
<400> 42
   gcgggcggcc gctcagcctc tgccaggcat gttgat 36
<210> 43
   <211> 33
   <212> DNA
   <213> Artificial
<220>
   <223> primer
<400> 43
   cgcgtcgact taagtcccgt acaggcccag agt 33
<210> 44
   <211> 36
   <212> DNA
   <213> Artificial
<220>
   <223> primer
<400> 44
   gcgggcggcc gctcatctgt aatattgcct ctgtgt 36
<210> 45
   <211> 24
   <212> DNA
   <213> Artificial
<220>
   <223> primer
<400> 45
   tgtgaatact acagaaaagc atcc 24
<210> 46
   <211> 24
   <212> DNA
   <213> Artificial
<220>
   <223> primer
<400> 46
   tccacctgcg gtcactgtac ccct 24
<210> 47
   <211> 24
   <212> DNA
   <213> Artificial
<220>
   <223> primer
<400> 47
   cttctggcac tgcagagtca ccct 24
<210> 48
   <211> 24
   <212> DNA
   <213> Artificial
<220>
   <223> primer
<400> 48
   ggagagtaca cctgtgaata ctac 24
<210> 49
   <211> 24
   <212> DNA
   <213> Artificial
<220>
   <223> primer
<400> 49
   tgtgaatact acagaaaagc atcc 24
<210> 50
   <211> 24
   <212> DNA
   <213> Artificial
<220>
   <223> primer
<400> 50
   tccacctgcg gtcactgtac ccct 24
<210> 51
   <211> 39
   <212> DNA
   <213> Artificial
<220>
   <223> primer
<400> 51
   cgcgtcgacg ccaccatgat ccctaagctg ctttccctc 39
<210> 52
   <211> 35
   <212> DNA
   <213> Artificial
<220>
   <223> primer
<400> 52
   cgcgcggccg cctagcgcat gctacccttg gcagc 35
<210> 53
   <211> 36
   <212> DNA
   <213> Artificial
<220>
   <223> primer
<400> 53
   gcgggcggcc gcacccaggg agtagttgct cgatgt 36
<210> 54
   <211> 20
   <212> DNA
   <213> Artificial
<220>
   <223> primer
<400> 54
   cagctggcaa gaggaacagt 20
<210> 55
   <211> 20
   <212> DNA
   <213> Artificial
<220>
   <223> primer
<400> 55
   gagcatcggc acttatctcc 20
<210> 56
   <211> 39
   <212> DNA
   <213> Artificial
<220>
   <223> primer
<400> 56
   cgcgtcgacg ccaccatgca gctggcaaga ggaacagta 39
<210> 57
   <211> 36
   <212> DNA
   <213> Artificial
<220>
   <223> primer
<400> 57
   gcgggcggcc gctcagtacg cctcttcttc gtagtc 36
<210> 58
   <211> 39
   <212> DNA
   <213> Artificial
<220>
   <223> primer
<400> 58
   cgcgtcgacg ccaccatgca gcagagagga gcagctgga 39
<210> 59
   <211> 36
   <212> DNA
   <213> Artificial
<220>
   <223> primer
<400> 59
   gcgggcggcc gctcaatatg tctcttcata gtctga 36